# EUROPEAN PATENT SPECIFICATION

(11) **EP 1 848 737 B1**
(45) Date of publication and mention of the grant of the patent: **18.05.2016**
(21) Application number: 06734098.4
(22) Date of filing: 30.01.2006
(51) Int. Cl.: C07K 14/53, C07K 16/24, C12N 15/24, C12N 5/10, A61K 38/19

(54) **HOMOGENEOUS PREPARATIONS OF IL-31**
HOMOGENE PRÄPARATE VON IL-31
PREPARATIONS HOMOGENES D'IL-31

(30) Priority: 28.01.2005 US 648189 P
(43) Date of publication of application: 31.10.2007
(73) Proprietor: ZymoGenetics, Inc., Seattle, WA 98102 (US)
(72) Inventor: BRADY, Lowell J., Tacoma, Washington 98402 (US); BUKOWSKI, Thomas R., Seattle, Washington 98103 (US); CHAN, Chung-leung, Sammamish, WA 98075 (US)
(74) Representative: Griffin, Philippa Jane
(86) International application number: PCT/US2006/003318
(87) International publication number: WO 2006/081573

(56) References cited:
- WO-A2-03/060090
- DILLON STACEY R ET AL: "Interleukin 31, a cytokine produced by activated T cells, induces dermatitis in mice." NATURE IMMUNOLOGY. JUL 2004, vol. 5, no. 7, July 2004 (2004-07), pages 752-760, XP002407998 ISSN: 1529-2908 cited in the application

## Description

### BACKGROUND OF THE INVENTION

The increased availability and identification of genes from human and other genomes has led to an increased need for efficient expression and purification of recombinant proteins. The expression of proteins in bacteria is by far the most widely used approach for the production of cloned genes. For many reasons, expression in bacteria is preferred to expression in eukaryotic cells. For example, bacteria are much easier to grow than eukaryotic cells. More specifically, the availability of a wealth of sophisticated molecular genetic tools and thousands of mutants make *E. coli*, as an expression host, extremely useful for protein production. However, the high-level production of functional proteins in *E. coli.*, especially those from eukaryotic sources has often been difficult.

IL-31 is a recently discovered protein having the structure of a four-helical-bundle cytokine. This new cytokine is fully described in co-owned PCT application WO 03/060090 and Dillon, et al., Nautre Immunol. 5:752-760, 2004. IL-31 is a ligand with high specificity for the receptor IL-31RA and at least one additional subunit comprising OncostatinM receptor beta. IL-31 was isolated from a cDNA library generated from activated human peripheral blood cells (hPBCs), which were selected for CD3. CD3 is a cell surface marker unique to cells of lymphoid origin, particularly T cells.

Both the murine and human forms of IL-31 are known to have an odd number of cysteines. (PCT application WO 03/060090 and Dillon, et al., supra.) Expression of recombinant IL-31 can result in a heterologous mixture of proteins composed of intramolecular disulfide binding in multiple conformations. The separation of these forms can be difficult and laborious. It is therefore desirable to provide IL-31 molecules having a single intramolecular disulfide bonding pattern upon expression and methods for refolding and purifying these preparations to maintain homogeneity. Thus, the present invention provides for compositions and methods to produce homogeneous preparations of IL-31.

Despite advances in the expression of recombinant proteins in bacterial hosts, there exists a need for improved methods for producing biologically active and purified recombinant IL-31 proteins in prokaryotic systems which result in higher yields for protein production. These and other aspects of the invention will become evident upon reference to the following detailed description. In addition, various references are identified below and are incorporated by reference in their entirety.

WO 03/060090 describes the cytokine "Zcytor17 ligand".

The present invention provides an isolated polypeptide comprising an amino acid sequence selected from the group consisting of SEQ ID NOs: 15, 18, 23, 28, and residue 2 to residue 133 of SEQ ID NO:23.

The present invention also provides an expression vector comprising the following operably linked elements: a transcription promoter; a DNA segment encoding said polypeptide; and a transcription terminator.

The present invention also provides a cultured cell into which has been introduced said expression vector, wherein the cell expresses the polypeptide encoded by the DNA segment.

The present invention further provides a process for producing a polypeptide comprising: culturing a cell into which has been introduced said expression vector, wherein the cell expresses the polypeptide encoded by the DNA segment; and recovering the expressed polypeptide.

The present invention further provides an antibody or antibody fragment that specifically binds to said polypeptide. The invention also provides an anti-idiotype antibody that specifically binds to said antibody.

The present invention also provides a formulation comprising said isolated polypeptide and a pharmaceutically acceptable vehicle. The present invention also provides a kit comprising said formulation.

Within one aspect, the invention provides an isolated polypeptide comprising an amino acid sequence selected from the group consisting of SEQ ID NOs: 15, 18, 23, and 28.

Within another aspect, the invention provides an expression vector comprising the following operably linked elements: a transcription promoter; a DNA segment encoding a polypeptide comprising an amino acid sequence selected from the group consisting of SEQ ID NOs: 15, 18, 23 and 28; and a transcription terminator.

Within another aspect, the invention provides a cultured cell into which has been introduced an expression vector comprising a DNA segment encoding a polypeptide comprising an amino acid sequence selected from the group consisting of SEQ ID NOs: 15, 18, 23, and 28, wherein the cell expresses the polypeptide encoded by the DNA segment. Within an embodiment the cultured cell is a prokaryotic cell. Within another embodiment the cell is a gram negative cell. Within another embodiment the cell is E. coli. Within another embodiment, the E. coli cell is *E. coli* strain W3110.

Within another aspect, the invention provides a process for producing a polypeptide comprising:

culturing a cell into which has been introduced an expression vector comprising a DNA segment encoding a polypeptide comprising an amino acid sequence selected from the group consisting of SEQ ID NOs: 15, 18, 23, and 28, and 28, wherein the cell expresses the polypeptide encoded by the DNA segment; and recovering the expressed polypeptide.

Within another aspect, the invention provides an antibody or antibody fragment that specifically binds to a polypeptide comprising an amino acid sequence selected from the group consisting of SEQ ID NOs: 15, 18, 23, and 28. Within an embodiment the antibody is selected from the group consisting of a polyclonal antibody, a murine monoclonal antibody, a humanized antibody derived from a murine monoclonal antibody, an antibody fragment, neutralizing antibody, and a human monoclonal antibody. Within another embodiment the antibody fragment is selected from the group consisting of F(ab'), F(ab), Fab', Fab, Fv, scFv, and minimal recognition unit.

Within another aspect is provided an anti-idiotype antibody comprising an anti-idiotype antibody that specifically binds to the antibody.

Within another aspect the invention provides an isolated polypeptide consisting of an amino acid sequence selected from the group consisting of SEQ ID NOs: 15, 18, 23, and 28.

Within another aspect is provided a formulation comprising:

an isolated polypeptide selected from the group consisting of SEQ ID NOs: 15, 18, 23, and 28; and

a pharmaceutically acceptable vehicle. Within an embodiment, formulation is provide in a kit.

Within another aspect, the polypeptide comprising an amino acid sequence selected from the group consisting of SEQ ID NOs: 15, 18, 23, and 28 is proinflammatory.

Within another aspect the invention provides an isolated polypeptide comprising the amino acid sequence from residue 2 to residue 133 of SEQ ID NO: 23.

### DETAILED DESCRIPTION OF THE INVENTION

Prior to setting forth the invention in detail, it may be helpful to the understanding thereof to define the following terms:

The term "affinity tag" is used herein to denote a polypeptide segment that can be attached to a second polypeptide to provide for purification or detection of the second polypeptide or provide sites for attachment of the second polypeptide to a substrate. In principal, any peptide or protein for which an antibody or other specific binding agent is available can be used as an affinity tag. Affinity tags include a poly-histidine tract, protein A (Nilsson et al., EMBO J. 4:1075, 1985; Nilsson et al., Methods Enzymol. 198:3, 1991), glutathione S transferase (Smith and Johnson, Gene 67:31, 1988), Glu-Glu affinity tag (Grussenmeyer et al., Proc. Natl. Acad. Sci. USA 82:7952-4, 1985), substance P, Flag™ peptide (Hopp et al., Biotechnology 6:1204-10, 1988), streptavidin binding peptide, or other antigenic epitope or binding domain. See, in general, Ford et al., Protein Expression and Purification 2: 95-107, 1991. DNAs encoding affinity tags are available from commercial suppliers (e.g., Pharmacia Biotech, Piscataway, NJ).

The term "allelic variant" is used herein to denote any of two or more alternative forms of a gene occupying the same chromosomal locus. Allelic variation arises naturally through mutation, and may result in phenotypic polymorphism within populations. Gene mutations can be silent (no change in the encoded polypeptide) or may encode polypeptides having altered amino acid sequence. The term allelic variant is also used herein to denote a protein encoded by an allelic variant of a gene.

The terms "amino-terminal" and "carboxyl-terminal" are used herein to denote positions within polypeptides. Where the context allows, these terms are used with reference to a particular sequence or portion of a polypeptide to denote proximity or relative position. For example, a certain sequence positioned carboxyl-terminal to a reference sequence within a polypeptide is located proximal to the carboxyl terminus of the reference sequence, but is not necessarily at the carboxyl terminus of the complete polypeptide.

The term "complement/anti-complement pair" denotes non-identical moieties that form a non-covalently associated, stable pair under appropriate conditions. For instance, biotin and avidin (or streptavidin) are prototypical members of a complement/anti-complement pair. Other exemplary complement/anti-complement pairs include receptor/ligand pairs, antibody/antigen (or hapten or epitope) pairs, sense/antisense polynucleotide pairs, and the like. Where subsequent dissociation of the complement/anti-complement pair is desirable, the complement/anti-complement pair preferably has a binding affinity of <109 M-1.

The term "complements of a polynucleotide molecule" denotes a polynucleotide molecule having a complementary base sequence and reverse orientation as compared to a reference sequence. For example, the sequence 5' ATGCACGGG 3' is complementary to 5' CCCGTGCAT 3'.

The term "contig" denotes a polynucleotide that has a contiguous stretch of identical or complementary sequence to another polynucleotide. Contiguous sequences are said to "overlap" a given stretch of polynucleotide sequence either in their entirety or along a partial stretch of the polynucleotide. For example, representative contigs to the polynucleotide sequence 5'-ATGGCTTAGCTT-3' are 5'-TAGCTTgagtct-3' and 3'-gtcgacTACCGA-5'.

The term "degenerate nucleotide sequence" denotes a sequence of nucleotides that includes one or more degenerate codons (as compared to a reference polynucleotide molecule that encodes a polypeptide). Degenerate codons contain different triplets of nucleotides, but encode the same amino acid residue (i.e., GAU and GAC triplets each encode Asp).

The term "expression vector" is used to denote a DNA molecule, linear or circular, that comprises a segment encoding a polypeptide of interest operably linked to additional segments that provide for its transcription. Such additional segments include promoter and terminator sequences, and may also include one or more origins of replication, one or more selectable markers, an enhancer, a polyadenylation signal, etc. Expression vectors are generally derived from plasmid or viral DNA, or may contain elements of both.

The term "isolated", when applied to a polynucleotide, denotes that the polynucleotide has been removed from its natural genetic milieu and is thus free of other extraneous or unwanted coding sequences, and is in a form suitable for use within genetically engineered protein production systems. Such isolated molecules are those that are separated from their natural environment and include cDNA and genomic clones. Isolated DNA molecules of the present invention are free of other genes with which they are ordinarily associated, but may include naturally occurring 5' and 3' untranslated regions such as promoters and terminators. The identification of associated regions will be evident to one of ordinary skill in the art (see for example, Dynan and Tijan, Nature 316:774-78, 1985).

An "isolated" polypeptide or protein is a polypeptide or protein that is found in a condition other than its native environment, such as apart from blood and animal tissue. In a preferred form, the isolated polypeptide is substantially free of other polypeptides, particularly other polypeptides of animal origin. It is preferred to provide the polypeptides in a highly purified form, i.e., greater than 95% pure, more preferably greater than 99% pure. When used in this context, the term "isolated" does not exclude the presence of the same polypeptide in alternative physical forms, such as dimers or alternatively glycosylated or derivatized forms.

The term "neoplastic", when referring to cells, indicates cells undergoing new and abnormal proliferation, particularly in a tissue where in the proliferation is uncontrolled and progressive, resulting in a neoplasm. The neoplastic cells can be either malignant, i.e., invasive and metastatic, or benign.

The term "operably linked", when referring to DNA segments, indicates that the segments are arranged so that they function in concert for their intended purposes, e.g., transcription initiates in the promoter and proceeds through the coding segment to the terminator.

The term "ortholog" denotes a polypeptide or protein obtained from one species that is the functional counterpart of a polypeptide or protein from a different species. Sequence differences among orthologs are the result of speciation.

"Paralogs" are distinct but structurally related proteins made by an organism. Paralogs are believed to arise through gene duplication. For example, α-globin, β-globin, and myoglobin are paralogs of each other.

A "polynucleotide" is a single- or double-stranded polymer of deoxyribonucleotide or ribonucleotide bases read from the 5' to the 3' end. Polynucleotides include RNA and DNA, and may be isolated from natural sources, synthesized in vitro, or prepared from a combination of natural and synthetic molecules. Sizes of polynucleotides are expressed as base pairs (abbreviated "bp"), nucleotides ("nt"), or kilobases ("kb"). Where the context allows, the latter two terms may describe polynucleotides that are single-stranded or double-stranded. When the term is applied to double-stranded molecules it is used to denote overall length and will be understood to be equivalent to the term "base pairs". It will be recognized by those skilled in the art that the two strands of a double-stranded polynucleotide may differ slightly in length and that the ends thereof may be staggered as a result of enzymatic cleavage; thus all nucleotides within a double-stranded polynucleotide molecule may not be paired.

A "polypeptide" is a polymer of amino acid residues joined by peptide bonds, whether produced naturally or synthetically. Polypeptides of less than about 10 amino acid residues are commonly referred to as "peptides".

The term "promoter" is used herein for its art-recognized meaning to denote a portion of a gene containing DNA sequences that provide for the binding of RNA polymerase and initiation of transcription. Promoter sequences are commonly, but not always, found in the 5' non-coding regions of genes.

A "protein" is a macromolecule comprising one or more polypeptide chains. A protein may also comprise non-peptidic components, such as carbohydrate groups. Carbohydrates and other non-peptidic substituents may be added to a protein by the cell in which the protein is produced, and will vary with the type of cell. Proteins are defined herein in terms of their amino acid backbone structures; substituents such as carbohydrate groups are generally not specified, but may be present nonetheless.

The term "receptor" denotes a cell-associated protein that binds to a bioactive molecule (i.e., a ligand) and mediates the effect of the ligand on the cell. Membrane-bound receptors are characterized by a multi-peptide structure comprising an extracellular ligand-binding domain and an intracellular effector domain that is typically involved in signal transduction. Binding of ligand to receptor results in a conformational change in the receptor that causes an interaction between the effector domain and other molecule(s) in the cell. This interaction in turn leads to an alteration in the metabolism of the cell. Metabolic events that are linked to receptor-ligand interactions include gene transcription, phosphorylation, dephosphorylation, increases in cyclic AMP production, mobilization of cellular calcium, mobilization of membrane lipids, cell adhesion, hydrolysis of inositol lipids and hydrolysis of phospholipids. In general, receptors can be membrane bound, cytosolic or nuclear; monomeric (e.g., thyroid stimulating hormone receptor, beta-adrenergic receptor) or multimeric (e.g., PDGF receptor, growth hormone receptor, IL-3 receptor, GM-CSF receptor, G-CSF receptor, erythropoietin receptor and IL-6 receptor).

The term "secretory signal sequence" denotes a DNA sequence that encodes a polypeptide (a "secretory peptide") that, as a component of a larger polypeptide, directs the larger polypeptide through a secretory pathway of a cell in which it is synthesized. The larger polypeptide is commonly cleaved to remove the secretory peptide during transit through the secretory pathway.

The term "splice variant" is used herein to denote alternative forms of RNA transcribed from a gene. Splice variation arises naturally through use of alternative splicing sites within a transcribed RNA molecule, or less commonly between separately transcribed RNA molecules, and may result in several mRNAs transcribed from the same gene. Splice variants may encode polypeptides having altered amino acid sequence. The term splice variant is also used herein to denote a protein encoded by a splice variant of an mRNA transcribed from a gene.

Molecular weights and lengths of polymers determined by imprecise analytical methods (e.g., gel electrophoresis) will be understood to be approximate values. When such a value is expressed as "about" X or "approximately" X, the stated value of X will be understood to be accurate to ±10%.

The present invention provides expression vectors and methods for producing recombinant IL-31 protein from a prokaryotic host (as defined in the claims) and is based in part upon the discovery of compositions and methods to produce homogeneous preparations of IL-31. IL-31 is a recently discovered protein having the structure of a four-helical-bundle cytokine. This cytokine was previously identified as IL-31 and is fully described in co-assigned U.S. Patent Application No. 10/352,554, filed January 21, 2003. See published U.S. Patent Application No. 2003-0224487, and PCT application WO 03/060090. See also, Dillon, et al., Nautre Immunol. 5:752-760, 2004. IL-31 is a ligand with high specificity for the receptor IL-31RA and at least one additional subunit comprising OncostatinM receptor beta (OSMRbeta). The native polynucleotide and polypeptide sequences for human IL-31 are shown in SEQ ID NOs: 1 and 2, respectively. SEQ ID NO:3 shows the degenerate polynucleotide for the polypeptide having the amino acid sequence as shown in SEQ ID NO:2. The native polynucleotide and polypeptide sequences for mouse IL-31 are shown in SEQ ID NOs: 4 and 5, respectively. SEQ ID NO:6 shows the degenerate polynucleotide for the polypeptide having the amino acid sequence as shown in SEQ ID NO:5. The native polynucleotide and polypeptide sequences for human IL-31RA are shown in SEQ ID NOs: 7 and 8, respectively. The native polynucleotide and polypeptide sequences for mouse IL-31RA are shown in SEQ ID NOs: 9 and 10, respectively. The native polynucleotide and polypeptide sequences for human OSMRbeta are shown in SEQ ID NOs: 11 and 12, respectively.

Both the murine and human forms of IL-31 are known to have an odd number of cysteines. (PCT application WO 03/060090 and Dillon, et al., supra.) Expression of recombinant IL-31 can result in a heterologous mixture of proteins composed of intramolecular disulfide binding in multiple conformations. The separation of these forms can be difficult and laborious. It is therefore desirable to provide IL-31 molecules having a single intramolecular disulfide bonding pattern upon expression and methods for refolding and purifying these preparations to maintain homogeneity.

In particular, the expression vectors and methods described herein comprise an E. coli expression system. Using the expression vectors described herein significantly improved the yield of recombinant protein recovered from the bacteria.

The present specification describes polynucleotide molecules, including DNA and RNA molecules, that encode Cysteine mutants of IL-31 that result in expression of a recombinant IL-31 preparation that is a homogeneous preparation. For the purposes of this invention, a homogeneous preparation of IL-31 is a preparation which comprises at least 98% of a single intramolecular disulfide bonding pattern in the purified polypeptide. In other embodiments, the single disulfide conformation in a preparation of purified polypeptide is at 99% homogeneous. In general, these Cysteine mutants will maintain some biological activity of the wildtype IL-31, as described herein. For example, the molecules of the present invention can bind to the IL-31 receptor with some specificity. Generally, a ligand binding to its cognate receptor is specific when the KD falls within the range of 100 nM to 100 pM. Specific binding in the range of 100 mM to 10 nM KD is low affinity binding. Specific binding in the range of 2.5 pM to 100 pM KD is high affinity binding. In another example, biological activity of IL-31 Cysteine mutants is present when the molecules are capable of some level of activity associated with wildtype IL-31as described in detail herein.

When referring to native IL-31, the term shall mean IL-31 and zeytor17lig. When referring to IL-31RA, the term shall mean IL-31RA and zcytor17.

The present specification describes methods for recovering recombinant IL-31 protein from a prokaryotic host when the IL-31 protein is expressed by the host and found within the host cell as an unglycosylated, insoluble inclusion body. When the prokaryotic cell is lysed to isolate the inclusion bodies (also called refractile bodies), the inclusion bodies are aggregates of IL-31. Therefore, the inclusion bodies must be disassociated and dissolved to isolate the IL-31 protein, and generally this requires the use of a denaturing chaotropic solvent, resulting in recovering a polypeptide that must be refolded to have significant biological activity. Once the IL-31 protein is refolded, the protein must be captured and purified. Thus, the present specification describes methods for isolating insoluble IL-31 protein from prokaryotic cells, dissolving the insoluble IL-31 protein material in a chaotropic solvent, diluting the chaotropic solvent in such a manner that the IL-31 protein is refolded and isolated. The present specification describes methods for capturing the renatured IL-31 from the dilute refold buffer using cation exchange chromatography, and purifying the refolded IL-31 protein using hydrophobic interaction chromatography. Further purification is achieved using anion exchange in binding assays using an IL-31 receptor and the like.

The present invention provides mutations in the IL-31 wildtype sequences as shown in SEQ ID NOs: 1, 2, 3, 4, 5, and 6, that result in expression of single forms of the IL-31 molecule. Because the heterogeneity of forms is believed to be a result of multiple intramolecular disulfide bonding patterns, specific embodiments of the present invention includes mutations to the cysteine residues within the wildtype IL-31 sequences. The mature human IL-31 polypeptide is shown in SEQ ID NO:13, with SEQ ID NO:49 showing the mature human IL-31 polypeptide with a start methionine. Molecules of the mature human IL-31 polypeptide can have disulfide bonds between the cysteine residue at position 46 and position 107 of SEQ ID NO:13, between position 46 and 121 of SEQ ID NO:13, and between position 107 and 121 of SEQ ID NO:13. A mutation of any of these three cysteines results in a mutant form of the human IL-31 protein that will only form one disulfide bond. Thus a mutation at postion 46 will result in a protein that forms a disulfide bond between position 107 and position 121 of SEQ ID NO:13; a mutation at position 107 will result in a protein that forms a disulfide bond between position 46 and position 121 of SEQ ID NO:13; and a mutation at position 121 will result in a protein that forms a disulfide bond between position 46 and position 107 of SEQ ID NO:13. The cysteines in these positions can be mutated, for example, to a serine, alanine, threonine, valine, or asparagine. For example, a human IL-31 protein having a mutation from cysteine to serine at position 46 of SEQ ID NO:13 is shown in SEQ ID NO:14; a human IL-31 protein having a mutation from cysteine to serine at position 107 of SEQ ID NO:13 is shown in SEQ ID NO:15; a human IL-31 protein having a mutation from cysteine to serine at position 121 of SEQ ID NO:13 is shown in SEQ ID NO:16.

When human IL-31 is expressed in E. coli, an N-terminal or amino-terminal Methionine is present. SEQ ID NOs: 17-19, for example, show the nucleotide and amino acid residue sequences for IL-31 when the N-terminal Met is present in these mutants.

Similar mutations can be made to the mouse IL-31 polypeptide sequence. The mature mouse IL-31 polypeptide is shown in SEQ ID NO:20. Molecules of the mature murine IL-31 polypeptide can have disulfide bonds between the cysteine residue at position 44 and position 87 of SEQ ID NO:20, between position 44 and 107 of SEQ ID NO:20, between position 44 and 121 of SEQ ID NO:20; between position 44 and 133 of SEQ ID NO:20; between position 87 and 107of SEQ ID NO:20; between position 87 and 121 of SEQ ID NO:20; between position 87 and 133 of SEQ ID NO:20; between position 107 and 121 of SEQ ID NO:20; between position 107 and 133 of SEQ ID NO:20; and between position 121 and 133 of SEQ ID NO:20. A mutation of any of these cysteines results in a mutant form of the mouse IL-31 protein. The cysteines in these positions can be mutated, for example, to a serine, alanine, threonine, valine, or asparagine. For example, a mouse IL-31 protein having a mutation from cysteine to serine at position 44 of SEQ ID NO:20 is shown in SEQ ID NO:21; a mouse IL-31 protein having a mutation from cysteine to serine at position 87 of SEQ ID NO:20 is shown in SEQ ID NO:22; a mouse IL-31 protein having a mutation from cysteine to serine at position 107 of SEQ ID NO:20 is shown in SEQ ID NO:23; a mouse IL-31 protein having a mutation from cysteine to serine at position 121 of SEQ ID NO:20 is shown in SEQ ID NO:24; and a mouse IL-31 protein having a mutation from cysteine to serine at position 133 of SEQ ID NO:20 is shown in SEQ ID NO:25.

When mouse IL-31 is expressed in E. coli, an N-terminal or amino-terminal Methionine is present. SEQ ID NOs:26-30, for example, show the nucleotide and amino acid residue sequences for IL-31 when the N-terminal Met is present in these mutants. When the mouse IL-31 Cys mutants of the present invention were made in E. coli with serine at position 107 of SEQ ID NO: 20, the purified N-terminus was determined to begin at the phenylalanine (Phe) instead of the alanine. Thus, one embodiment of the invention is the polypeptide comprising or consisting of the amino acid sequence from position 2 (Phe) to position 133 (Cys) of SEQ ID NO: 23.

The polynucleotide and polypeptide molecules of the present invention have a mutation at one or more of the cysteines present in the native IL-31 molecules, yet retain some biological activity as described herein. When referring to the cysteine mutants of IL-31, the term shall mean any of the mutated forms of IL-31 described above, as shown in any of SEQ ID NOs: 15, 18, 23 or 28, generally referred to as IL-31Cys mutants.

A cell line that is dependent on the OSMRbeta and zcytor17 receptor linked pathway for survival and growth in the absence of other growth factors can be used to measure the activity of the IL-31 Cys mutants described herein. The preferred growth factor-dependent cell line that can be used for transfection and expression of IL-31 receptor is BaF3 (Palacios and Steinmetz, Cell 41: 727-734, 1985; Mathey-Prevot et al., Mol. Cell. Biol. 6: 4133-4135, 1986). However, other growth factor-dependent cell lines, such as FDC-P1 (Hapel et.al., Blood 64: 786-790, 1984), and MO7e (Kiss et al., Leukemia 7: 235-240, 1993) are suitable for this purpose.

One of ordinary skill in the art will appreciate that different species can exhibit "preferential codon usage." In general, see, Grantham, et al., Nuc. Acids Res. 8:1893-912, 1980; Haas, et al. Curr. Biol. 6:315-24, 1996; Wain-Hobson, et al., Gene 13:355-64, 1981; Grosjean and Fiers, Gene 18:199-209, 1982; Holm, Nuc. Acids Res. 14:3075-87, 1986; Ikemura, J. Mol. Biol. 158:573-97, 1982. As used herein, the term "preferential codon usage" or "preferential codons" is a term of art referring to protein translation codons that are most frequently used in cells of a certain species, thus favoring one or a few representatives of the possible codons encoding each amino acid. For example, the amino acid Threonine (Thr) may be encoded by ACA, ACC, ACG, or ACT, but in mammalian cells ACC is the most commonly used codon; in other species, for example, insect cells, yeast, viruses or bacteria, different Thr codons may be preferential. Preferential codons for a particular species can be introduced into the polynucleotides of the present invention by a variety of methods known in the art. Introduction of preferential codon sequences into recombinant DNA can, for example, enhance production of the protein by making protein translation more efficient within a particular cell type or species. Therefore, the degenerate codon sequence disclosed in SEQ ID NO:3 serves as a template for optimizing expression of polynucleotides in various cell types and species commonly used in the art and disclosed herein. Sequences containing preferential codons can be tested and optimized for expression in various species, and tested for functionality as disclosed herein.

As previously noted, the isolated polynucleotides described herein include DNA and RNA. Methods for preparing DNA and RNA are well known in the art. In general, RNA is isolated from a tissue or cell that produces large amounts of IL-31 RNA. Such tissues and cells are identified by Northern blotting (Thomas, Proc. Natl. Acad. Sci. USA 77:5201, 1980), or by screening conditioned medium from various cell types for activity on target cells or tissue. Once the activity or RNA producing cell or tissue is identified, total RNA can be prepared using guanidinium isothiocyanate extraction followed by isolation by centrifugation in a CsCl gradient (Chirgwin et al., Biochemistry 18:52-94, 1979). Poly (A)+ RNA is prepared from total RNA using the method of Aviv and Leder (Proc. Natl. Acad. Sci. USA 69:1408-12, 1972). Complementary DNA (cDNA) is prepared from poly(A)+ RNA using known methods. In the alternative, genomic DNA can be isolated. Polynucleotides encoding IL-31 polypeptides are then identified and isolated by, for example, hybridization or PCR.

Variant IL-31 polypeptides or polypeptides with substantially similar sequence identity are characterized as having one or more amino acid substitutions, deletions or additions. These changes are preferably of a minor nature, that is conservative amino acid substitutions and other substitutions that do not significantly affect the folding or activity of the polypeptide; small deletions, typically of one to about 30 amino acids; and amino- or carboxyl-terminal extensions, such as an amino-terminal methionine residue, a small linker peptide of up to about 20-25 residues, or an affinity tag. The present specification describes polypeptides of from about 108 to 216 amino acid residues that comprise a sequence that is at least 70%, at least 80%, at least 90%, at least 95%, at least 96%, at least 97%, at least 98%, at least 99%, or greater than 99% identical to the corresponding region of SEQ ID NO:2. Polypeptides comprising affinity tags can further comprise a proteolytic cleavage site between the IL-31 polypeptide and the affinity tag. Preferred such sites include thrombin cleavage sites and factor Xa cleavage sites.

A Hopp/Woods hydrophilicity profile of the IL-31 protein sequence as shown in SEQ ID NOs: 15-30, and SEQ ID NO:49 can be generated (Hopp et al., Proc. Natl. Acad. Sci.78:3824-3828, 1981; Hopp, J. Immun. Meth. 88:1-18, 1986 and Triquier et al., Protein Engineering 11:153-169, 1998). The profile is based on a sliding six-residue window. Buried G, S, and T residues and exposed H, Y, and W residues were ignored.

In addition, the proteins of the present invention (or polypeptide fragments thereof) can be joined to other bioactive molecules, particularly other cytokines, to provide multi-functional molecules. For example, one or more helices from IL-31can be joined to other cytokines to enhance their biological properties or efficiency of production.

The present specification describes a series of novel, hybrid molecules in which a segment comprising one or more of the helices of IL-31 is fused to another polypeptide. Fusion is preferably done by splicing at the DNA level to allow expression of chimeric molecules in recombinant production systems. The resultant molecules are then assayed for such properties as improved solubility, improved stability, prolonged clearance half-life, improved expression and secretion levels, and pharmacodynamics. Such hybrid molecules may further comprise additional amino acid residues (e.g., a polypeptide linker) between the component proteins or polypeptides.

Non-naturally occurring amino acids include, without limitation, trans-3-methylproline, 2,4-methanoproline, cis-4-hydroxyproline, trans-4-hydroxyproline, N-methylglycine, allo-threonine, methylthreonine, hydroxyethylcysteine, hydroxyethylhomocysteine, nitroglutamine, homoglutamine, pipecolic acid, thiazolidine carboxylic acid, dehydroproline, 3- and 4-methylproline, 3,3-dimethylproline, tert-leucine, norvaline, 2-azaphenylalanine, 3-azaphenylalanine, 4-azaphenylalanine, and 4-fluorophenylalanine. Several methods are known in the art for incorporating non-naturally occurring amino acid residues into proteins. For example, an in vitro system can be employed wherein nonsense mutations are suppressed using chemically aminoacylated suppressor tRNAs. Methods for synthesizing amino acids and aminoacylating tRNA are known in the art. Transcription and translation of plasmids containing nonsense mutations is typically carried out in a cell-free system comprising an E. coli S30 extract and commercially available enzymes and other reagents. Proteins are purified by chromatography. See, for example, Robertson et al., J. Am. Chem. Soc. 113:2722 (1991), Ellman et al., Methods Enzymol. 202:301 (1991), Chung et al., Science 259:806 (1993), and Chung et al., Proc. Nat'l Acad. Sci. USA 90:10145 (1993).

In a second method, translation is carried out in Xenopus oocytes by microinjection of mutated mRNA and chemically aminoacylated suppressor tRNAs (Turcatti et al., J. Biol. Chem. 271:19991 (1996)). Within a third method, E. coli cells are cultured in the absence of a natural amino acid that is to be replaced (e.g., phenylalanine) and in the presence of the desired non-naturally occurring amino acid(s) (e.g., 2-azaphenylalanine, 3-azaphenylalanine, 4-azaphenylalanine, or 4-fluorophenylalanine). The non-naturally occurring amino acid is incorporated into the protein in place of its natural counterpart. See, Koide et al., Biochem. 33:7470 (1994). Naturally occurring amino acid residues can be converted to non-naturally occurring species by in vitro chemical modification. Chemical modification can be combined with site-directed mutagenesis to further expand the range of substitutions (Wynn and Richard, Protein Sci. 2:395 (1993). It may be advantageous to stabilize IL-31 and IL-31Cys mutants to extend the half-life of the molecule, particularly for extending metabolic persistence in an active state. To achieve extended half-life, IL-31 and IL-31Cys mutants molecules can be chemically modified using methods described herein. PEGylation is one method commonly used that has been demonstrated to increase plasma half-life, increased solubility, and decreased antigenicity and immunogenicity (Nucci et al., Advanced Drug Delivery Reviews 6:133-155, 1991 and Lu et al., Int. J. Peptide Protein Res. 43:127-138, 1994).

A limited number of non-conservative amino acids, amino acids that are not encoded by the genetic code, non-naturally occurring amino acids, and unnatural amino acids may be substituted for IL-31 and IL-31Cys mutant amino acid residues.

In general, a DNA sequence encoding a IL-31 and IL-31Cys mutants polypeptide can be operably linked to other genetic elements required for its expression, generally including a transcription promoter and terminator, within an expression vector. The vector will also commonly contain one or more selectable markers and one or more origins of replication, although those skilled in the art will recognize that within certain systems selectable markers may be provided on separate vectors, and replication of the exogenous DNA may be provided by integration into the host cell genome. Selection of promoters, terminators, selectable markers, vectors and other elements is a matter of routine design within the level of ordinary skill in the art. Many such elements are described in the literature and are available through commercial suppliers.

Expression vectors that are suitable for production of a desired protein in prokaryotic cells typically comprise (1) prokaryotic DNA elements coding for a bacterial origin for the maintenance of the expression vector in a bacterial host; (2) DNA elements that control initiation of transcription, such as a promoter; (3) DNA elements that control the processing of transcripts, such as a transcriptional terminator, and (4) a gene encoding a selectable marker, such as antibiotic resistance. The prokaryotic host cell produces IL-31 upon introduction of an expression vector. Accordingly, the present invention contemplates expression vectors comprising a promoter, the IL-31 nucleotide sequence, and a terminator sequence. In another embodiment, the expression vector further comprises a selectable marker. In one embodiment, the selectable marker is kanamycin resistance.

Expression vectors can also comprise nucleotide sequences that encode a peptide tag to aid in purification of the desired protein. Peptide tags that are useful for isolating recombinant polypeptides include, for example, polyHistidine tags (which have an affinity for nickel-chelating resin), c-myc tags, calmodulin binding protein (isolated with calmodulin affinity chromatography), substance P, the RYIRS tag (which binds with anti-RYIRS antibodies), the Glu-Glu tag, and the FLAG tag (which binds with anti-FLAG antibodies). See, for example, Luo et al., Arch. Biochem. Biophys. 329:215 (1996), Morganti et al., Biotechnol. Appl. Biochem. 23:67 (1996), and Zheng et al., Gene 186:55 (1997). Nucleic acid molecules encoding such peptide tags are available, for example, from Sigma-Aldrich Corporation (St. Louis, MO).

One of ordinary skill in the art will be familiar with a multitude of molecular techniques for the preparation of the expression vector. For example, the IL-31 polynucleotide can be prepared by synthesizing nucleic acid molecules using mutually priming, long oligonucleotides and the nucleotide sequences described herein (see, for example, Ausubel (1995) at pages 8-8 to 8-9). Established techniques using the polymerase chain reaction provide the ability to synthesize DNA molecules at least two kilobases in length (Adang et al., Plant Molec. Biol. 21:1131 (1993), Bambot et al., PCR Methods and Applications 2:266 (1993), Dillon et al., "Use of the Polymerase Chain Reaction for the Rapid Construction of Synthetic Genes," in Methods in Molecular Biology, Vol. 15: PCR Protocols: Current Methods and Applications, White (ed.), pages 263-268, (Humana Press, Inc. 1993), and Holowachuk et al., PCR Methods Appl. 4:299 (1995)).

Another method for constructing expression systems utilizes homologous recombination using a yeast system. See U.S. Patent No. 6,207,442, Plasmid Construction by Homologous Recombination. The system provides a universal acceptor plasmid that can be used to clone a DNA encoding any polypeptide of interest, including polypeptide fusions. The system provides methods for preparing double stranded, circular DNA molecules comprising a region encoding a protein of interest. One or more donor DNA fragments encoding the protein of interest, i.e., IL-31, are combined with an acceptor plasmid, a first DNA linker, and a second DNA linker in a Saccharomyces cerevisiae host cell whereby the donor DNA fragment is joined to the acceptor plasmid by homologous recombination of the donor DNA, acceptor plasmid, and linkers to form the closed, circular plasmid.

The nucleic acid molecules described herein can also be synthesized with "gene machines" using protocols such as the phosphoramidite method. If chemically-synthesized, double stranded DNA is required for an application such as the synthesis of a gene or a gene fragment, then each complementary strand is made separately. The production of short genes (60 to 80 base pairs) is technically straightforward and can be accomplished by synthesizing the complementary strands and then annealing them. For the production of longer genes (>300 base pairs), however, special strategies may be required, because the coupling efficiency of each cycle during chemical DNA synthesis is seldom 100%. To overcome this problem, synthetic genes (double-stranded) are assembled in modular form from single-stranded fragments that are from 20 to 100 nucleotides in length. For reviews on polynucleotide synthesis, see, for example, Glick and Pasternak, Molecular Biotechnology, Principles and Applications of Recombinant DNA (ASM Press 1994), Itakura et al., Annu. Rev. Biochem. 53:323 (1984), and Climie et al., Proc. Nat'l Acad. Sci. USA 87:633 (1990).

Examples of alternate techniques that can be used to prepare the IL-31 gene and expression vector include, for example, restriction endonuclease digestion and ligation, and polymerase chain reaction, all of which are well known in the art.

A wide variety of selectable marker genes is available (see, for example, Kaufman, Meth. Enzymol. 185:487 (1990); Kaufman, Meth. Enzymol. 185:537 (1990)). It is common for expression vectors to comprise selection markers, such as tetracycline resistance, amplicillin resistance, kanamycin resistance, neomycin resistance, or chlormaphenicol resistance. A selectable marker will permit selection and/or detection of cells that have been transformed with expression vector from cells that have not been transformed. An expression vector can carry more than one such antibiotic resistance gene. An example of selectable marker without antibiotic resistance uses the hok/sok system from plasmid R1. The hok gene encodes the toxic Hok protein of 52 amino acids and the sok gene encodes an antisense RNA, which is complementary to the hok mRNA leader sequence. This selectable marker is known to one skilled in the art and is described in more detail by Gerdes, K. et al., Genetic Engineering, 19:49-61, 1997.

A wide variety of suitable recombinant host cells is encompassed by the present invention and includes, but is not limited to, gram-negative prokaryotic host organisms. Suitable strains of E. coli include W3110, K12-derived strains MM294, TG-1, JM-107, BL21, and UT5600. Other suitable strains include: BL21(DE3), BL21(DE3)pLysS, BL21(DE3)pLysE, DH1, DH4I, DH5, DH5I, DHSIF', DH5IMCR, DH10B, DH10B/p3, DH11S, C600, HB101, JM101, JM105, JM109, JM110, K38, RR1, Y1088, Y1089, CSH18, ER1451, ER1647, E. coli K12, E. coli K12 RV308, E. coli K12 C600, E. coliHB101, E. coli K12 C600 R.sub.k-M.sub.k-, E. coli K12 RR1 (see, for example, Brown (ed.), Molecular Biology Labfax (Academic Press 1991)). Other gram-negative prokaryotic hosts can include Serratia, Pseudomonas, Caulobacter. Prokaryotic hosts can include gram-positive organisms such as Bacillus, for example, B. subtilis and B. thuringienesis, and B. thuringienesis var. israelensis, as well as Streptomyces, for example, S. lividans, S. ambofaciens, S. fradiae, and S. griseofuscus. Suitable strains of Bacillus subtilus include BR151, YB886, MI119, MI120, and B170 (see, for example, Hardy, "Bacillus Cloning Methods," in DNA Cloning: A Practical Approach, Glover (ed.) (IRL Press 1985)). Standard techniques for propagating vectors in prokaryotic hosts are well-known to those of skill in the art (see, for example, Ausubel et al. (eds.), Short Protocols in Molecular Biology, 3rd Edition (John Wiley & Sons 1995); Wu et al., Methods in Gene Biotechnology (CRC Press, Inc. 1997)). For an overview of protease deficient strains in prokaryotes, see, Meerman et al., Biotechnology 12:1107-1110, 1994. The present invention is exemplified using the W3110 strain, which has been deposited at the American Type Culture Collection (ATCC) as ATCC # 27325.

Techniques for manipulating cloned DNA molecules and introducing exogenous DNA into a variety of host cells are disclosed by Sambrook et al., Molecular Cloning: A Laboratory Manual, 2nd ed., Cold Spring Harbor Laboratory Press, Cold Spring Harbor, NY, 1989, and Ausubel et al., eds., Current Protocols in Molecular Biology, John Wiley and Sons, Inc., NY, 1987. Transformed or transfected host cells are cultured according to conventional procedures in a culture medium containing nutrients and other components required for the growth of the chosen host cells. A variety of suitable media, including defined media and complex media, are known in the art and generally include a carbon source, a nitrogen source, essential amino acids, vitamins and minerals. Media may also contain such components as growth factors or serum, as required. The growth medium will generally select for cells containing the exogenously added DNA by, for example, drug selection or deficiency in an essential nutrient that is complemented by the selectable marker carried on the expression vector or co-transfected into the host cell. Liquid cultures are provided with sufficient aeration by conventional means, such as shaking of small flasks or sparging of fermentors. Transformed cells can be selected and propagated to provide recombinant host cells that express the gene of interest. IL-31 can be expressed in E. coli using the MBP (maltose binding protein) fusion system (New England Biolabs (NEB; Beverly, MA)). In this system, the IL-31 cDNA is attached to the 3' end of the malE gene to form an MBP-IL-31 fusion protein. Fusion protein expression is driven by the tac promoter and is "off" until the promoter is induced by addition of 1 mmol IPTG (isopropyl b-thiogalactosylpyranoside). The constructs can be built as in-frame fusions with MBP in accordance with the Multiple Cloning Site (MCS) of the pMAL-c2 vector (NEB), and according to the manufacturer's specifications.

Fermentation of proteins from prokaryotic hosts is well known to one of ordinary skill in the art. Following fermentation the cells are harvested by centrifugation, re-suspended in homogenization buffer and homogenized, for example, in an APV-Gaulin homogenizer (Invensys APV, Tonawanda, New York) or other type of cell disruption equipment, such as bead mills and sonicators. Alternatively, the cells are taken directly from the fermentor and homogenized in an APV-Gaulin homogenizer. Alternatively, the fermentation broth may be diluted with water or buffer prior to homogenization and recovery of IL-31 or IL-31 Cys mutants.

Additionally, the methods of recovering IL-31 can comprise a further step of precipitating, washing, and resolubilizing the IL-31. The washed inclusion bodies are solubilized in 6 M guanidine or 8 M urea, diluted 6-10 fold in water or buffer, incubated 30 minutes, and centrifuged or filtered. Alternatively, ultrafiltration or macrofiltration can be used wash inclusion bodies after homogenization. The resulting precipitate is washed in 2-6 M urea, and contains the IL-31 protein. The precipatate is then washed with water prior to solublization. Addition of Al3+ or Fe3+ or anionic and cationic polymers or agents such as spermine, PEI and benzonase may be added to precipitate cell debris, soluble proteins, DNA, RNA, and carbohydrates.

The washed inclusion body prep can be solubilized using guanidine hydrochloride (5-8 M), guanidine thiocyanate (5-6 M), or urea (7 - 8 M) containing a reducing agent such as beta mercaptoethanol (10 - 100 mM), or dithiothreitol (5-50 mM). The solutions can be prepared in Tris, phopshate, HEPES or other appropriate buffers. Inclusion bodies can also be solubilized with urea (2-4 M) containing sodium lauryl sulfate (0.1-2%). Inclusion bodies from 1 liter of fermentation broth can be solubilized using 50 - 200 ml of the described solutions. The one method provides solubilizing the inclusion body pellets from 1 liter of fermentation broth in 150 ml of 6 M GuHCl prepared in 100 mM Tris, pH 8.0, containing 40 mM DTT. In another embodiment, an inclusion body slurry is mixed with 50-100 ml 8 M GuHCL. The slurry is re-suspended by mixing with a spatula followed by homogenization with an Omni EZ homogenizer (Omni International, Warrenton, VA) or mixing with a mechanical device. The suspension is mixed for 30 - 120 minutes, at 3-37°C. In one embodiment, the suspension is mixed at 15-25°C, to finish the solubilization process. The sample is then centrifuged at 7,500 - 16,000 x G at 4°C for 10 -30 minutes using an appropriate centrifuge. The supernatant sample containing the solubilized IL-31 is decanted and retained, and the concentration of OL-31 in the solubilized fraction is determined

In one aspect of the process for recovering purified IL-31 from transformed E. coli host strains in which the IL-31 is expressed as refractile inclusion bodies, the cells are disrupted and the inclusion bodies are recovered by processes well known in the art.

Refolding can also be done in the presence of EDTA to decrease methionine oxidation, or on a size exclusion column, or using tangential flow filtration, or electrodialysis.

Purification of IL-31 to remove the remaining impurities and contaminants may be desirable. For example, an anion exchange column can be used to reduce the endotoxin level. IL-31 is diluted to a conductivity level of < 10 mS/cm and the pH is adjusted to 8.0. It is applied to a Q Sepharose FF column (Amersham Biosciences) which has been equilibrated to 20 mM Tris, pH 8.0. The IL-31 passes through the column and has an approximately 80 % reduction in endotoxin compared to the load. Mustang Q or Mustang E (Pall, Port Washington, NY) membranes can also be used to reduce endotoxin levels between pH 5.0 and 9.0.

Other purification steps that could potentially be used to further purify IL-31 include metal chelate chromatography, anion exchange chromatography, or hydrophobic interaction chromatography on a phenyl column. It is also possible to carry out purification prior to refolding the IL-31, using for example reversed phase HPLC, ion exchange chromatography or metal chelate chromatography. Thus, the present specification describes methods comprising the additional steps of purification disclosed herein.

The present specification describes polypeptide fragments or peptides comprising an epitope-bearing portion of a IL-31 and IL-31Cys mutants polypeptide described herein. Such fragments or peptides may comprise an "immunogenic epitope," which is a part of a protein that elicits an antibody response when the entire protein is used as an immunogen. Immunogenic epitope-bearing peptides can be identified using standard methods (see, for example, Geysen et al., Proc. Nat'l Acad. Sci. USA 81:3998 (1983)).

In contrast, polypeptide fragments or peptides may comprise an "antigenic epitope," which is a region of a protein molecule to which an antibody can specifically bind. Certain epitopes consist of a linear or contiguous stretch of amino acids, and the antigenicity of such an epitope is not disrupted by denaturing agents. It is known in the art that relatively short synthetic peptides that can mimic epitopes of a protein can be used to stimulate the production of antibodies against the protein (see, for example, Sutcliffe et al., Science 219:660 (1983)). Accordingly, antigenic epitope-bearing peptides and polypeptides of the present invention are useful to raise antibodies (e.g., neutralizing antibodies) that bind with the polypeptides described herein. Hopp/Woods hydrophilicity profiles can be used to determine regions that have the most antigenic potential (Hopp et al., 1981, ibid. and Hopp, 1986, ibid.). For example, in human IL-31, hydrophilic regions include amino acid residues 54-59 of SEQ ID NO:49, amino acid residues 129-134 of SEQ ID NO:49, amino acid residues 53-58 of SEQ ID NO:49, amino acid residues 35-40 of SEQ ID NO:49, and amino acid residues 33-38 of SEQ ID NO:49. For example, in mouse IL-31, hydrophilic regions include amino acid residues 34-39 of SEQ ID NO:20 amino acid residues 46-51 of SEQ ID NO:20 amino acid residues 131-136 of SEQ ID NO:20 amino acid residues 158-163 of SEQ ID NO:20 and amino acid residues 157-162 of SEQ ID NO:20

Antigenic epitope-bearing peptides and polypeptides preferably contain at least four to ten amino acids, at least ten to fourteen amino acids, or about fourteen to about thirty amino acids of SEQ ID NO:2 or SEQ ID NO:5. Such epitope-bearing peptides and polypeptides can be produced by fragmenting a IL-31 polypeptide, or by chemical peptide synthesis, as described herein. Moreover, epitopes can be selected by phage display of random peptide libraries (see, for example, Lane and Stephen, Curr. Opin. Immunol. 5:268 (1993); and Cortese et al., Curr. Opin. Biotechnol. 7:616 (1996)). Standard methods for identifying epitopes and producing antibodies from small peptides that comprise an epitope are described, for example, by Mole, "Epitope Mapping," in Methods in Molecular Biology, Vol. 10, Manson (ed.), pages 105-116 (The Humana Press, Inc. 1992); Price, "Production and Characterization of Synthetic Peptide-Derived Antibodies," in Monoclonal Antibodies: Production, Engineering, and Clinical Application, Ritter and Ladyman (eds.), pages 60-84 (Cambridge University Press 1995), and Coligan et al. (eds.), Current Protocols in Immunology, pages 9.3.1 - 9.3.5 and pages 9.4.1 - 9.4.11 (John Wiley & Sons 1997).

In an experiment where the proteins of the present invention were screened as an antigen for efficacy in producing mouse anti-human IL-31 monoclonal antibodies, fusions derived from mice immunized with the BHK produced IL-31 had the better neutralizing capability that E, coli produced IL-31 with the amino acid sequence from SEQ ID NO: 23.

Regardless of the particular nucleotide sequence of a variant IL-31 and IL-31Cys mutants polynucleotide, the polynucleotide encodes a polypeptide that is characterized by its proliferative or differentiating activity, its ability to induce or inhibit specialized cell functions, or by the ability to bind specifically to an anti-IL-31 and IL-31Cys mutants antibody or zcytor17 receptor. More specifically, variant IL-31 and IL-31Cys mutants polynucleotides will encode polypeptides which exhibit at least 50% and preferably, at least 70%, at least 80%, at least 90%, at least 95%, at least 96%, at least 97%, at least 98%, at least 99%, or greater than 99%, of the activity of the polypeptide as shown in SEQ ID NO:2.

For any IL-31 and IL-31Cys mutants polypeptide, including variants and fusion proteins, one of ordinary skill in the art can readily generate a fully degenerate polynucleotide sequence encoding that variant using the information set forth in Tables 1 and 2 above.

The present specification describes a variety of other polypeptide fusions (and related multimeric proteins comprising one or more polypeptide fusions). For example, a IL-31 and IL-31Cys mutant polypeptide can be prepared as a fusion to a dimerizing protein as disclosed in U.S. Patents Nos. 5,155,027 and 5,567,584. Preferred dimerizing proteins in this regard include immunoglobulin constant region domains. Immunoglobulin- IL-31 and IL-31Cys mutants polypeptide fusions can be expressed in genetically engineered cells (to produce a variety of multimeric IL-31 and IL-31Cys mutants analogs). Auxiliary domains can be fused to IL-31 and IL-31Cys mutants polypeptides to target them to specific cells, tissues, or macromolecules. For example, a IL-31 and IL-31Cys mutants polypeptide or protein could be targeted to a predetermined cell type by fusing a IL-31 and IL-31Cys mutants polypeptide to a ligand that specifically binds to a receptor on the surface of that target cell. In this way, polypeptides and proteins can be targeted for therapeutic or diagnostic purposes. A IL-31 and IL-31Cys mutants polypeptide can be fused to two or more moieties, such as an affinity tag for purification and a targeting domain. Polypeptide fusions can also comprise one or more cleavage sites, particularly between domains. See, Tuan et al., Connective Tissue Research 34:1-9, 1996.

The IL-31 and IL-31Cys mutant polypeptides described herein, including full-length polypeptides, functional fragments, and fusion polypeptides, can be produced in genetically engineered host cells according to conventional techniques. Suitable host cells are those cell types that can be transformed or transfected with exogenous DNA and grown in culture, and include bacteria, fungal cells, and cultured higher eukaryotic cells. Eukaryotic cells, particularly cultured cells of multicellular organisms, are preferred. Techniques for manipulating cloned DNA molecules and introducing exogenous DNA into a variety of host cells are disclosed by Sambrook et al., Molecular Cloning: A Laboratory Manual, 2nd ed., Cold Spring Harbor Laboratory Press, Cold Spring Harbor, NY, 1989, and Ausubel et al., eds., Current Protocols in Molecular Biology, John Wiley and Sons, Inc., NY, 1987.

To direct a IL-31 and IL-31Cys mutants polypeptide into the secretory pathway of a host cell, a secretory signal sequence (also known as a leader sequence, prepro sequence or pre sequence) is provided in the expression vector. The secretory signal sequence may be that of IL-31 and IL-31Cys mutants, or may be derived from another secreted protein (e.g., t-PA) or synthesized de novo. The secretory signal sequence is operably linked to the IL-31 and IL-31Cys mutants DNA sequence, i.e., the two sequences are joined in the correct reading frame and positioned to direct the newly synthesized polypeptide into the secretory pathway of the host cell. Secretory signal sequences are commonly positioned 5' to the DNA sequence encoding the polypeptide of interest, although certain secretory signal sequences may be positioned elsewhere in the DNA sequence of interest (see, e.g., Welch et al., U.S. Patent No. 5,037,743; Holland et al., U.S. Patent No. 5,143,830).

Alternatively, the secretory signal sequence contained in the polypeptides of the present invention is used to direct other polypeptides into the secretory pathway. The present invention provides for such fusion polypeptides. A signal fusion polypeptide can be made wherein a secretory signal sequence derived from amino acid residue 1-23 of SEQ ID NO:2 or residues 1-23 SEQ ID NO:5 is be operably linked to a DNA sequence encoding another polypeptide using methods known in the art and disclosed herein. The secretory signal sequence contained in the fusion polypeptides of the present invention is preferably fused amino-terminally to an additional peptide to direct the additional peptide into the secretory pathway. Such constructs have numerous applications known in the art. For example, these novel secretory signal sequence fusion constructs can direct the secretion of an active component of a normally non-secreted protein. Such fusions may be used in vivo or in vitro to direct peptides through the secretory pathway.

Prokaryotic host cells, including strains of the bacteria Escherichia coli, Bacillus and other genera are also useful host cells within the present invention. Techniques for transforming these hosts and expressing foreign DNA sequences cloned therein are well known in the art (see, e.g., Sambrook et al., ibid.). When expressing a IL-31 and IL-31Cys mutants polypeptide in bacteria such as E. coli, the polypeptide may be retained in the cytoplasm, typically as insoluble granules, or may be directed to the periplasmic space by a bacterial secretion sequence. In the former case, the cells are lysed, and the granules are recovered and denatured using, for example, guanidine isothiocyanate or urea. The denatured polypeptide can then be refolded and dimerized by diluting the denaturant, such as by dialysis against a solution of urea and a combination of reduced and oxidized glutathione, followed by dialysis against a buffered saline solution. In the latter case, the polypeptide can be recovered from the periplasmic space in a soluble and functional form by disrupting the cells (by, for example, sonication or osmotic shock) to release the contents of the periplasmic space and recovering the protein, thereby obviating the need for denaturation and refolding.

Transformed or transfected host cells are cultured according to conventional procedures in a culture medium containing nutrients and other components required for the growth of the chosen host cells. A variety of suitable media, including defined media and complex media, are known in the art and generally include a carbon source, a nitrogen source, essential amino acids, vitamins and minerals. Media may also contain such components as growth factors or serum, as required. The growth medium will generally select for cells containing the exogenously added DNA by, for example, drug selection or deficiency in an essential nutrient which is complemented by the selectable marker carried on the expression vector or co-transfected into the host cell. P. methanolica cells are cultured in a medium comprising adequate sources of carbon, nitrogen and trace nutrients at a temperature of about 25°C to 35°C. Liquid cultures are provided with sufficient aeration by conventional means, such as shaking of small flasks or sparging of fermentors. A preferred culture medium for P. methanolica is YEPD (2% D-glucose, 2% Bacto^{™} Peptone (Difco Laboratories, Detroit, MI), 1% Bacto^{™} yeast extract (Difco Laboratories), 0.004% adenine and 0.006% L-leucine).

It is preferred to purify the polypeptides of the present invention to ≥80% purity, more preferably to ≥90% purity, even more preferably ≥95% purity, and particularly preferred is a pharmaceutically pure state, that is greater than 99.9% pure with respect to contaminating macromolecules, particularly other proteins and nucleic acids, and free of infectious and pyrogenic agents. Preferably, a purified polypeptide is substantially free of other polypeptides, particularly other polypeptides of animal origin.

Expressed recombinant IL-31 and IL-31Cys mutants polypeptides (or chimeric IL-31 polypeptides) can be purified using fractionation and/or conventional purification methods and media. Ammonium sulfate precipitation and acid or chaotrope extraction may be used for fractionation of samples. Exemplary purification steps may include hydroxyapatite, size exclusion, FPLC and reverse-phase high performance liquid chromatography. Suitable chromatographic media include derivatized dextrans, agarose, cellulose, polyacrylamide, specialty silicas, and the like. PEI, DEAE, QAE and Q derivatives are preferred. Exemplary chromatographic media include those media derivatized with phenyl, butyl, or octyl groups, such as Phenyl-Sepharose FF (Pharmacia), Toyopearl butyl 650 (Toso Haas, Montgomeryville, PA), Octyl-Sepharose (Pharmacia) and the like; or polyacrylic resins, such as Amberchrom CG 71 (Toso Haas) and the like. Suitable solid supports include glass beads, silica-based resins, cellulosic resins, agarose beads, cross-linked agarose beads, polystyrene beads, cross-linked polyacrylamide resins and the like that are insoluble under the conditions in which they are to be used. These supports may be modified with reactive groups that allow attachment of proteins by amino groups, carboxyl groups, sulfhydryl groups, hydroxyl groups and/or carbohydrate moieties. Examples of coupling chemistries include cyanogen bromide activation, N-hydroxysuccinimide activation, epoxide activation, sulfhydryl activation, hydrazide activation, and carboxyl and amino derivatives for carbodiimide coupling chemistries. These and other solid media are well known and widely used in the art, and are available from commercial suppliers. Methods for binding receptor polypeptides to support media are well known in the art. Selection of a particular method is a matter of routine design and is determined in part by the properties of the chosen support. See, for example, Affinity Chromatography: Principles & Methods, Pharmacia LKB Biotechnology, Uppsala, Sweden, 1988.

The polypeptides of the present invention can be isolated by exploitation of their physical or biochemical properties. For example, immobilized metal ion adsorption (IMAC) chromatography can be used to purify histidine-rich proteins, including those comprising polyhistidine tags. Briefly, a gel is first charged with divalent metal ions to form a chelate (Sulkowski, Trends in Biochem. 3:1-7, 1985). Histidine-rich proteins will be adsorbed to this matrix with differing affinities, depending upon the metal ion used, and will be eluted by competitive elution, lowering the pH, or use of strong chelating agents. Other methods of purification include purification of glycosylated proteins by lectin affinity chromatography and ion exchange chromatography (Methods in Enzymol., Vol. 182, "Guide to Protein Purification", M. Deutscher, (ed.), Acad. Press, San Diego, 1990, pp.529-39) and use of the soluble zcytor17 receptor. Within additional embodiments of the invention, a fusion of the polypeptide of interest and an affinity tag (e.g., maltose-binding protein, an immunoglobulin domain) may be constructed to facilitate purification.

Moreover, using methods described in the art, polypeptide fusions, or hybrid IL-31 and IL-31Cys mutants proteins, are constructed using regions or domains of the inventive IL-31 and IL-31Cys mutants in combination with those of other human cytokine family proteins (e.g. interleukins or GM-CSF), or heterologous proteins (Sambrook et al., ibid., Altschul et al., ibid., Picard, Cur. Opin. Biology, 5:511-5, 1994, and references therein). These methods allow the determination of the biological importance of larger domains or regions in a polypeptide of interest. Such hybrids may alter reaction kinetics, binding, constrict or expand the substrate specificity, or alter tissue and cellular localization of a polypeptide, and can be applied to polypeptides of unknown structure.

Fusion proteins can be prepared by methods known to those skilled in the art by preparing each component of the fusion protein and chemically conjugating them. Alternatively, a polynucleotide encoding both components of the fusion protein in the proper reading frame can be generated using known techniques and expressed by the methods described herein. For example, part or all of a helix conferring a biological function may be swapped between IL-31 and IL-31Cys mutants of the present invention with the functionally equivalent helices from another family member, such as IL-15, IL-2, IL-4 or GM-CSF. Such components include, but are not limited to, the secretory signal sequence; helices A, B, C, D; loops A/B, B/C, C/D; of four-helical-bundle cytokines. Such fusion proteins would be expected to have a biological functional profile that is the same or similar to polypeptides of the present invention or other known four-helical-bundle cytokine family proteins, depending on the fusion constructed. Moreover, such fusion proteins may exhibit other properties as disclosed herein.

Standard molecular biological and cloning techniques can be used to swap the equivalent domains between the IL-31 and IL-31Cys mutants polypeptide and those polypeptides to which they are fused. Generally, a DNA segment that encodes a domain of interest, e.g., IL-31 and IL-31Cys mutants helices A through D, or other domain described herein, is operably linked in frame to at least one other DNA segment encoding an additional polypeptide (for instance a domain or region from another cytokine, such as the IL-2, or the like), and inserted into an appropriate expression vector, as described herein. Generally DNA constructs are made such that the several DNA segments that encode the corresponding regions of a polypeptide are operably linked in frame to make a single construct that encodes the entire fusion protein, or a functional portion thereof. For example, a DNA construct would encode from N-terminus to C-terminus a fusion protein comprising a signal polypeptide followed by a mature four helical bundle cytokine fusion protein containing helix A, followed by helix B, followed by helix C, followed by helix D. Such fusion proteins can be expressed, isolated, and assayed for activity as described herein.

IL-31 and IL-31Cys mutants polypeptides or fragments thereof may also be prepared through chemical synthesis. IL-31 and IL-31Cys mutants polypeptides may be monomers or multimers; glycosylated or non-glycosylated; pegylated or non-pegylated; and may or may not include an initial methionine amino acid residue. For example, the polypeptides can be prepared by solid phase peptide synthesis, for example as described by Merrifield, J. Am. Chem. Soc. 85:2149, 1963.

The activity of molecules of the present invention can be measured using a variety of assays that measure proliferation of and/or binding to cells expressing the zcytor17 receptor. Of particular interest are changes in IL-31-dependent cells. Suitable cell lines to be engineered to be IL-31-dependent include the IL-3-dependent BaF3 cell line (Palacios and Steinmetz, Cell 41: 727-734, 1985; Mathey-Prevot et al., Mol. Cell. Biol. 6: 4133-4135, 1986), FDC-P1 (Hapel et al., Blood 64: 786-790, 1984), and MO7e (Kiss et al., Leukemia 7: 235-240, 1993). Growth factor-dependent cell lines can be established according to published methods (e.g. Greenberger et al., Leukemia Res. 8: 363-375, 1984; Dexter et al., in Baum et al. Eds., Experimental Hematology Today, 8th Ann. Mtg. Int. Soc. Exp. Hematol. 1979, 145-156, 1980).

As a ligand, the activity of IL-31 and IL-31Cys mutants polypeptide can be measured by a silicon-based biosensor microphysiometer which measures the extracellular acidification rate or proton excretion associated with receptor binding and subsequent physiologic cellular responses. An exemplary device is the Cytosensor^{™} Microphysiometer manufactured by Molecular Devices, Sunnyvale, CA. A variety of cellular responses, such as cell proliferation, ion transport, energy production, inflammatory response, regulatory and receptor activation, and the like, can be measured by this method. See, for example, McConnell, H.M. et al., Science 257:1906-1912, 1992; Pitchford, S. et al., Meth. Enzymol. 228:84-108, 1997; Arimilli, S. et al., J. Immunol. Meth. 212:49-59, 1998; Van Liefde, I. et al., Eur. J. Pharmacol. 346:87-95, 1998.

Moreover, IL-31 and IL-31Cys mutants can be used to identify cells, tissues, or cell lines which respond to a IL-31 and IL-31Cys mutants-stimulated pathway. The microphysiometer, described above, can be used to rapidly identify ligand-responsive cells, such as cells responsive to IL-31 and IL-31Cys mutants of the present invention. Cells can be cultured in the presence or absence of IL-31 and IL-31Cys mutants polypeptide. Those cells which elicit a measurable change in extracellular acidification in the presence of IL-31 and IL-31Cys mutants are responsive to IL-31 and IL-31Cys mutants. Such cells or cell lines, can be used to identify antagonists and agonists of IL-31 and IL-31Cys mutants polypeptide as described above.

IL-31 and IL-31Cys mutants can also be used to identify inhibitors (antagonists) of its activity. Test compounds are added to the assays disclosed herein to identify compounds that inhibit the activity of IL-31 and IL-31Cys mutants. In addition to those assays disclosed herein, samples can be tested for inhibition of IL-31 and IL-31Cys mutants activity within a variety of assays designed to measure receptor binding, the stimulation/inhibition of IL-31 and IL-31Cys mutants-dependent cellular responses or proliferation of zcytor17 receptor-expressing cells.

A IL-31 and IL-31Cys mutants polypeptide can be expressed as a fusion with an immunoglobulin heavy chain constant region, typically an Fc fragment, which contains two constant region domains and lacks the variable region. Methods for preparing such fusions are disclosed in U.S. Patents Nos. 5,155,027 and 5,567,584. Such fusions are typically secreted as multimeric molecules wherein the Fc portions are disulfide bonded to each other and two non-Ig polypeptides are arrayed in closed proximity to each other. Fusions of this type can be used for example, for dimerization, increasing stability and in vivo half-life, to affinity purify ligand, as in vitro assay tool or antagonist. For use in assays, the chimeras are bound to a support via the Fc region and used in an ELISA format.

An assay system that uses a ligand-binding receptor (or an antibody, one member of a complement/anti-complement pair) or a binding fragment thereof, and a commercially available biosensor instrument (BIAcore, Pharmacia Biosensor, Piscataway, NJ) may be advantageously employed. Such receptor, antibody, member of a complement/anti-complement pair or fragment is immobilized onto the surface of a receptor chip. Use of this instrument is disclosed by Karlsson, J. Immunol. Methods 145:229-40, 1991 and Cunningham and Wells, J. Mol. Biol. 234:554-63, 1993. A receptor, antibody, member or fragment is covalently attached, using amine or sulfhydryl chemistry, to dextran fibers that are attached to gold film within the flow cell. A test sample is passed through the cell. If a ligand, epitope, or opposite member of the complement/anti-complement pair is present in the sample, it will bind to the immobilized receptor, antibody or member, respectively, causing a change in the refractive index of the medium, which is detected as a change in surface plasmon resonance of the gold film. This system allows the determination of on- and off-rates, from which binding affinity can be calculated, and assessment of stoichiometry of binding. Alternatively, ligand/receptor binding can be analyzed using SELDI(TM) technology (Ciphergen, Inc., Palo Alto, CA).

Ligand-binding receptor polypeptides can also be used within other assay systems known in the art. Such systems include Scatchard analysis for determination of binding affinity (see Scatchard, Ann. NY Acad. Sci. 51: 660-72, 1949) and calorimetric assays (Cunningham et al., Science 253:545-48, 1991; Cunningham et al., Science 245:821-25,1991).

IL-31 and IL-31Cys mutants polypeptides can also be used to prepare antibodies that bind to IL-31 epitopes, peptides or polypeptides. The IL-31 and IL-31Cys mutants polypeptide or a fragment thereof serves as an antigen (immunogen) to inoculate an animal and elicit an immune response. Such antibodies can be used to block the biological action of pro-inflammatory IL-31 and IL-31Cys mutants and are useful as anti-inflammatory therapeutics in a variety of diseases as described herein. One of skill in the art would recognize that antigenic, epitope-bearing polypeptides contain a sequence of at least 6, preferably at least 9, and more preferably at least 15 to about 30 contiguous amino acid residues of a IL-31 and IL-31Cys mutants polypeptide (e.g., SEQ ID NO:2). Polypeptides comprising a larger portion of a IL-31 and IL-31Cys mutants polypeptide, i.e., from 30 to 100 residues up to the entire length of the amino acid sequence are included. Antigens or immunogenic epitopes can also include attached tags, adjuvants, vehicles and carriers, as described herein. Suitable antigens include the IL-31 polypeptide encoded by SEQ ID NO:2 from amino acid number 24 to amino acid number 164, or a contiguous 9 to 141 amino acid fragment thereof. Other suitable antigens include, the full length and the mature IL-31, helices A-D, and individual or multiple helices A, B, C, and D, of the IL-31 four-helical-bundle structure, as described herein. Preferred peptides to use as antigens are hydrophilic peptides such as those predicted by one of skill in the art from a hydrophobicity plot, as described herein, for example, amino acid residues 114-119, 101-105, 126-131, 113-118, and 158-162 of SEQ ID NO:2; and amino acid residues 34-39, 46-51, 131-136, 158-163 and 157-162 of SEQ ID NO:11. Moreover, IL-31 and IL-31Cys mutants antigenic epitopes as predicted by a Jameson-Wolf plot, e.g., using DNASTAR Protean program (DNASTAR, Inc., Madison, WI) serve as preferred antigens, and are readily determined by one of skill in the art.

Antibodies from an immune response generated by inoculation of an animal with these antigens can be isolated and purified as described herein. Methods for preparing and isolating polyclonal and monoclonal antibodies are well known in the art. See, for example, Current Protocols in Immunology, Cooligan, et al. (eds.), National Institutes of Health, John Wiley and Sons, Inc., 1995; Sambrook et al., Molecular Cloning: A Laboratory Manual, Second Edition, Cold Spring Harbor, NY, 1989; and Hurrell, J. G. R., Ed., Monoclonal Hybridoma Antibodies: Techniques and Applications, CRC Press, Inc., Boca Raton, FL, 1982.

As would be evident to one of ordinary skill in the art, polyclonal antibodies can be generated from inoculating a variety of warm-blooded animals such as horses, cows, goats, sheep, dogs, chickens, rabbits, mice, and rats with a IL-31 polypeptide or a fragment thereof. The immunogenicity of a IL-31 and IL-31Cys mutants polypeptide may be increased through the use of an adjuvant, such as alum (aluminum hydroxide) or Freund's complete or incomplete adjuvant. Polypeptides useful for immunization also include fusion polypeptides, such as fusions of IL-31 and IL-31Cys mutants or a portion thereof with an immunoglobulin polypeptide or with maltose binding protein. The polypeptide immunogen may be a full-length molecule or a portion thereof. If the polypeptide portion is "hapten-like", such portion, may be advantageously joined or linked to a macromolecular carrier (such as keyhole limpet hemocyanin (KLH), bovine serum albumin (BSA) or tetanus toxoid) for immunization.

As used herein, the term "antibodies" includes polyclonal antibodies, affinity-purified polyclonal antibodies, monoclonal antibodies, and antigen-binding fragments, such as F(ab')2 and Fab proteolytic fragments. Genetically engineered intact antibodies or fragments, such as chimeric antibodies, Fv fragments, single chain antibodies and the like, as well as synthetic antigen-binding peptides and polypeptides, are also included. Non-human antibodies may be humanized by grafting non-human CDRs onto human framework and constant regions, or by incorporating the entire non-human variable domains (optionally "cloaking" them with a human-like surface by replacement of exposed residues, wherein the result is a "veneered" antibody). In some instances, humanized antibodies may retain non-human residues within the human variable region framework domains to enhance proper binding characteristics. Through humanizing antibodies, biological half-life may be increased, and the potential for adverse immune reactions upon administration to humans is reduced. Moreover, human antibodies can be produced in transgenic, non-human animals that have been engineered to contain human immunoglobulin genes as disclosed in WIPO Publication No. WO 98/24893. It is preferred that the endogenous immunoglobulin genes in these animals be inactivated or eliminated, such as by homologous recombination.

Antibodies are considered to be specifically binding if: 1) they exhibit a threshold level of binding activity, and 2) they do not significantly cross-react with related polypeptide molecules. A threshold level of binding is determined if anti-IL-31 and IL-31Cys mutants antibodies herein bind to a IL-31 and IL-31Cys mutants polypeptide, peptide or epitope with an affinity at least 10-fold greater than the binding affinity to control (non-IL-31) polypeptide. It is preferred that the antibodies exhibit a binding affinity (Ka) of 106 M-1 or greater, preferably 107 M-1 or greater, more preferably 108 M-1 or greater, and most preferably 109 M-1 or greater. The binding affinity of an antibody can be readily determined by one of ordinary skill in the art, for example, by Scatchard analysis (Scatchard, G., Ann. NY Acad. Sci. 51: 660-672,1949).

Whether anti-IL-31 and IL-31Cys mutants antibodies do not significantly cross-react with related polypeptide molecules is shown, for example, by the antibody detecting IL-31 and IL-31Cys mutants polypeptide but not known related polypeptides using a standard Western blot analysis (Ausubel et al., ibid.). Examples of known related polypeptides are those disclosed in the prior art, such as known orthologs, and paralogs, and similar known members of a protein family. Screening can also be done using non-human IL-31, and IL-31 mutant polypeptides. Moreover, antibodies can be "screened against" known related polypeptides, to isolate a population that specifically binds to the IL-31 and IL-31Cys mutants polypeptides. For example, antibodies raised to IL-31 and IL-31Cys mutants are adsorbed to related polypeptides adhered to insoluble matrix; antibodies specific to IL-31 and IL-31Cys mutants will flow through the matrix under the proper buffer conditions. Screening allows isolation of polyclonal and monoclonal antibodies non-crossreactive to known closely related polypeptides (Antibodies: A Laboratory Manual, Harlow and Lane (eds.), Cold Spring Harbor Laboratory Press, 1988; Current Protocols in Immunology, Cooligan, et al. (eds.), National Institutes of Health, John Wiley and Sons, Inc., 1995). Screening and isolation of specific antibodies is well known in the art. See, Fundamental Immunology, Paul (eds.), Raven Press, 1993; Getzoff et al., Adv. in Immunol. 43: 1-98, 1988; Monoclonal Antibodies: Principles and Practice, Goding, J.W. (eds.), Academic Press Ltd., 1996; Benjamin et al., Ann. Rev. Immunol. 2: 67-101, 1984. Specifically binding anti-IL-31 and IL-31Cys mutants antibodies can be detected by a number of methods in the art, and disclosed below.

A variety of assays known to those skilled in the art can be utilized to detect antibodies which bind to IL-31 and IL-31Cys mutants proteins or polypeptides. Exemplary assays are described in detail in Antibodies: A Laboratory Manual, Harlow and Lane (Eds.), Cold Spring Harbor Laboratory Press, 1988. Representative examples of such assays include: concurrent immunoelectrophoresis, radioimmunoassay, radioimmuno-precipitation, enzyme-linked immunosorbent assay (ELISA), dot blot or Western blot assay, inhibition or competition assay, and sandwich assay. In addition, antibodies can be screened for binding to wild-type versus mutant IL-31 protein or polypeptide.

Antibodies to IL-31 and IL-31Cys mutants may be used for tagging cells that express IL-31; for isolating IL-31 and IL-31Cys mutants by affinity purification; for diagnostic assays for determining circulating levels of IL-31 polypeptides; for detecting or quantitating soluble IL-31 as a marker of underlying pathology or disease; in analytical methods employing FACS; for screening expression libraries; for generating anti-idiotypic antibodies; and as neutralizing antibodies or as antagonists to block IL-31 activity in vitro and in vivo. Suitable direct tags or labels include radionuclides, enzymes, substrates, cofactors, inhibitors, fluorescent markers, chemiluminescent markers, magnetic particles and the like; indirect tags or labels may feature use of biotin-avidin or other complement/anti-complement pairs as intermediates. Antibodies herein may also be directly or indirectly conjugated to drugs, toxins, radionuclides and the like, and these conjugates used for in vivo diagnostic or therapeutic applications. Moreover, antibodies to IL-31 and IL-31Cys mutants or fragments thereof may be used in vitro to detect denatured IL-31 and IL-31Cys mutants or fragments thereof in assays, for example, Western Blots or other assays known in the art.

Suitable detectable molecules may be directly or indirectly attached to the polypeptide or antibody, and include radionuclides, enzymes, substrates, cofactors, inhibitors, fluorescent markers, chemiluminescent markers, magnetic particles and the like. Suitable cytotoxic molecules may be directly or indirectly attached to the polypeptide or antibody, and include bacterial or plant toxins (for instance, diphtheria, toxin, saporin, Pseudomonas exotoxin, ricin, abrin and the like), as well as therapeutic radionuclides, such as iodine-131, rhenium-188 or yttrium-90 (either directly attached to the polypeptide or antibody, or indirectly attached through means of a chelating moiety, for instance). Polypeptides or antibodies may also be conjugated to cytotoxic drugs, such as adriamycin. For indirect attachment of a detectable or cytotoxic molecule, the detectable or cytotoxic molecule can be conjugated with a member of a complementary/anticomplementary pair, where the other member is bound to the polypeptide or antibody portion. For these purposes, biotin/streptavidin is an exemplary complementary/ anticomplementary pair.

Binding polypeptides can also act as IL-31 and IL-31Cys mutants "antagonists" to block IL-31 and IL-31Cys mutants binding and signal transduction in vitro and in vivo. These anti-IL-31 and IL-31Cys mutants binding polypeptides would be useful for inhibiting IL-31 activity or protein-binding.

Polypeptide-toxin fusion proteins or antibody-toxin fusion proteins can be used for targeted cell or tissue inhibition or ablation (for instance, to treat cancer cells or tissues). Alternatively, if the polypeptide has multiple functional domains (i.e., an activation domain or a receptor binding domain, plus a targeting domain), a fusion protein including only the targeting domain may be suitable for directing a detectable molecule, a cytotoxic molecule or a complementary molecule to a cell or tissue type of interest. In instances where the domain only fusion protein includes a complementary molecule, the anti-complementary molecule can be conjugated to a detectable or cytotoxic molecule. Such domain-complementary molecule fusion proteins thus represent a generic targeting carrier or vehicle for cell/tissue-specific delivery of generic anti-complementary-detectable/ cytotoxic molecule conjugates.

In another embodiment, IL-31 and IL-31Cys mutants cytokine fusion proteins or antibody-cytokine fusion proteins can be used for in vivo killing of target tissues (for example, leukemia, lymphoma, lung cancer, colon cancer, melanoma, pancreatic cancer, ovanian cancer, skin, blood and bone marrow cancers, or other cancers wherein IL-31 receptors ar expressed) (See, generally, Hornick et al., Blood 89:4437-47, 1997). The described fusion proteins enable targeting of a cytokine to a desired site of action, thereby providing an elevated local concentration of cytokine. Suitable IL-31 and IL-31Cys mutants polypeptides or anti-IL-31 antibodies target an undesirable cell or tissue (i.e., a tumor or a leukemia), and the fused cytokine mediated improved target cell lysis by effector cells. Suitable cytokines for this purpose include interleukin 2 and granulocyte-macrophage colony-stimulating factor (GM-CSF), for instance.

The bioactive polypeptide or antibody conjugates described herein can be delivered intravenously, intraarterially or intraductally, or may be introduced locally at the intended site of action.

Inflammation is a protective response by an organism to fend off an invading agent. Inflammation is a cascading event that involves many cellular and humoral mediators. On one hand, suppression of inflammatory responses can leave a host immunocompromised; however, if left unchecked, inflammation can lead to serious complications including chronic inflammatory diseases (e.g., rheumatoid arthritis, multiple sclerosis, inflammatory bowel disease and the like), septic shock and multiple organ failure. Importantly, these diverse disease states share common inflammatory mediators. The collective diseases that are characterized by inflammation have a large impact on human morbidity and mortality. Therefore it is clear that anti-inflammatory antibodies and binding polypeptides, such as anti-IL-31 and IL-31Cys mutants antibodies and binding polypeptides described herein, could have crucial therapeutic potential for a vast number of human and animal diseases, from asthma and allergy to autoimmunity and septic shock. As such, use of anti-inflammatory anti IL-31 and IL-31Cys mutants antibodies and binding polypeptides described herein can be used therapeutically as IL-31 antagonists described herein, particularly in diseases such as arthritis, endotoxemia, inflammatory bowel disease, psoriasis, related disease and the like.

1. Arthritis

Arthritis, including osteoarthritis, rheumatoid arthritis, arthritic joints as a result of injury, and the like, are common inflammatory conditions which would benefit from the therapeutic use of anti-inflammatory antibodies and binding polypeptides, such as anti-IL-31 and IL-31Cys mutants antibodies and binding polypeptides of the present invention. For Example, rheumatoid arthritis (RA) is a systemic disease that affects the entire body and is one of the most common forms of arthritis. It is characterized by the inflammation of the membrane lining the joint, which causes pain, stiffness, warmth, redness and swelling. Inflammatory cells release enzymes that may digest bone and cartilage. As a result of rheumatoid arthritis, the inflamed joint lining, the synovium, can invade and damage bone and cartilage leading to joint deterioration and severe pain amongst other physiologic effects. The involved joint can lose its shape and alignment, resulting in pain and loss of movement.

Rheumatoid arthritis (RA) is an immune-mediated disease particularly characterized by inflammation and subsequent tissue damage leading to severe disability and increased mortality. A variety of cytokines are produced locally in the rheumatoid joints. Numerous studies have demonstrated that IL-1 and TNF-alpha, two prototypic pro-inflammatory cytokines, play an important role in the mechanisms involved in synovial inflammation and in progressive joint destruction. Indeed, the administration of TNF-alpha and IL-1 inhibitors in patients with RA has led to a dramatic improvement of clinical and biological signs of inflammation and a reduction of radiological signs of bone erosion and cartilage destruction. However, despite these encouraging results, a significant percentage of patients do not respond to these agents, suggesting that other mediators are also involved in the pathophysiology of arthritis (Gabay, Expert. Opin. Biol. Ther. 2(2):135-149, 2002). One of those mediators could be IL-31 and IL-31Cys mutants, and as such a molecule that binds or inhibits IL-31 and IL-31Cys mutants, such as anti IL-31 and IL-31Cys mutants antibodies or binding partners, could serve as a valuable therapeutic to reduce inflammation in rheumatoid arthritis, and other arthritic diseases.

There are several animal models for rheumatoid arthritis known in the art. For example, in the collagen-induced arthritis (CIA) model, mice develop chronic inflammatory arthritis that closely resembles human rheumatoid arthritis. Since CIA shares similar immunological and pathological features with RA, this makes it an ideal model for screening potential human anti-inflammatory compounds. The CIA model is a well-known model in mice that depends on both an immune response, and an inflammatory response, in order to occur. The immune response comprises the interaction of B-cells and CD4+ T-cells in response to collagen, which is given as antigen, and leads to the production of anti-collagen antibodies. The inflammatory phase is the result of tissue responses from mediators of inflammation, as a consequence of some of these antibodies cross-reacting to the mouse's native collagen and activating the complement cascade. An advantage in using the CIA model is that the basic mechanisms of pathogenesis are known. The relevant T-cell and B-cell epitopes on type II collagen have been identified, and various immunological (e.g., delayed-type hypersensitivity and anti-collagen antibody) and inflammatory (e.g., cytokines, chemokines, and matrix-degrading enzymes) parameters relating to immune-mediated arthritis have been determined, and can thus be used to assess test compound efficacy in the CIA model (Wooley, Curr. Opin. Rheum. 3:407-20, 1999; Williams et al., Immunol. 89:9784-788, 1992; Myers et al., Life Sci. 61:1861-78, 1997; and Wang et al., Immunol. 92:8955-959, 1995).

The administration of soluble zcytor17 comprising polypeptides (including heterodimeric and multimeric receptors described herein), such as zcytor17-Fc4 or other zcytor17 soluble and fusion proteins to these CIA model mice was used to evaluate the use of zcytor17to ameliorate symptoms and alter the course of disease. As a molecule that modulates immune and inflammatory response, IL-31 and IL-31Cys mutants, may induce production of SAA, which is implicated in the pathogenesis of rheumatoid arthritis, IL-31 and IL-31Cys mutants antagonists may reduce SAA activity in vitro and in vivo, the systemic or local administration of IL-31 and IL-31Cys mutants antagonists such as anti-IL-31 and IL-31Cys mutants antibodies or binding partners, zcytor17 comprising polypeptides (including heterodimeric and multimeric receptors described herein), such as zcytor17-Fc4 or other zcytor17 soluble and fusion proteins can potentially suppress the inflammatory response in RA. Other potential therapeutics include zcytor17 polypeptides, soluble heterodimeric and multimeric receptor polypeptides, or anti IL-31 and IL-31Cys mutants antibodies or binding partners of the present invention, and the like.

### 2. Endotoxemia

Endotoxemia is a severe condition commonly resulting from infectious agents such as bacteria and other infectious disease agents, sepsis, toxic shock syndrome, or in immunocompromised patients subjected to opportunistic infections, and the like. Therapeutically useful anti-inflammatory antibodies and binding polypeptides, such as anti-IL-31 and IL-31Cys mutants antibodies and binding polypeptides of the present invention, could aid in preventing and treating endotoxemia in humans and animals. Other potential therapeutics include zcytor17 polypeptides, soluble heterodimeric and multimeric receptor polypeptides, or anti IL-31 and IL-31Cys mutants antibodies or binding partners of the present invention, and the like, could serve as a valuable therapeutic to reduce inflammation and pathological effects in endotoxemia.

Lipopolysaccharide (LPS) induced endotoxemia engages many of the proinflammatory mediators that produce pathological effects in the infectious diseases and LPS induced endotoxemia in rodents is a widely used and acceptable model for studying the pharmacological effects of potential pro-inflammatory or immunomodulating agents. LPS, produced in gram-negative bacteria, is a major causative agent in the pathogenesis of septic shock (Glausner et al., Lancet 338:732, 1991). A shock-like state can indeed be induced experimentally by a single injection of LPS into animals. Molecules produced by cells responding to LPS can target pathogens directly or indirectly. Although these biological responses protect the host against invading pathogens, they may also cause harm. Thus, massive stimulation of innate immunity, occurring as a result of severe Gram-negative bacterial infection, leads to excess production of cytokines and other molecules, and the development of a fatal syndrome, septic shock syndrome, which is characterized by fever, hypotension, disseminated intravascular coagulation, and multiple organ failure (Dumitru et al. Cell 103:1071-1083, 2000).

These toxic effects of LPS are mostly related to macrophage activation leading to the release of multiple inflammatory mediators. Among these mediators, TNF appears to play a crucial role, as indicated by the prevention of LPS toxicity by the administration of neutralizing anti-TNF antibodies (Beutler et al., Science 229:869, 1985). It is well established that 1ug injection of E. coli LPS into a C57B1/6 mouse will result in significant increases in circulating IL-6, TNF-alpha, IL-1, and acute phase proteins (for example, SAA) approximately 2 hours post injection. The toxicity of LPS appears to be mediated by these cytokines as passive immunization against these mediators can result in decreased mortality (Beutler et al., Science 229:869, 1985). The potential immunointervention strategies for the prevention and/or treatment of septic shock include anti-TNF mAb, IL-1 receptor antagonist, LIF, IL-10, and G-CSF. Since LPS induces the production of pro-inflammatory factors possibly contributing to the pathology of endotoxemia, the neutralization of IL-31 and IL-31Cys mutants activity, SAA or other pro- inflammatory factors by antagonizing IL-31 and IL-31Cys mutants polypeptide can be used to reduce the symptoms of endotoxemia, such as seen in endotoxic shock. Other potential therapeutics include zcytor17polypeptides, soluble heterodimeric and multimeric receptor polypeptides, or anti-IL-31 and IL-31Cys mutants antibodies or binding partners of the present invention, and the like.

### 3 Inflammatory Bowel Disease. IBD

In the United States approximately 500,000 people suffer from Inflammatory Bowel Disease (IBD) which can affect either colon and rectum (Ulcerative colitis) or both, small and large intestine (Crohn's Disease). The pathogenesis of these diseases is unclear, but they involve chronic inflammation of the affected tissues. Potential therapeutics include zcytor17 polypeptides, soluble heterodimeric and multimeric receptor polypeptides, or anti-IL-31 and IL-31Cys mutants antibodies or binding partners of the present invention, and the like., could serve as a valuable therapeutic to reduce inflammation and pathological effects in IBD and related diseases.

Ulcerative colitis (UC) is an inflammatory disease of the large intestine, commonly called the colon, characterized by inflammation and ulceration of the mucosa or innermost lining of the colon. This inflammation causes the colon to empty frequently, resulting in diarrhea. Symptoms include loosening of the stool and associated abdominal cramping, fever and weight loss. Although the exact cause of UC is unknown, recent research suggests that the body's natural defenses are operating against proteins in the body which the body thinks are foreign (an "autoimmune reaction"). Perhaps because they resemble bacterial proteins in the gut, these proteins may either instigate or stimulate the inflammatory process that begins to destroy the lining of the colon. As the lining of the colon is destroyed, ulcers form releasing mucus, pus and blood. The disease usually begins in the rectal area and may eventually extend through the entire large bowel. Repeated episodes of inflammation lead to thickening of the wall of the intestine and rectum with scar tissue. Death of colon tissue or sepsis may occur with severe disease. The symptoms of ulcerative colitis vary in severity and their onset may be gradual or sudden. Attacks may be provoked by many factors, including respiratory infections or stress.

Although there is currently no cure for UC available, treatments are focused on suppressing the abnormal inflammatory process in the colon lining. Treatments including corticosteroids immunosuppressives (eg. azathioprine, mercaptopurine, and methotrexate) and aminosalicytates are available to treat the disease. However, the long-term use of immunosuppressives such as corticosteroids and azathioprine can result in serious side effects including thinning of bones, cataracts, infection, and liver and bone marrow effects. In the patients in whom current therapies are not successful, surgery is an option. The surgery involves the removal of the entire colon and the rectum.

There are several animal models that can partially mimic chronic ulcerative colitis. The most widely used model is the 2,4,6-trinitrobenesulfonic acid/ethanol (TNBS) induced colitis model, which induces chronic inflammation and ulceration in the colon. When TNBS is introduced into the colon of susceptible mice via intra-rectal instillation, it induces T-cell mediated immune response in the colonic mucosa, in this case leading to a massive mucosal inflammation characterized by the dense infiltration of T-cells and macrophages throughout the entire wall of the large bowel. Moreover, this histopathologic picture is accompanies by the clinical picture of progressive weight loss (wasting), bloody diarrhea, rectal prolapse, and large bowel wall thickening (Neurath et al. Intern. Rev. Immunol. 19:51-62, 2000).

Another colitis model uses dextran sulfate sodium (DSS), which induces an acute colitis manifested by bloody diarrhea, weight loss, shortening of the colon and mucosal ulceration with neutrophil infiltration. DSS-induced colitis is characterized histologically by infiltration of inflammatory cells into the lamina propria, with lymphoid hyperplasia, focal crypt damage, and epithelial ulceration. These changes are thought to develop due to a toxic effect of DSS on the epithelium and by phagocytosis of lamina propria cells and production of TNF-alpha and IFN-gamma. Despite its common use, several issues regarding the mechanisms of DSS about the relevance to the human disease remain unresolved. DSS is regarded as a T cell-independent model because it is observed in T cell-deficient animals such as SCID mice.

The administration of anti-IL-31 and IL-31Cys mutants antibodies or binding partners, soluble zcytor17 comprising polypeptides (including heterodimeric and multimeric receptors), such as zcytor17-Fc4 or other zcytor17 soluble and fusion proteins to these TNBS or DSS models can be used to evaluate the use of IL-31 and IL-31Cys mutants antagonists to ameliorate symptoms and alter the course of gastrointestinal disease. IL-31 and IL-31Cys mutants may play a role in the inflammatory response in colitis, and the neutralization of IL-31 and IL-31Cys mutants activity by administrating IL-31 and IL-31Cys mutants antagonists is a potential therapeutic approach for IBD. Other potential therapeutics include zcytor17 polypeptides, soluble heterodimeric and multimeric receptor polypeptides, or anti-IL-31 and IL-31Cys mutants antibodies or binding partners of the present invention, and the like.

### 4. Psoriasis

Psoriasis is a chronic skin condition that affects more than seven million Americans. Psoriasis occurs when new skin cells grow abnormally, resulting in inflamed, swollen, and scaly patches of skin where the old skin has not shed quickly enough. Plaque psoriasis, the most common form, is characterized by inflamed patches of skin ("lesions") topped with silvery white scales. Psoriasis may be limited to a few plaques or involve moderate to extensive areas of skin, appearing most commonly on the scalp, knees, elbows and trunk. Although it is highly visible, psoriasis is not a contagious disease. The pathogenesis of the diseases involves chronic inflammation of the affected tissues. Zcytor17 polypeptides, soluble heterodimeric and multimeric receptor polypeptides, or anti-IL-31 and IL-31Cys mutants antibodies or binding partners of the present invention, and the like, could serve as a valuable therapeutic to reduce inflammation and pathological effects in psoriasis, other inflammatory skin diseases, skin and mucosal allergies, and related diseases.

Psoriasis is a T-cell mediated inflammatory disorder of the skin that can cause considerable discomfort. It is a disease for which there is no cure and affects people of all ages. Psoriasis affects approximately two percent of the populations of European and North America. Although individuals with mild psoriasis can often control their disease with topical agents, more than one million patients worldwide require ultraviolet or systemic immunosuppressive therapy. Unfortunately, the inconvenience and risks of ultraviolet radiation and the toxicities of many therapies limit their long-term use. Moreover, patients usually have recurrence of psoriasis, and in some cases rebound, shortly after stopping immunosuppressive therapy.

IL-31 was isolated from tissue known to have important immunological function and which contain cells that play a role in the immune system. IL-31 is expressed in CD3+ selected, activated peripheral blood cells, and it has been shown that IL-31 expression increases after T cell activation. Moreover, polypeptides of the present invention can have an effect on the growth/expansion of monocytes/macrophages, T-cells, B-cells, NK cells and/or differentiated state of monocytes/macrophages, T-cells, B-cells, NK cells or these cells' progenitors. Factors that both stimulate proliferation of hematopoietic progenitors and activate mature cells are generally known, however, proliferation and activation can also require additional growth factors. For example, it has been shown that IL-7 and Steel Factor (c-kit ligand) were required for colony formation of NK progenitors. IL-15 + IL-2 in combination with IL-7 and Steel Factor was more effective (Mrózek et al., Blood 87:2632-2640, 1996). However, unidentified cytokines may be necessary for proliferation of specific subsets of NK cells and/or NK progenitors (Robertson et. al., Blood 76:2451-2438,1990). Similarly, IL-31 and IL-31Cys mutants may act alone or in concert or synergy with other cytokines to enhance growth, proliferation expansion and modification of differentiation of monocytes/macrophages, T-cells, B-cells or NK cells.

Assays measuring differentiation include, for example, measuring cell markers associated with stage-specific expression of a tissue, enzymatic activity, functional activity or morphological changes (Watt, FASEB, 5:281-284, 1991; Francis, Differentiation 57:63-75, 1994; Raes, Adv. Anim. Cell Biol. Technol. Bioprocesses, 161-171, 1989; all incorporated herein by reference). Alternatively, IL-31 polypeptide itself can serve as an additional cell-surface or secreted marker associated with stage-specific expression of a tissue. As such, direct measurement of IL-31 polypeptide, or its loss of expression in a tissue as it differentiates, can serve as a marker for differentiation of tissues.

IL-31 and IL-31Cys mutants or antibodies thereto can be useful in treating tumorgenesis, and therefore would be useful in the treatment of cancer. IL-31 and IL- is expressed in activated T-cells, monocytes and macrophages, and is linked to a region of the human chromosome wherein translocations are common in leukemias. Moreover, the IL-31 is shown to act through a cytokine receptor, zcytor17, which is also expressed in activated T-cells, monocytes and macrophages. Over stimulation of activated T-cells, monocytes and macrophages by IL-31 and IL-31Cys mutants could result in a human disease state such as, for instance, an immune cell cancer or other cancers. As such, identifying IL-31 expression, polypeptides (e.g., by anti-IL-31 antibodies, zcytor17 soluble receptors (e.g., zcytor17 receptor, heterodimers, multimers, or other IL-31 binding partners) can serve as a diagnostic, and can serve as antagonists of IL-31 and IL-31Cys mutants proliferative activity. The ligand could be administered in combination with other agents already in use including both conventional chemotherapeutic agents as well as immune modulators such as interferon alpha. Alpha/beta interferons have been shown to be effective in treating some leukemias and animal disease models, and the growth inhibitory effects of interferon-alpha and IL-31 and IL-31Cys mutants may be additive.

NK cells are thought to play a major role in elimination of metastatic tumor cells and patients with both metastases and solid tumors have decreased levels of NK cell activity (Whiteside et. al., Curr. Top. Microbiol. Immunol. 230:221-244, 1998). An agent that stimulates NK cells would be useful in the elimination of tumors.

The present specification describes reducing proliferation of a neoplastic monocytes/macrophages comprising administering to a mammal with a monocyte/macrophage neoplasm an amount of a composition of IL-31 and IL-31Cys mutants or anti-IL-31 and IL-31Cys mutants sufficient to reduce proliferation of the neoplastic monocytes/macrophages. In other embodiments, the composition can comprise at least one other cytokine. A second cytokine may be selected from the group consisting of IL-2, IL-3, IL-12, IL-21, IL-22, IL-15, IL-4, GM-CSF, Flt3 ligand or stem cell factor.

The present specification describes inhibiting activation or differentiation of monocytes/macrophages. Monocytes are incompletely differentiated cells that migrate to various tissues where they mature and become macrophages. Macrophages play a central role in the immune response by presenting antigen to lymphocytes and play a supportive role as accessory cells to lymphocytes by secreting numerous cytokines. Macrophages can internalize extracellular molecules and upon activation have an increased ability to kill intracellular microorganisms and tumor cells. Activated macrophages are also involved in stimulating acute or local inflammation.

In another aspect, the present specification describes reducing proliferation of a neoplastic B or T-cells comprising administering to a mammal with a B or T cell neoplasm an amount of a composition of IL-31 and IL-31Cys mutants antagonist sufficient to reducing proliferation of the neoplastic monocytes/macrophages. In other embodiments, the composition can comprise at least one other cytokine, wherein the cytokine may be selected from the group consisting of IL-2, IL-3, IL-12, IL-21, IL-22, IL-15, IL-4, GM-CSF, Flt3 ligand or stem cell factor. Furthermore, the IL-31 and IL-31Cys mutants antagonist can be a ligand/toxin fusion protein.

A IL-31 and IL-31Cys mutants-saporin fusion toxin may be employed against a similar set of leukemias and lymphomas, extending the range of leukemias that can be treated with IL-31and IL-31Cys mutants. For example, such leukemias can be those that over-express zcytor17 receptors (e.g., zcytor17receptor, heterodimers (e.g., zcytor17/OSMRbeta,), multimers (e.g., zcytor17/OSMRbeta)). Fusion toxin mediated activation of the zcytor17 receptor, zcytor17receptor heterodimers or multimers (e.g., zcytor19/OSMRbeta) provides two independent means to inhibit the growth of the target cells, the first being identical to the effects seen by the ligand alone, and the second due to delivery of the toxin through receptor internalization. The lymphoid and monocyte restricted expression pattern of the zcytor17 receptor suggests that the ligand-saporin conjugate can be tolerated by patients.

When treatment for malignancies includes allogeneic bone marrow or stem cell transplantation, IL-31 and IL-31Cys mutants may be valuable in enhancement of the graft-vs-tumor effect. IL-31 and IL-31Cys mutants may stimulate the generation of lytic NK cells from marrow progenitors and can stimulate the proliferation of monocytes and macrophages following activation of the antigen receptors. Therefore, when patients receive allogeneic marrow transplants, IL-31 and IL-31Cys mutants will enhance the generation of anti-tumor responses, with or without the infusion of donor lymphocytes.

The tissue distribution of receptors for a given cytokine offers a strong indication of the potential sites of action of that cytokine. Expression of zcytor17 was seen in monocytes and B-cells, with a dramatic increase of expression upon activation for CD3+, CD4+, and CD8+ T-cells. In addition, two monocytic cell lines, THP-1 (Tsuchiya et al., Int. J. Cancer 26:171-176, 1980) and U937 (Sundstrom et al., Int. J. Cancer 17:565-577, 1976), were also positive for zcytor17expression.

Expression of OSMR is reported to be very broad (Mosley et al, JBC 271:32635-32643, 1996). This distribution of zcytor17and OSM receptors supports a role for IL-31 and IL-31Cys mutants in immune responses, especially expansion of T-cells upon activation or a role in the monocyte/macrophage arm of the immune system.

IL-31 and IL-31Cys mutants may find utility in the suppression of the immune system, such as in the treatment of autoimmune diseases, including rheumatoid arthritis, multiple sclerosis, diabetes mellitis, inflammatory bowel disease, Crohn's disease, etc. Immune suppression can also be used to reduce rejection of tissue or organ transplants and grafts and to treat T-cell, B-cell or monocyte-specific leukemias or lymphomas, and other cancers, by inhibiting proliferation of the affected cell type. Moreover IL-31 and IL-31Cys mutants can be used to detect monocytes, macrophages, and activated T-cells and aid in the diagnosis of such autoimmuine disease, particularly in disease states where monocytes are elevated or activated.

IL-31 and IL-31Cys mutants polypeptides, peptides, antibodies, and the like may also be used within diagnostic systems for the detection of circulating levels of IL-31. Within a related embodiment, antibodies or other agents that specifically bind to IL-31 polypeptides can be used to detect circulating IL-31 polypeptides. Elevated or depressed levels of ligand polypeptides may be indicative of pathological conditions, including cancer. IL-31 polypeptides may contribute to pathologic processes and can be an indirect marker of an underlying disease.

Also, the IL-31 and IL-31Cys mutants can be used to detect or target its receptor(s) in certain disease states. For example, elevated levels of soluble IL-2 receptor in human serum have been associated with a wide variety of inflammatory and neoplastic conditions, such as myocardial infarction, asthma, myasthenia gravis, rheumatoid arthritis, acute T-cell leukemia, B-cell lymphomas, chronic lymphocytic leukemia, colon cancer, breast cancer, and ovarian cancer (Heaney et al., Blood 87:847-857, 1996). Similarly, zcytor17 receptor is elevated in activated monocytes, and hence zcytor17receptor and/or its soluble receptors may be associated with or serve as a marker for inflammatory and neoplastic conditions associated therewith. The IL-31 and IL-31Cys mutants, including cytotoxic conjugates, hence can be used to detect or target such tissues, and disease states.

The molecules of the present invention have particular use in the monocyte/macrophage arm of the immune system. Methods are known that can assess such activity. For example, interferon gamma (IFNγ) is a potent activator of mononuclear phagocytes. For example, an increase in expression of zcytor17 upon activation of THP-1 cells (ATCC No. TIB-202) with interferon gamma could suggest that this receptor is involved in monocyte activation. Monocytes are incompletely differentiated cells that migrate to various tissues where they mature and become macrophages. Macrophages play a central role in the immune response by presenting antigen to lymphocytes and play a supportive role as accessory cells to lymphocytes by secreting numerous cytokines. Macrophages can internalize extracellular molecules and upon activation have an increased ability to kill intracellular microorganisms and tumor cells. Activated macrophages are also involved in stimulating acute or local inflammation. Moreover, monocyte-macrophage function has been shown to be abnormal in a variety of diseased states. For example see, Johnston, RB, New Eng. J. Med. 318:747-752,1998.

One of skill in the art would recognize that agonists of zcytor17 receptor, such as IL-31 and IL-31Cys mutants, are useful. For example, depressed migration of monocytes has been reported in populations with a predisposition to infection, such as newborn infants, patients receiving corticosteroid or other immunosuppressive therapy, and patients with diabetes mellitus, burns, or AIDS. Agonists for zcytor17, such as IL-31 and IL-31Cys mutants, could result in an increase in the ability of monocytes to migrate and possibly prevent infection in these populations. There is also a profound defect of phagocytic killing by mononuclear phagocytes from patients with chronic granulomatous disease. This results in the formation of subcutaneous abscesses, as well as abscesses in the liver, lungs, spleen, and lymph nodes. An agonist of zcytor17 receptor such as IL-31 and IL-31Cys mutants, could correct or improve this phagocytic defect. In addition, defective monocyte cytotoxicity has been reported in patients with cancer and Wiskott-Aldrich syndrome (eczema, thrombocytopenia, and recurrent infections). Activation of monocytes by agonists of zcytor17 receptor such as IL-31 and IL-31Cys mutants, could aid in treatment of these conditions. The monocyte-macrophage system is prominently involved in several lipid-storage diseases (sphingolipidoses) such as Gaucher's disease. Resistance to infection can be impaired because of a defect in macrophage function, which could be treated by agonists to zcytor17 receptor such as IL-31 and IL-31Cys mutants.

Moreover, one of skill in the art would recognize that antagonists of IL-31 and IL-31Cys mutants are useful. For example, in atherosclerotic lesions, one of the first abnormalities is localization of monocyte/macrophages to endothelial cells. These lesions could be prevented by use of antagonists to IL-31 and IL-31Cys mutants. Anti-IL-31 and IL-31Cys mutants antibodies (e.g., IL-31 and IL-31Cys mutants neutralizing antibody), zcytor17 soluble receptors, heterodimers and multimers, and IL-31 and IL-31Cys mutants binding partners can also be used as antagonists to the IL-31 and IL-31Cys mutants. Moreover, monoblastic leukemia is associated with a variety of clinical abnormalities that reflect the release of the biologic products of the macrophage, examples include high levels of lysozyme in the serum and urine and high fevers. Moreover, such leukemias exhibit an abnormal increase of monocytic cells. These effects could possibly be prevented by antagonists to IL-31 and IL-31Cys mutants, such as described herein. Moreover, anti- IL-31 and IL-31Cys mutants can be conjugated to molecules such as toxic moieties and cytokines, as described herein to direct the killing of leukemia monocytic cells.

Using methods known in the art, and disclosed herein, one of skill could readily assess the activity of IL-31 and IL-31Cys mutants agonists and antagonists in the disease states disclosed herein, inflammation, immune (e.g., autoimmune), cancer, or infection as well as other disease states involving monocytic cells. In addition, as IL-31 is expressed in a T-cell, macrophage and monocyte-specific manner, and these diseases involve abnormalities in monocytic cells, such as cell proliferation, function, localization, and activation, the polynucleotides, polypeptides, and antibodies of the present invention can be used to as diagnostics to detect such monocytic cell abnormalities, and indicate the presence of disease. Such methods involve taking a biological sample from a patient, such as blood, saliva, or biopsy, and comparing it to a normal control sample. Histological, cytological, flow cytometric, biochemical and other methods can be used to determine the relative levels or localization of IL-31, or cells expressing IL-31, i.e., monocytes, in the patient sample compared to the normal control. A change in the level (increase or decrease) of IL-31 expression, or a change in number or localization of monocytes (e.g., increase or infiltration of monocytic cells in tissues where they are not normally present) compared to a control would be indicative of disease. Such diagnostic methods can also include using radiometric, fluorescent, and colorimetric tags attached to polynucleotides, polypeptides or antibodies of the present invention. Such methods are well known in the art and disclosed herein.

Amino acid sequences having IL-31 and IL-31Cys mutants activity can be used to modulate the immune system by binding zcytor17 receptor, and thus, preventing the binding of IL-31 with endogenous IL-31 receptor. IL-31 and IL-31Cys mutants antagonists, such as anti- IL-31 and IL-31Cys mutants antibodies, can also be used to modulate the immune system by inhibiting the binding of IL-31 and IL-31Cys mutants with the endogenous IL-31 and IL-31Cys mutants receptor. Accordingly, the present specification describes the use of proteins, polypeptides, and peptides having IL-31 activity (such as IL-31 polypeptides, IL-31 analogs (*e*.*g*., anti- IL-31 anti-idiotype antibodies), and IL-31 fusion proteins) to a subject which lacks an adequate amount of this polypeptide, or which produces an excess of zcytor17 comprising receptor(s). Zcytor17. antagonists (*e*.*g*., anti-Zcytor17 antibodies) can be also used to treat a subject which produces an excess of either IL-31 or Zcytor17 comprising receptor(s). Suitable subjects include mammals, such as humans.

IL-31 has been shown to be expressed in activated mononuclear cells, and may be involved in regulating inflammation. As such, polypeptides of the present invention can be assayed and used for their ability to modify inflammation, or can be used as a marker for inflammation. Methods to determine pro-inflammatory and antiinflammatory qualities of Il-31 are known in the art and discussed herein. Moreover, it may be involved in up-regulating the production of acute phase reactants, such as serum amyloid A (SAA), α1-antichymotrypsin, and haptoglobin, and that expression of zcytor17 receptor ligand may be increased upon injection of lipopalysaccharide (LPS) *in vivo* that are involved in inflammatory response (Dumoutier, L. et al., Proc. Nat'1. Acad. Sci. 97:10144-10149, 2000). Production of acute phase proteins, such as SAA, is considered s short-term survival mechanism where inflammation is beneficial; however, maintenance of acute phase proteins for longer periods contributes to chronic inflammation and can be harmful to human health. For review, see Uhlar, CM and Whitehead, AS, Eur. J. Biochem. 265:501-523, 1999, and Baumann H. and Gauldie, J. Immunology Today 15:74-80, 1994. Moreover, the acute phase protein SAA is implicated in the pathogenesis of several chronic inflammatory diseases, is implicated in atherosclerosis and rheumatoid arthritis, and is the precursor to the amyloid A protein deposited in amyloidosis (Uhlar, CM and Whitehead, sura.). Thus, where a ligand such as IL-31 and IL-31Cys mutants that act as a pro-inflammatory molecule and induce production of SAA, antagonists would be useful in treating inflammatory disease and other diseases associated with acute phase response proteins induced by the ligand. Such antagonists are described herein. For example, a method of reducing inflammation comprises administering to a mammal with inflammation an amount of a composition of IL-31 and IL-31Cys mutants, or anti- IL-31 antibody (e.g., neutralizing antibody) that is sufficient to reduce inflammation. Moreover, a method of suppressing an inflammatory response in a mammal with inflammation can comprise: (1) determining a level of serum amyloid A protein; (2) administering a composition comprising a IL-31 and IL-31Cys mutants polypeptide or anti- IL-31 and IL-31Cys mutants antibody as described herein in an acceptable pharmaceutical carrier; (3) determining a post administration level of serum amyloid A protein; (4) comparing the level of serum amyloid A protein in step (1) to the level of serum amyloid A protein in step (3), wherein a lack of increase or a decrease in serum amyloid A protein level is indicative of suppressing an inflammatory response.

Like IL-31, analysis of the tissue distribution of the mRNA corresponding to its zcytor17 receptor cDNA showed that mRNA level was highest in monocytes and prostate cells, and is elevated in activated monocytes, and activated CD4+, activated CD8+, and activated CD3+ cells. Hence, zcytor17 receptor is also implicated in inducing inflammatory and immune response. Thus, the present specification describes use of IL-31 and IL-31Cys mutants-antibodies, and IL-31 and IL-31Cys mutants, as well as soluble zcytor17 receptor heterodimers as antagonists in inflammatory and immune diseases or conditions such as, pancreatitis, type I diabetes (IDDM), pancreatic cancer, pancreatitis, Graves Disease, inflammatory bowel disease (IBD), Crohn's Disease, colon and intestinal cancer, diverticulosis, autoimmune disease, sepsis, organ or bone marrow transplant; inflammation due to trauma, sugery or infection; amyloidosis; splenomegaly; graft versus host disease; and where inhibition of inflammation, immune suppression, reduction of proliferation of hematopoietic, immune, inflammatory or lymphoid cells, macrophages, T-cells (including Th1 and Th2 cells, CD4+ and CD8+ cells), suppression of immune response to a pathogen or antigen. Moreover the presence of zeytor17 receptor and IL-31 expression in activated immune cells such as activated CD3+, monocytes, CD4+ and CD19+ cells showed that zcytor17 receptor may be involved in the body's immune defensive reactions against foreign invaders: such as. microorganisms and cell debris, and could play a role in immune responses during inflammation and cancer formation. As such, IL-31 and IL-31Cys mutants and IL-31-antibodies of the present invention that are agonistic or antagonistic to zcytor17 receptor function, can be used to modify immune response and inflammation.

Moreover, IL-31 and IL-31Cys mutants polypeptides that bind zcytor17 receptor polypeptides, and antibodies thereto are useful to:
1) Antagonize or block signaling via zcytor17-comprising receptors in the treatment of acute inflammation, inflammation as a result of trauma, tissue injury, surgery, sepsis or infection, and chronic inflammatory diseases such as asthma, inflammatory bowel disease (IBD), chronic colitis, splenomegaly, rheumatoid arthritis, recurrent acute inflammatory episodes (e.g., tuberculosis), and treatment of amyloidosis, and atherosclerosis, Castleman's Disease, asthma, and other diseases associated with the induction of acute-phase response.
2) Antagonize or block signaling via the zcytor17 receptor receptors in the treatment of autoimmune diseases such as IDDM, multiple sclerosis (MS), systemic Lupus erythematosus (SLE), myasthenia gravis, rheumatoid arthritis, and IBD to prevent or inhibit signaling in immune cells (e.g. lymphocytes, monocytes, leukocytes) via zcytor17 receptor (Hughes C et al., J. Immunol 153: 3319-3325, 1994). Alternatively antibodies, such as monoclonal antibodies (MAb) to IL-31 and IL-31Cys mutants, can also be used as an antagonist to deplete unwanted immune cells to treat autoimmune disease. Asthma, allergy and other atopic disease may be treated with an MAb against, for example, anti-IL-31 and IL-31Cys mutants antibodies, soluble zcytor17 receptor soluble receptors or zcytor17/CRF2-4 heterodimers, to inhibit the immune response or to deplete offending cells. Blocking or inhibiting signaling via zcytor17, using the polypeptides and antibodies of the present invention, may also benefit diseases of the pancreas, kidney, pituitary and neuronal cells. IDDM, NIDDM, pancreatitis, and pancreatic carcinoma may benefit. Zcytor17 may serve as a target for MAb therapy of cancer where an antagonizing MAb inhibits cancer growth and targets immune-mediated killing. (Holliger P, and Hoogenboom, H: Nature Biotech. 16: 1015-1016, 1998). Mabs to soluble zcytor17 receptor monomers, homodimers, heterodimers and multimers may also be useful to treat nephropathies such as glomerulosclerosis, membranous neuropathy, amyloidosis (which also affects the kidney among other tissues), renal arteriosclerosis, glomerulonephritis of various origins, fibroproliferative diseases of the kidney, as well as kidney dysfunction associated with SLE, IDDM, type II diabetes (NIDDM), renal tumors and other diseases.
3) Agonize or initiate signaling via the zcytor17 receptors in the treatment of autoimmune diseases such as IDDM, MS, SLE, myasthenia gravis, rheumatoid arthritis, and IBD. IL-31 and IL-31Cys mutants may signal lymphocytes or other immune cells to differentiate, alter proliferation, or change production of cytokines or cell surface proteins that ameliorate autoimmunity. Specifically, modulation of a T-helper cell response to an alternate pattern of cytokine secretion may deviate an autoimmune response to ameliorate disease (Smith JA et al., J. Immunol. 160:4841-4849, 1998). Similarly, IL-31 and IL-31Cys mutants may be used to signal, deplete and deviate immune cells involved in asthma, allergy and atopoic disease. Signaling via zcytor17 receptor may also benefit diseases of the pancreas, kidney, pituitary and neuronal cells. IDDM, NIDDM, pancreatitis, and pancreatic carcinoma may benefit. Zcytor17 may serve as a target for MAb therapy of pancreatic cancer where a signaling MAb inhibits cancer growth and targets immune-mediated killing (Tutt, AL et al., J Immunol. 161: 3175-3185, 1998). Similarly T-cell specific leukemias, lymphomas, plasma cell dyscrasia (e.g., multiple myeloma), and carcinoma may be treated with monoclonal antibodies (e.g., neutralizing antibody) to zcytor17-comprising soluble receptors of the present invention.

Anti-IL-31 and IL-31Cys mutants antibodies, soluble zcytor17 receptor monomeric, homodimeric, heterodimeric and multimeric polypeptides described herein can be used to neutralize/block zcytor17 receptor ligand activity in the treatment of autoimmune disease, atopic disease, NIDDM, pancreatitis and kidney dysfunction as described above. A soluble form of zcytor17 receptor may be used to promote an antibody response mediated by T cells and/or to promote the production of IL-4 or other cytokines by lymphocytes or other immune cells.

Anti-IL-31 and IL-31Cys mutants antibodies, and soluble zcytor17-comprising receptors are useful as antagonists of IL-31 and IL-31Cys mutants. Such antagonistic effects can be achieved by direct neutralization or binding of its natural ligand. In addition to antagonistic uses, the soluble receptors can bind IL-31 and IL-31Cys mutants and act as carrier or carrier proteins, in order to transport IL-31 and IL-31Cys mutants to different tissues, organs, and cells within the body. As such, the soluble receptors can be fused or coupled to molecules, polypeptides or chemical moieties that direct the soluble-receptor-Ligand complex to a specific site, such as a tissue, specific immune cell, monocytes, or tumor. For example, in acute infection or some cancers, benefit may result from induction of inflammation and local acute phase response proteins. Thus, the soluble receptors described herein or antibodies of the present invention can be used to specifically direct the action of a pro-inflammatory IL-31 and IL-31Cys mutants ligand. See, Cosman, D. Cytokine 5: 95-106, 1993; and Fernandez-Botran, R. Exp. Opin. Invest. Drugs 9:497-513, 2000.

IL-31 and IL-31Cys mutants may activate the immune system which would be important in boosting immunity to infectious diseases, treating immunocompromised patients, such as HIV+ patients, cancer patients, or in improving vaccines. In particular, IL-31 and IL-31Cys mutants stimulation or expansion of monocytes/macrophages, T-cells, B-cells, NK cells, or their progenitors, would provide therapeutic value in treatment of viral infection, and as an anti-neoplastic factor. Similarly, IL-31 and IL-31Cys mutants stimulation of the immune response against viral and non-viral pathogenic agents (including bacteria, protozoa, and fungi) would provide therapeutic value in treatment of such infections by inhibiting the growth of such infections agents. Determining directly or indirectly the levels of a pathogen or antigen, such as a tumor cell, present in the body can be achieved by a number of methods known in the art and described herein.

Experimental evidence suggests a role for IL-31 in the progression of diseases that involve the skin or epithelium of internal surfaces, such as, for instance, large intestine, small intestine, pancrease, lung, prostate, uterus, and the like. First, as disclosed herein, zcytor17 receptors, including both OSM receptor beta and zcytor17, are expressed in several cell types located in epithelial surfaces including cell lines derived from lung epithelium, lung fibroblast, prostate, colon, breast, liver epithelium, bone and skin epithelium, bone fibroblast, and the like. Moreover, as disclosed herein, examples from each of these cell types also responded to IL-31 activation of a STAT reporter construct. In addition, several cell lines responded to IL-31 stimulation by producing increased levels of IL-6, IL-8, MCP-1 (a chemotactic factor) as described herein. In whole, these data suggest a role for IL-31 and IL-31Cys mutants in diseases that involve the epithelium such as, for instance, atopic dermatitis; dermatitis; psoriasis; psoriatic arthritis; eczema; gingivitis; peridontal disease; inflammatory bowel diseases (IBD) (e.g., ulcerative colitis, Crohn's disease); reproductive disorders, such as, for instance, cervical dysplasia, cervical cancer; other skin diseases like cancers: sarcomas; canrcinomas; melanoma, etc. i.e., not just inflammatory diseases, since immune system is involved in activating/curing cancers; diseases involving barrier dysfunction, such as, for instance, graft-versus-host disease (GVHD) and irritable bowel syndrome (IBS); and diseases that involve lung epithelium, such as asthma, emphysema, and the like. In addition, the release of cytokines IL-6, IL-8, and MCP-1 by cells exposed to IL-31 suggests that IL-31 is involved in inflammation. Therefore, regulation of IL-31 and IL-31Cys mutants can be useful in the treatment of autoimmune, inflammatory, or cancerous diseases associated with the tissues that express receptor. These diseases include, for example, prostatitis, hepatitis, osteoarthritis, and the like. IL-31 may positively or negatively directly or indirectly regulate these diseases. Therefore, the administration of IL-31 and IL-31Cys mutants can be used to treat diseases as described herein directly or with molecules that inhibit IL-31 and IL-31Cys mutants activity including, for example, both monoclonal antibodies to IL-31 and IL-31Cys mutants or monoclonal antibodies to zcytor17, or monoclonal antibodies that recognize the zcytor17 and OSM receptor beta complex.

Data also suggests that IL-31 may be involved in the regulation of TH2 T cell mediated diseases. First, IL-31 is made by the TH2 subset of activated T cells. TH2 cells express more IL-31 as compared to TH1 cells. In addition, at least two lung epithelial cell lines (SK-LU-1, A549) were stimulated to increase IL13 receptor alpha-2 mRNA in response to zcyto17 ligand stimulation as described herein. There is an association of IL-13 receptor alpha2 chain and tumorigenicity of human breast and pancreatic tumors. This suggests that IL-31 and IL-31Cys mutants may play a role in regulating tumorigenicity of these types of cancers, as well as other cancers. Therefore, the administration of a IL-31 and IL-31Cys mutants antagonist or direct use of IL-31 and IL-31Cys mutants may be useful in treatment of these types of cancers, benign or malignant and at various grades (grades I-IV) and stages (e.g., TNM or AJC staging methods) of tumor development, in mammals, preferably humans.

It is well-known in the art that IL13 is involved in the generation of activated TH2 cells and in TH2 mediated diseases, such as asthma, atopic dermatitis, and the like. IL-31 and IL-31Cys mutants or IL-31 and IL-31Cys mutants antagonists may be useful in the treatment of diseases that involved TH2 T cells. This would include diseases such as, for instance, atopic dermatitis, asthma, as well as other diseases that are exacerbated by activated TH2 cells. The involvement of IL-31 and IL-31Cys mutants in diseases, such as, for instance, atopic dermatitis, is also supported by the phenotype of the transgenic mice that overexpress IL-31 and IL-31Cys mutants and develop symptoms of atopic dermatitis as described herein.

Despite the preferential expression of IL-31 by TH2 cells, there is still some expression of IL-31 in TH1 cells and in CD8+ T cells. Therefore, IL-31 and IL-31Cys mutants or its antagonists may be useful in treating diseases that involve immune modulation of activated T cells including, for example, viral infection, cancers, graft rejection, and the like.

I1-31 may also be involved in the development of cancer. There is expression of the zcytor17 and OSM receptor beta receptors in human bone fibroblast osteosarcomas, human skin fibroblast melanoma, colon epithelial carcinoma, adenocarcinoma, breast epithelial adenocarcinoma, prostate epithelial adenosarcoma, and lung epithelial adenocarcinoma and carcinoma. Therefore, it may be useful to treat tumors of epithelial origin with either IL-31 and IL-31Cys mutants, fragments thereof, or IL-31 and IL-31Cys mutants antagonists which include, but are not limited to, carcinoma, adenocarcinoma, and melanoma. Notwithstanding, IL-31 and IL-31Cys mutants or a IL-31 and IL-31Cys mutants antagonist may be used to treat a cancer, or reduce one or more symptoms of a cancer, from a cancer including but not limited to squamous cell or epidermoid carcinoma, basal cell carcinoma, adenocarcinoma, papillary carcinoma, cystadenocarcinoma, bronchogenic carcinoma, bronchial adenoma, melanoma, renal cell carcinoma, hepatocellular carcinoma, transitional cell carcinoma, choriocarcinoma, seminoma, embryonal carcinoma, malignant mixed tumor of salivary gland origin, Wilms' tumor, immature teratoma, teratocarcinoma, and other tumors comprising at least some cells of epithelial origin.

Generally, the dosage of administered IL-31 and IL-31Cys mutants polypeptide (or zcytor17 analog or fusion protein) will vary depending upon such factors as the patient's age, weight, height, sex, general medical condition and previous medical history. Typically, it is desirable to provide the recipient with a dosage of IL-31 and IL-31Cys mutants polypeptide which is in the range of from about 1 pg/kg to 10 mg/kg (amount of agent/body weight of patient), although a lower or higher dosage also may be administered as circumstances dictate. One skilled in the art can readily determine such dosages, and adjustments thereto, using methods known in the art.

Administration of a IL-31 and IL-31Cys mutants polypeptide to a subject can be topical, inhalant, intravenous, intraarterial, intraperitoneal, intramuscular, subcutaneous, intrapleural, intrathecal, by perfusion through a regional catheter, or by direct intralesional injection. When administering therapeutic proteins by injection, the administration may be by continuous infusion or by single or multiple boluses.

Additional routes of administration include oral, mucosal-membrane, pulmonary, and transcutaneous. Oral delivery is suitable for polyester microspheres, zein microspheres, proteinoid microspheres, polycyanoacrylate microspheres, and lipid-based systems (see, for example, DiBase and Morrel, "Oral Delivery of Microencapsulated Proteins," in Protein Delivery: Playsical System, Sanders and Hendren (eds.), pages 255-288 (Plenum Press 1997)). The feasibility of an intranasal delivery is exemplified by such a mode of insulin administration (see, for example, Hinchcliffe and Illum, Adv. Drug Deliv. Rev. 35:199 (1999)). Dry or liquid particles comprising Il-31 and IL-31Cys mutants can be prepared and inhaled with the aid of dry-powder dispersers, liquid aerosol generators, or nebulizers (*e.g.,* Pettit and Gombotz, TIBTECH 16:343 (1998); Patton et al., Adv. Drug Deliv. Rev. 35:235 (1999)). This approach is illustrated by the AERX diabetes management system, which is a hand-held electronic inhaler that delivers aerosolized insulin into the lungs. Studies have shown that proteins as large as 48,000 kDa have been delivered across skin at therapeutic concentrations with the aid of low-frequency ultrasound, which illustrates the feasibility of trascutaneous administration (Mitragotri et al., Science 269:850 (1995)). Transdermal delivery using electroporation provides another means to administer a molecule having IL-31 and IL-31Cys mutants binding activity (Potts et al., Pharm. Biotechnol. 10:213 (1997)).

A pharmaceutical composition comprising a protein, polypeptide, or peptide having IL-31 and IL-31Cys mutants binding activity can be formulated according to known methods to prepare pharmaceutically useful compositions, whereby the therapeutic proteins are combined in a mixture with a pharmaceutically acceptable carrier. A composition is said to be a "pharmaceutically acceptable carrier" if its administration can be tolerated by a recipient patient. Sterile phosphate-buffered saline is one example of a pharmaceutically acceptable carrier. Other suitable carriers are well-known to those in the art. See, for example, Gennaro (ed.), Remington's Pharmaceutical Sciences, 19th Edition (Mack Publishing Company 1995).

For purposes of therapy, molecules having IL-31 and IL-31Cys mutants binding activity and a pharmaceutically acceptable carrier are administered to a patient in a therapeutically effective amount. A combination of a protein, polypeptide, or peptide having IL-31 and IL-31Cys mutants binding activity and a pharmaceutically acceptable carrier is said to be administered in a "therapeutically effective amount" if the amount administered is physiologically significant. An agent is physiologically significant if its presence results in a detectable change in the physiology of a recipient patient. For example, an agent used to treat inflammation is physiologically significant if its presence alleviates at least a portion of the inflammatory response.

A pharmaceutical composition comprising IL-31 and IL-31Cys mutants (or IL-31 and IL-31Cys mutants analog or fusion protein) can be furnished in liquid form, in an aerosol, or in solid form. Liquid forms, are illustrated by injectable solutions, aerosols, droplets, topological solutions and oral suspensions. Exemplary solid forms include capsules, tablets, and controlled-release forms. The latter form is illustrated by miniosmotic pumps and implants (Bremer et al., Pharm. Biotechnol. 10:239 (1997); Ranade, "Implants in Drug Delivery," in Drug Delivery Systems, Ranade and Hollinger (eds.), pages 95-123 (CRC Press 1995); Bremer et al., "Protein Delivery with Infusion Pumps," in Protein Delivery: Physical Systems, Sanders and Hendren (eds.), pages 239-254 (Plenum Press 1997); Yewey et al., "Delivery of Proteins from a Controlled Release Injectable Implant," in Protein Delivery: Physical Systems, Sanders and Hendren (eds.), pages 93-117 (Plenum Press 1997)). Other solid forms include creams, pastes, other topological applications, and the like.

The present specification describes chemically modified polypeptides having IL-31 and IL-31Cys mutants activity, such as a IL-31 and IL-31Cys mutants polypeptide, IL-31 and IL-31Cys mutants agonists, and IL-31 and IL-31Cys mutants antagonists, for example anti-IL-31 and IL-31Cys mutants antibodies, which a polypeptide is linked with a polymer, as discussed above.

Other dosage forms can be devised by those skilled in the art, as shown, for example, by Ansel and Popovich, Pharmaceutical Dotage Forms and Drug Delivery Systems, 5th Edition (Lea & Febiger 1990), Gennaro (ed.), Remington's Phamaceutical Sciences, 19th Edition (Mack Publishing Company 1995), and by Ranade and Hollinger, Drug Delivery Systems (CRC Press 1996).

As an illustration, pharmaceutical compositions may be supplied as a kit comprising a container that comprises a IL-31 and IL-31Cys mutants polypeptide or a IL-31 and IL-31Cys mutants antagonist (*e*.*g*., an antibody or antibody fragment that binds a IL-31 and IL-31Cys mutants polypeptide). Therapeutic polypeptides can be provided in the form of an injectable solution for single or multiple doses, or as a sterile powder that will be reconstituted before injection. Alternatively, such a kit can include a dry-powder disperser, liquid aerosol generator, or nebulizer for administration of a therapeutic polypeptide. Such a kit may further comprise written information on indications and usage of the pharmaceutical composition. Moreover, such information may include a statement that the IL-31 and IL-3ICys mutants composition is contraindicated in patients with known hypersensitivity to IL-31 and IL-31Cys mutants.

The invention is further illustrated by the following non-limiting examples.

### EXAMPLES

### Example 1

### Construction of mammalian expression vectors for IL-31-CEE

### A. Construction of human IL-31-CEE/pZMP21

An expression plasmid containing zcytor17lig-CEE was constructed via homologous recombination using a DNA fragment of zcytor17lig-CEE (SEQ ID NO: 31) and the expression vector pZMP21. The fragment was generated by PCR amplification using primers ZC41607 (SEQ ID NO:32) and ZC41605 (SEQ ID NO:33).

The PCR fragment zcytor17lig-CEE contains a zcytor17lig coding region, which was made using a previously generated clone of zcytor17lig as the template. The fragment includes a 5' overlap with the pZMP21 vector sequence, the zcytor17lig segment, a EE tag, and a 3' overlap with the pZMP21 vector. PCR conditions used were as follows: 1 cycle, 94°C, 5 minutes; 35 cycles, 94°C, 1 minute, followed by 55°C, 2 minutes, followed by 72°C, 3 minutes; 1 cycle, 72°C, 10 minutes.

The PCR reaction mixtures were run on a 1% agarose gel and a band corresponding to the sizes of the inserts were gel-extracted using a QIAquick™ Gel Extraction Kit (Qiagen, Cat. No. 28704).

Plasmid pZMP21 is a mammalian expression vector containing an expression cassette having the MPSV promoter, multiple restriction sites for insertion of coding sequences, and an otPA signal peptide sequence; an internal ribosome entry site (IRES) element from poliovirus, and the extracellular domain of CD8 truncated at the C-terminal end of the transmembrane domain; an E. coli origin of replication; a mammalian selectable marker expression unit comprising an SV40 promoter, enhancer and origin of replication, a DHFR gene, and the SV40 terminator; and URA3 and CEN-ARS sequences required for selection and replication in S. cerevisiae. pZMP21 is described in U. S. Patent Publication No. 20030232414 A1, and is deposited at the American Type Culture Collection, 10801 University Boulevard, Manassas, VA 20110-2209, designated as ATCC# PTA-5266.

The plasmid pZMP21 was cut with BgIII prior to recombination in yeast with the PCR fragment. One hundred microliters of competent yeast (S. cerevisiae) cells were independently combined with 10 µl of the insert DNA and 100ng of cut pZMP21 vector, and the mix was transferred to a 0.2-cm electroporation cuvette. The yeast/DNA mixture was electropulsed using power supply (BioRad Laboratories, Hercules, CA) settings of 0.75 kV (5 kV/cm), ∞ ohms, and 25 µF. Six hundred µl of 1.2 M sorbitol was added to the cuvette, and the yeast was plated in a 100-µl and 300µl aliquot onto two URA-D plates and incubated at 30°C. After about 72 hours, the Ura+ yeast transformants from a single plate were resuspended in 1 ml H₂O and spun briefly to pellet the yeast cells. The cell pellet was resuspended in 0.5 ml of lysis buffer (2% Triton X-100, 1% SDS, 100 mM NaCl, 10 mM Tris, pH 8.0, 1 mM EDTA). The five hundred microliters of the lysis mixture was added to an Eppendorf tube containing 250 µl acid-washed glass beads and 300 µl phenolchloroform, was vortexed for 3 minutes, and spun for 5 minutes in an Eppendorf centrifuge at maximum speed. Three hundred microliters of the aqueous phase was transferred to a fresh tube, and the DNA was precipitated with 600 µl ethanol (EtOH), followed by centrifugation for 30 minutes at maximum speed. The tube was decanted and the pellet was washed with 1 mL of 70% ethanol. The tube was decanted and the DNA pellet was resuspended in 30 µl TE.

Transformation of electrocompetent E. coli host cells (DH12S) was done using 5 µl of the yeast DNA prep and 50 µl of cells. The cells were electropulsed at 2.0 kV, 25 µF, and 400 ohms. Following electroporation, 1 ml SOC (2% Bacto™ Tryptone (Difco, Detroit, MI), 0.5% yeast extract (Difco), 10 mM NaCl, 2.5 mM KC1, 10 mM MgCl2, 10 mM MgSO4, 20 mM glucose) was added and then the cells were plated in a 50 µl and a 200 µl aliquot on two LB AMP plates (LB broth (Lennox), 1.8% Bacto™ Agar (Difco), 100 mg/L Ampicillin).

The inserts of three clones for the construct were subjected to sequence analysis and one clone for each construct, containing the correct sequence, was selected. Larger scale plasmid DNA was isolated using a commercially available kit (QIAGEN Plasmid Mega Kit, Qiagen, Valencia, CA) according to manufacturer's instructions.

### B. Construction of murine IL-31-CEE/pZMP21

An expression plasmid containing murine zcytor17lig-CEE was constructed in the same manner except the DNA fragment of murine zcytor17lig-CEE (SEQ ID NO: 34) was used with expression vector pZMP21. The fragment was generated by PCR amplification using primers ZC41643 (SEQ ID NO:35) and ZC41641 (SEQ ID NO:36). PCR and cloning conditions were the same as for the human construct.

### Example 2

### Transfection and Expression of human and murine IL-31-CEE

Human and murine zcytor17lig-CEE protein were produced in BHK cells transfected with human or murine zcytor17lig-CEE/pZMP21 (Example 1). BHK 570 cells (ATCC CRL-10314) were plated in T75 tissue culture flasks and allowed to grow to approximately 50 to 70% confluence at 37°C, 5% CO2, in growth media (SL7V4, 3%FBS, 1% pen/strep). The cells were then transfected with human or murine zcytor17Lig-CEE/pZMP21 by liposome-mediated transfection (using LipofectamineTM; Life Technologies), in serum free (SF) media (SL7V4). The plasmid (16µg) was diluted into 1.5 ml tubes to a total final volume of 640 µl with SF media. 35 µl of the lipid mixture was mixed with 605 µl of SF medium, and the resulting mixture was allowed to incubate approximately 15 minutes at room temperature. Five milliliters of SF media was then added to the DNA:lipid mixture. The cells were rinsed once with 10 ml of PBS, the PBS was decanted, and the DNA:lipid mixture was added. The cells were incubated at 37°C for five hours, then 15 ml of media (SL7V4, 3% FBS, 1% pen/strep) was added to each plate. The plates were incubated at 37°C overnight, and the DNA:lipid media mixture was replaced with selection media (SL7V4, 3% FBS, 1% pen/strep, 1µM methotrexate) the next day. Approximately 10 days post-transfection, methotrexate-resistant colonies from the T75 transfection flask were trypsinized, and the cells were pooled and plated into a T-162 flask and transferred to large-scale culture.

### Example 3

### Purification of human IL-31-CEE from BHK

Five hundred ml of resin is equilibrated by allowing the resin to settle, decanting the supernatant, and adding an equal volume of PBS. The resin is then gently slurried, transferred to a BioRad glass econo-column fitted with a stopcock and again allowed to settle. This step is repeated three times. The resin is then prepared for binding anti-EE antibody by washing in the same manner as above with 4 resin volumes of 200mM TEA pH 8.2, 1 CV at a time. The prepared resin is then transferred to a roller bottle and the Anti EE antibody is added. If the resulting slurry appears too thick, 200mM TEA pH 8.2 is added up to a 1:1 ratio of resin to liquid. The batch is allowed to bind overnight at 4° C while slowly rolling.

The cross-linking of the bound resin can be performed either at room temp or 4° C. The slurry is transferred to an appropriately sized glass econo-column fitted with a stopcock. The unbound material is collected via gravity flow. The resin is washed with 2CV of 200mM TEA pH 8.2, collecting in an appropriate vessel. The resin is transferred back to a roller bottle, taking out 50uL if analyzing coupling efficiency via SDS-PAGE gel. 36mgs of DMP is cross-linked to 1mL of resin by dissolving 18g of DMP in 100mL of 200mM TEA pH 8.2 and immediately adding it to the roller bottle containing the resin. If the slurry is thick, 200mM TEA pH 8.2 is added up to a 1:1 ratio of resin to liquid. This bottle is kept for at least 1 hour at room temp or overnight at 4° C.

The cross-linking reaction is terminated by transferring the slurry back to the glass column and washing with 2CV of 20mM Ethanolamine, 200mM TEA pH 8.2, and then with 4 CV of PBS. By knowing the amount of antibody used, the coupling efficiency can be determined via three methods: densitometry of SDS-PAGE gel using purified antibody as standard, RP-HPLC, or UV-Vis using an extinction coefficient of 1.44.

Affinity Resin is stored in either PBS with 0.05% Sodium Azide (short term) or 20% ethanol (long term). Storage is at 4° C.

Affinity Capture Chromatography is performed as follows: Delivered media is captured on the Anti-EE affinity resin and eluted via competition using EE peptide in physiological conditions. A low pH wash is employed to elute non-specific contaminants. Maximum pressure drop over the column should not exceed 1Mpa.

Chromatography Parameters are as follows: 175mL Anti-EE Affinity resin is packed in a Waters AP-5x200 glass column. The system is a Akta Explorer Workstation. The Equilibration Buffer (A) is 50mM NaPO4 (70:30 dibasic:monobasic), 120mM NaCl pH 7.2. The Elution Buffer (B) is 50mM NaPO4 (70:30 dibasic:monobasic), 0.28mg/mL EE Peptide, 120mM NaCl pH 7.2. The Wash Buffer (C): 0.1M Glycine pH 3.0. The Wash Buffer (D) is 50mM NaPO4 (70:30 dibasic:monobasic), 600mM NaCl pH 7.2. Temperature is 4°C. Flow Direction is downward. Flow Rates are load at 25cm/hr, elution at 15.3cm/hr, wash at 61.1cm/hr. Wavelengths of 215nm and 280nm are monitored. UV Averaging Timeis 0.1s. Fraction size is 25mL.

The column is cleaned and washed prior to loading media by washing the column with 1CV of wash buffer C, followed by 1 CV of wash buffer D, and then equilibrate in buffer A. The media is loaded over the column, followed by washing the column for 10CV using Buffer A (equilibration buffer).

Elution is via competition using EE peptide: two column volumes (CV) elution buffer B, 2CV of buffer A, and cleaned with 1 CV of each Buffer C and Buffer D. The column is regenerated with 2CV of Buffer A.

The eluate pool from the Anti-EE affinity column is concentrated to a volume less than 3% of the size exclusion column (10mL).

Concentration Parameters are as follows: The system is a Millipore Stirred Ultrafiltration Cell 8200. The membrane is YM 10 63.5mm. The membrane MWCOis 10kDa. The feed pressure is 50-55psi.

The system is set up according to the manufacturer's instructions. 50mM NaPO4 (70:30 dibasic:monobasic), 109mM NaCl pH 7.3 is allowed to run through the system for 5-10minutes. Any remaining solution is poured out.

The Superdex™ 75 pool is poured into the reservoir and concentrated to <10mL

The concentrate is aspirated using a pipette, and the membrane washed with 2mL of 50mM NaPO4 (70:30 dibasic:monobasic), 109mM NaCl pH 7.3

The chased solution is added to the concentrate- not to exceed a total concentrate volume of 10mL. The stirred cell is washed with DI water, and then soaked overnight in 0.5M NaOH. The unit is then thoroughly washed with DI water and stored in 20% ethanol

The concentrated affinity pool is injected over a Superdex™ 75 Prep Grade Column. The injection is never more than 3% of the volume of the column. The run will separate the high weight contaminants from the bulk of the zcytor17lig CEE and will buffer exchange the purified target into the current formulation buffer. Two pools are generated, one being highly pure zcytor17lig CEE and the other being somewhat impure. This impure pool is re-concentrated and reinjected to better separate the contaminants, and the resulting product is added to the first highly pure zcytor17lig CEE to yield the final product.

Chromatography Parameters are as follows. The column is 318mL Superdex™ 75 Prep Grade Column 26/60. The system is Akta Explorer. Elution Buffer is 50mM NaPO4 (70:30 dibasic:monobasic), 109mM NaCl pH 7.3. Temperature is 4°C. Flow Direction is downward. Flow Rate is 30.6cm/hr. Injection Volumeis <10mL. Wavelengths of 215nm and 280nm are monitored. UV Averaging Timeis 0.1s. Fraction Size is 2.5mL.

The affinity concentrate is loaded into a 10mL Superloop Injection of loop over column at flow rate specified, with isocratic elution using 1.5CV of elution buffer.

Pooling is determined via reducing silver stained SDS-PAGE gel. Two pools typically made - one being highly pure product, while the other being somewhat impure. This impure product is put through purification steps 4 and 5 a second time to generate the best possible purity. The column is cleaned in upward flow at 30cm/hr, 2CV each of 0.5M NaOH, 0.5M Tris pH 7.0, and Elution Buffer with 0.02% NaN3.

The eluate pool from the Superdex™ 75 column is concentrated to 1mg/mL, if needed. If the pool is already at 1mg/mL by RP-HPLC or BCA, then proceed directly to sterilization and characterization.

Concentration Parameters are as follows. The system is Millipore Amicon Ultra Device. The membrane is Ultracel Regenerated Cellulose. Membrane MWCO is 10kDa. Device Size is 15mL. Centrifuge speed is3000rpm. Temperature is 4° C.

The Superdex™ 75 pool is added to the device, cap, and spun at 10 minute intervals. The pool is added until the desired volume is reached to make a 1mg/mL solution. Determination of protein concentration is achieved via RP-HPLC analysis, BCA, or A280nm UV-Vis.

The purified zcytor17lig CEE is 0.2µm filtered under sterile conditions. Once filtered, aliquots are taken out for the various analytical and in vitro assays used to characterize the protein. The bulk protein is frozen at -80°C during this time.

Following this procedure, human IL-31CEE had a final recovery of 42%, resulting in 3.71-4.0mgs of Anti-EE bound per mL of Protein G Sepharose.

### Example 4

### Transfection and Expression of murine IL-31-CEE

Zcytor17ligm-CEE was purified using a mammalian BHK 570 expression system to provide a reagent for biological studies. During purification, a large amount of aggregate was present after the capture step which separated from the monomer using size exclusion chromatography. The final prep was highly glycosylated and had two predominant glycosylated forms visible on coomassie SDS-PAGE.

A total of 117 mg of zcytor17ligm-CEE was purified from 75L of BHK570 expressing factories.

All purification steps were performed at 4° C.

Five harvests were separately loaded and eluted from the capture step.

A harvest of 15 liters from factories was direct loaded onto antiEE-CNBR-Sepharose FF equilibrated with 7 mM Na Phosphate, pH 7.3, 1.5 mM KH2PO4, 2 mM KCl, 140 mM NaCl. The 50 ml column dimensions were 20mmD X160mmL. The harvest was loaded at a flow rate of 3.9-5.9 mL/minute. The protein was step eluted at a flow rate of 10 mL/minute using 0.1 M acetate, 0.5 M NaCL, pH 3. The fractions were immediately neutralized with 2 M Tris, pH 8. The pool of zcytor17ligm-CEE was determined by the A280nm peak. A small amount of pool was assayed on RP-HPLC, SDS -PAGE and western. The pool was then frozen, until the next 4 harvests were captured on the affinity step.

After the final harvest was delivered and the zcytor17ligm-CEE was captured and eluted from the affinity column, all pools were thawed, and then combined.

The combined eluate pool was then concentrated using a 5000 MWCO polyethersulfone filter in a Amicon Stirred Cell for a total concentration of 38X.

The concentrate was divided into two separate loads for the Superdex 75. The column volume was 180mL, dimensions= 16mmD X 900mmL. The column was equilibrated with 7.0 mM Na2H2PO4, pH7.3, 1.5 mM KH2PO4, 2mM KCl, 140 mM NaCl. The flow rate was 1 mL/minute. Fractions were collected, based on Coomassie SDS-PAGE and western data, a final pool of 47 mLs was made. This pool was sterile filtered and aliquotted.

### Example 5

### Construction of E. coli expression vectors for IL-31

### A. Construction of IL-31 cysteine mutant: human IL-31 C108S/pTAP433

The human IL31 C108S expression construct was generated as follows. The first 350 bases of the native IL31 sequence were generated by PCR amplification using pTAP433 as template and oligonucleotide primers zc43,156 ( SEQ ID NO:37) and zc 45,307 (SEQ ID NO:38). The region from base 302 to 421 was generated by PCR amplification using pTAP433 as template and oligonucleotide primers zc43,137 (SEQ ID NO:39) and zc45,306 (SEQ ID NO:40). The PCR conditions were as follows: 25 cycles at 94°C for 30 seconds, 50°C for 30 seconds, and 72°C for 1 minute; followed by a 4°C soak. These two DNA fragments were mixed together and were precipitated with 2 volume absolute ethanol. Pellet was resuspended in 10 µL H₂O and used for recombination into Sma1 cut recipient vector, pTAP238 to produce the constructs encoding human IL31 C108S. The resulting clones were designated as pCHAN7. They were digested with NotI (10 µl DNA, 5 µl buffer 3 New England BioLabs, 2 µL NotI, 33 µL H₂O for 1 hour at 37°C) and religated with T4 DNA ligase buffer (7 µL of the previous digest, 2 µL of 5X buffer, 1 µL of T4 DNA ligase). This step removed the yeast sequence, CEN-ARS, to streamline the vector. Aliquots of the DNA were digested with PvuII and PstI to confirm the absence of the yeast sequence. The human IL31 C108S expression constructs were transformed into *E. coli* strain W3110. The polynucleotide sequence for human IL-31 C108S cysteine mutant is shown in SEQ ID NO:41. The corresponding polypeptide sequence is shown in SEQ ID NO:42.

### B. Construction of IL-31 cysteine mutant: murine IL-31 C108S/nTAP433

The murine IL31 C108S expression construct was generated as follow. The first 350 bases of the native IL31 sequence were generated by PCR amplification using pTAP433 as template and oligonucleotide primers zc43,883 (SEQ ID NO:43) and zc 45,302 (SEQ ID NO:44). The region from base 302 to 406 was generated by PCR amplification using pTAP433 as template and oligonucleotide primers zc43,875 (SEQ ID NO:45) and zc45,303 (SEQ ID NO:46). The PCR conditions were as follows: 25 cycles at 94°C for 30 seconds, 50°C for 30 seconds, and 72°C for 1 minute; followed by a 4°C soak. These two DNA fragments were mixed together and were precipitated with 2 volume absolute ethanol. Pellet was resuspended in 10 µL H₂O and used for recombination into Sma1 cut recipient vector, pTAP238 to produce the constructs encoding murine IL31 C108S. The resulting clones were designated as pCHAN8. They were digested with NotI (10 µl DNA, 5 µl buffer 3 New England BioLabs, 2 µL NotI, 33 µL H₂O for 1 hour at 37°C) and religated with T4 DNA ligase buffer (7 µL of the previous digest, 2 µL of 5X buffer, 1 µL of T4 DNA ligase). This step removed the yeast sequence, CEN-ARS, to streamline the vector. Aliquots of the DNA were digested with PvuII and PstI to confirm the absence of the yeast sequence. The murine IL31 C108S expression constructs were transformed into *E. coli* strain W3110. The polynucleotide sequence for murine IL-31 C108S cysteine mutant is shown in SEQ ID NO:47. The corresponding polypeptide sequence is shown in SEQ ID NO:48.

### Example 6

### Refolding and purification of human IL-31 Ligand following expression in E.coli

E. coli cells transfected with human IL-31 polynucleotide are thawed in a beaker and 4 ml ice cold lysis buffer per gram wet weight of cells is added. The cells are kept cool by placing the beaker on ice in an ice bucket.

The cells are homogenized using a Polytron tissue-grinder homogenizer until all clumps are disrupted, then lysed with two passes through a APV 2000 @ 8500 -9000 psi keeping the cell suspension chilled to 4 °C. An aliquot of whole cell lysate is saved for SDS PAGE. The viscosity of the suspension is reduced by sonicating 5 min. at full power with 50% duty cycle (on for 5 sec, off for 5 sec) using an ultrasonic homogenizer or make a third pass through the APV. The lysed cell suspension is clarified by centrifugation for 30 min. at 22,000 x g (12,000 rpm in a JA-14 rotor in a Beckman J2-21M centrifuge), 4 °C. Unbroken cells, large cellular debris, and the inclusion body protein are pelleted by centrifugation.

The supernatant is carefully poured off from the pellet. Using a tissue homogenizer, the pellet is suspended with 4 to 6 ml wash buffer per gram wet weight cells. Complete homogenization of the pellet is important to wash out soluble proteins and cellular components. Removal of cell wall and outer membrane material can be improved by increasing the amount of wash solution to 10 ml per gram cells.

The suspension is centrifuged for 30 min at 22,000 x g (12,000 rpm in JA-14), at 4 °C. The supernatant is discarded and, using the tissue homogenizer, the pellet is suspended in 4 to 6 ml wash buffer per gram, wet weight of cells. This step is repeated 2 two more times. If the supernatant is still cloudy or colored, the washing is continued until the supernatant is clear. The pellet is suspended with wash buffer minus the urea, using 4 to 6 ml buffer per gram wet cells. Centrifuge 30 min at 22,000 x g (12,000 rpm in (JA-14 rotor), 4 °C. If necessary the washed pellets can be stored at -80 °C.

Using the tissue homogenizer, the pellet is suspended with guanidine-HCl-containing extraction buffer. If the extract will be subjected to gel filtration, 0.5 to 1.0 ml buffer per gram wet weight of original cells is used. If the extract will be used in protein folding procedures, 2 to 4 ml buffer is used. This step is performed at room temperature then allowed to gently agitate overnight at 4 °C. The suspension is centrifuged 1 hr at 35,000 x g at 4 °C. The supernatant is carefully poured off from the pellet and filtered through a 0.45-um filter. The clarified inclusion body extract is used for preparing folded protein. The extract can be stored at -80 °C until required.

The inclusion bodies are diluted into the following buffer: 0.75 M Arginine, PEG 3350 0.055 % (w/v) ; 10.56 mM NaCl; 0.44 mM KCl; 2.2 mM MgCl2; 2.2 mM CaCl2; 0.055 M Tris at pH 8.2 (room temperature pH). The redox pair and concentrations in this refold buffer are as follows: [GSH] = 1 mM:[GSSG] =0.1 mM. The redox pair is added to the buffer immediately prior to dilution of the solubilized inclusion bodies. 18 ml of the soluble inclusion bodies 12 mg/ml (By RP HPLC assay) are added drop-wise, at room temperature, to 2250 ml of the above refold buffer with vigorous stirring. The final target protein concentration during refolding is 0.10 mg/ml. Following dilution, the vessel was capped and allowed to gently stir at room temperature for 16 hours. At this point the RP HPLC assay indicates two sharp peaks in roughly equivalent quantities. The earliest eluting peak is the S-glutathiolyated adduct of the free Cys (Odd Cysteine residue in native sequence). The next peak of similar area is the free Cys moiety. The reaction is quenched through addition of Acetic acid to 25 mM and titration of the pH down to pH 5.2. The refold reaction is now ready for HIC capture of the product. The quenched, titrated refold media was passed through 0.45micron filtration prior to loading the butyl HIC column for product capture.

The quenched refold reaction (pH 5.5) was 0.45 micron filtered. The entire filtered preparation is fed to a bed of Toso Haas Butyl 650-M (2cm. dia. 23 ml bed) at 30 ml/min via in-line proportioning with 2 M (NH4)2S04; 25 mM Acetic acid @ pH 5.2 as the diluent (room temperature process). The ratio for proportioning is 62.5 % refold reaction to 37.5% diluent (to deliver 0.75 M (NH4)2S04 nominal feed conc.). No target was passed during the load under the operational parameters. The column was washed to baseline with 62.5 % 25 mM Acetic acid: 37.5 % 2M(NH4)2 S04; 25mM Acetic acid (to deliver 0.75 M (NH4)2S04; 25 mM Acetic acid @ pH 5.2). Upon completing the wash, a 3 CV gradient from the wash condition to 25 mM Acetic acid; 25 mM MOPS; 25 mM Boric acid @ pH 5.2 ("multibuffer") is initiated. During this conversion to low ionic strength, little protein elutes from the HIC matrix. Upon washing for another 5CV an ascending pH Gradient (over 10 CV) is formed between the pH 5.2 "multibuffer" and the same multibuffer at pH 8.65. During this ascending pH gradient the target protein elutes with a maxima occurring ~ @ pH 6.2, followed by a slight bump during tailing fractions at higher pH. By SDS-PAGE analysis, the eluting material is monomeric and exhibits a mobility shift when reduced and non-reduced SDS-PAGE samples are compared. The later fractions (tailing bump) reveal some higher order multimers which are excluded from the pooled monomeric fractions.

The HIC pool is adjusted to 20 mM Tris and the pH is adjusted to 7.8. Thus adjusted, the material is loaded directly to a Poros HQ 50 anion exchange bed (1cm. dia. 14 ml vol.) at 8 ml / min. The column is equilibrated in 20 mM Tris at pH 7.8 (Buffer A). No protein target is passed under these conditions as determined by RP HPLC assay on the column effluent. Upon completing the load, the bed was washed with equilibration buffer for 10 CV prior to initiating a 20 CV gradient formed between equilibration buffer (Buffer A) and the same buffer containing 0.5 M NaCl ( Buffer B) (exactly 0% to 60 % B over 20 CV). Very early in this gradient a sharp symmetric peak elutes followed by a large broad peak. By SDS-PAGE and HPLC analysis the first peak is the monomeric form, the second peak contains mostly dimeric and higher order molecular weight species. The protein in each peak is separately pooled and concentrated for application to the SEC column step.

Each of the AIEX pools was concentrated and injected to a 120 ml bed (1.6 cm. dia.) of Superdex 75 equilibrated in 50 mM Na Phosphate; 109 mM NaCl @ pH 7.0. The SEC profile for AIEX peak 1 application exhibits some pre-main peak optical density that becomes a symmetric peak eluting between 0.6 and 0.7 CV and is monomeric by SDS-PAGE analysis. The SEC profile for AIEX peak 2 application elutes between 0.4 and 0.6 CV and contains dimeric as well as higher molecular weight species. The eluate from application of AIEX pool 1 is pooled between 0.6 and 0.7 CV. This material is predominantly monomeric IL31h Ligand as seen in the SDS-PAGE coomassie stained gels of final product. The peak fractions were pooled; 0.2 micron sterile filtered and stored as such at 4 °C for 2 days prior to aliquoting and freezing at - 80 °C. Aliquots are submitted for AAA, N-terminal sequence determination, Endotoxin testing and SEC-MALLS analysis.

### Example 7

### Refolding and Purification of human IL-31 C108S from E.coli expression

E. coli cells transfected with human IL-31 C108S mutant polynucleotide sequence are thawed in a beaker and 4 ml ice cold lysis buffer per gram wet weight of cells is added. The bacterial cells are kept cool by placing the beaker on ice in an ice bucket.

The cells are homogenized using a Polytron tissue-grinder homogenizer until all clumps are disrupted. The cells are lysed with two passes through the APV 2000 @ 8500 -9000 psi keeping the cell suspension chilled to 4°C. An aliquot of whole cell lysate is saved for SDS PAGE. The viscosity of the suspension is reduced by sonicating 5 min at full power with 50% duty cycle (on for 5 sec, off for 5 sec) using an ultrasonic homogenizer or make a third pass through the APV. The lysed cell suspension is clarified by centrifugation for 30 min. at 22,000 x g (12,000 rpm in a JA-14 rotor in a Beckman J2-21M centrifuge), 4 °C. Unbroken cells, large cellular debris, and the inclusion body protein are pelleted by centrifugation.

The supernatant is carefully poured from the pellet, which is suspended using a tissue homogenizer with 4 to 6 ml wash buffer per gram wet weight cells. Complete homogenization of the pellet is important to wash out soluble proteins and cellular components. Removal of cell wall and outer membrane material can be improved by increasing the amount of wash solution to 10 ml per gram cells.

The suspension is centrifuged 30 min at 22,000 x g (12,000 rpm in JA-14), 4∞C. The supernatant is discarded and, using the tissue homogenizer, the pellet is suspended in 4 to 6 ml wash buffer per gram, wet weight of cells. This step is repeated two more times.

If the supernatant is still cloudy or colored, the washing is continued until the supernatant is clear. The pellet is suspended with wash buffer minus the urea, using 4 to 6 ml buffer per gram wet cells, and centrifuged 30 min at 22,000 x g (12,000 rpm in (JA-14 rotor), 4 o C. If necessary the washed pellets can be stored at -80 °C. It is better to store material at this stage rather than after the extraction stage.

The pellet is suspended using the tissue homogenizer with guanidine-HCI-containing extraction buffer. If the extract will be subjected to gel filtration, 0.5 to 1.0 ml buffer per gram wet weight of original cells is used. If the extract will be used in protein folding procedures, 2 to 4 ml buffer is used. This step is performed at room temperature then allow to gently agitate overnight at 4 °C. The suspension is centrifuged 1 hr at 35,000 x g at 4 °C. The supernatant is carefully poured off from the pellet and filtered through a 0.45-um filter. The clarified inclusion body extract is used for preparing folded protein. The extract is stored at -80 °C until required.

### Refolding and purification

The inclusion bodies are diluted into the following buffer: 0.75 M Arginine, PEG 3350 0.055 % (w/v), 20 % glycerol; 10.56 mM NaCl; 0.44 mM KCl; 2.2 mM MgCl2; 2.2 mM CaCl2; 0.055 M Tris at pH 8.2 (room temperature pH). The redox pair and concentrations in this refold buffer are as follows: [DTT] = 1.25 mM:[Cystamine] = 0.5 mM. The redox pair is added to the buffer immediately prior to dilution of the solubilized inclusion bodies. 16 ml of the soluble inclusion bodies @ 28.6 mg/ ml (By RP HPLC assay) are added drop-wise, at room temperature, to 3200 ml of the above refold buffer with vigorous stirring. The final target protein concentration during refolding is 0.15 mg/ml. Following dilution, the vessel is capped and allowed to gently stir at room temperature for 16 hours. At this point the RP HPLC assay indicates a single sharp peak. The reaction is quenched through addition of Acetic acid to 25 mM and titration of the pH down to pH 5.2. The refold reaction is now ready for HIC capture of the product. The quenched, titrated refold media was passed through 0.45micron filtration prior to loading the butyl HIC column for product capture.

The quenched refold reaction (pH 5.5) was 0.45 micron filtered. The entire filtered preparation is fed to a bed of Toso Haas Butyl 650-M (2cm. dia. 29 ml bed) at 30 ml/min via in-line proportioning with 2 M (NH4)2S04; 25 mM Acetic acid @ pH 5.2 as the diluent (room temperature process). The ratio for proportioning is 62.5 % refold reaction to 37.5 % diluent (to deliver 0.75 M (NH4)2S04 nominal feed conc.).

The feed stream behaves ideal during the HIC column loading, zero deviation in operational pressure was observed throughout the entire load. No target was passed during the load under the operational parameters. The column is washed to baseline (20 CV) with 62.5 % 25 mM Acetic acid: 37.5 % 2M(NH4)2 S04; 25mM Acetic acid (to deliver 0.75 M (NH4)2S04; 25 mM Acetic acid @ pH 5.2). Upon completing the wash, a 3 CV gradient from the wash condition to 25 mM Acetic acid; 25 mM MOPS; 25 mM Boric acid @ pH 5.2 (multibuffer A) is initiated. During this conversion to low ionic strength, little protein elutes from the HIC matrix. Upon washing for another 5CV an ascending pH Gradient (over 30 CV) is formed between the pH 5.2 multibuffer A and the same multibuffer at pH 8.65 (multibuffer B). During this ascending pH gradient the target protein elutes with a maxima occurring ~ @ pH 6.2, followed by a slight bump during tailing fractions at higher pH. By SDS-PAGE analysis, the early eluting material is monomeric and exhibits a mobility shift when reduced and non-reduced SDS-PAGE samples are compared. The later fractions (tailing bump) reveal higher order multimers and were excluded from the pooled monomeric fractions.

The HIC pool is adjusted to 20 mM Tris and the pH is adjusted to 7.8. Thus adjusted, the material is loaded directly to a Poros HQ 50 anion exchange bed (2cm. dia. 44 ml vol) at 30 ml / min. The column is equilibrated in 20 mM Tris at pH 7.8 (Buffer A). No protein target is passed under these conditions as determined by RP HPLC assay on the column effluent. Upon completing the load, the bed was washed with equilibration buffer for 10 CV prior to initiating a 15 CV gradient formed between equilibration buffer (Buffer A) and the same buffer containing 0.5 M NaCl (Buffer B) (exactly 0% to 60 % B over 20 CV). Very early in this gradient a sharp symmetric peak elutes followed by a broad low level peak. By SDS-PAGE and HPLC analysis the early symmetric peak is the product, in monomeric form, whilst the later, low level absorption, broad peak is mostly aggregate species not completely excluded from the pool generated in the previous HIC step. The protein in the symmetric peak is pooled and concentrated for application to the SEC column step.

The Poros HQ 50 AIEX pool was concentrated and injected to a 320 ml bed (2.6 cm. dia.) of Superdex 75 equilibrated in 50 mM Na Phosphate; 109 mM NaCl @ pH 7.0. The protein eluted as a sharp symmetric peak @ ~ 0.55-0.6 CV and there was no detectable multimer in any fraction by HPLC and overloaded SDS-PAGE coomassie stained gels. The peak fractions were pooled; 0.2 micron sterile filtered and stored as such at 4 degrees for 2 days prior to aliquoting and freezing at - 80 °C. Aliquots are submitted for AAA, N-terminal sequence determination, Endotoxin testing and SEC-MALLS analysis.

### Example 8

Refolding and purification of murine IL-31 Ligand following expression in E.coli

E. coli cells transfected with murine IL-31 polynucleotide sequence are thawed in a beaker and 4 ml ice cold lysis buffer per gram wet weight of cells is added. The bacterial cells are kept cool by placing the beaker on ice in an ice bucket. The cells are homogenized using a Polytron tissue-grinder homogenizer until all clumps are disrupted. The cells are lysed with two passes through the APV 2000 @ 8500 -9000 psi while keeping the cell suspension chilled to 4 °C. An aliquot of whole cell lysate is saved for SDS PAGE. The viscosity of the suspension is reduced by sonicating 5 min at full power with 50% duty cycle (on for 5 sec, off for 5 sec) using an ultrasonic homogenizer or make a third pass through the APV.

Clarify the lysed cell suspension by centrifugation for 30 min. at 22,000 x g (12,000 rpm in a JA-14 rotor in a Beckman J2-21M centrifuge), 4 °C. Unbroken cells, large cellular debris, and the inclusion body protein are pelleted by centrifugation.

The supernatant is carefully poured off from the pellet, which is suspend with a tissue homogenizer and 4 to 6 ml wash buffer per gram wet weight cells. Complete homogenization of the pellet is important to wash out soluble proteins and cellular components. Removal of cell wall and outer membrane material can be improved by increasing the amount of wash solution to 10 ml per gram cells. The suspension is centrifuged the 30 min at 22,000 x g (12,000 rpm in JA-14), 4 °C. The supernatant is discarded and, using the tissue homogenizer, the pellet is suspended in 4 to 6 ml wash buffer per gram, wet weight of cells. This step is repeated two more times. If the supernatant is still cloudy or colored, the washing is continued until the supernatant is clear. The pellet is suspended with wash buffer minus the urea, using 4 to 6 ml buffer per gram wet cells and centrifuged 30 min at 22,000 x g (12,000 rpm in (JA-14 rotor), 4 °C.

If necessary the washed pellets can be stored at -80 °C.

The pellet is suspended using the tissue homogenizer with guanidine-HCl-containing extraction buffer. If the extract will be subjected to gel filtration, 0.5 to 1.0 ml buffer per gram wet weight of original cells is used. If the extract will be used in protein folding procedures 2 to 4 ml buffer is used. This step is performed at room temperature then allow to gently agitate overnight at 4 °C. The suspension is centrifuged 1 hr at 35,000 x g at 4 °C. The supernatant is carefully poured off from the pellet and filtered through a 0.45-um filter. The clarified inclusion body extract is used for preparing folded protein. The extract can be stored at -80 °C until required.

The inclusion bodies are diluted into the following buffer: 0.75 M Arginine, PEG 3350 0.055 % (w/v), 20 % glycerol; 10.56 mM NaCl; 0.44 mM KCl; 2.2 mM MgCl2; 2.2 mM CaCl2; 0.055 M Tris at pH 8.2 (room temperature pH). The redox pair and concentrations in this refold buffer are as follows: [Cysteamine] = 1.25 mM: [Cystamine] = 0.5 mM. The redox pair is added to the buffer immediately prior to dilution of the solubilized inclusion bodies. 9.5 ml of the soluble inclusion bodies @ 15.4 mg/ ml (By RP HPLC assay) are added drop-wise, at room temperature, to 1600 ml of the above refold buffer with vigorous stirring. The final target protein concentration during refolding is 0.10 mg/ml. Following dilution, the vessel was caped and allowed to gently stir at room temperature for 16 hours. At this point the RP HPLC assay indicates a single sharp peak. The reaction is quenched through addition of Acetic acid to 25 mM and titration of the pH down to pH 5.2. The refold reaction is now ready for HIC capture of the product. The quenched, titrated refold media was passed through 0.45micron filtration prior to loading the butyl HIC column for product capture.

The quenched refold reaction (pH 5.5) was 0.45 micron filtered. The entire filtered preparation is fed to a bed of Toso Haas Butyl 650-M (2cm. dia. 30 ml bed) at 30 ml/min via in-line proportioning with 3 M (NH4)2S04; 25 mM Acetic acid @ pH 5.2 as the diluent (room temperature process). The ratio for proportioning is 75 % refold reaction to 25 % diluent (to deliver 0.75 M (NH4)2S04 nominal feed conc.).

The feed stream behaves ideal during the HIC column loading, zero deviation in operational pressure was observed throughout the entire load. About 8% of target was passed during the load under these operational parameters. The column was washed to baseline with 20 CV of 0.75 M(NH4)2 S04; 25mM Acetic acid buffer at pH 5.2. Upon completing the wash, a 3 CV gradient from the wash condition to 25 mM Acetic acid; 25 mM MOPS; 25 mM Boric acid @ pH 5.2 (multibuffer A) is initiated. During this conversion to low ionic strength, little protein elutes from the HIC matrix. Upon washing for another 5CV an ascending pH Gradient (over 10 CV) is formed between the pH 5.2 "multibuffer A" and the same buffer at pH 8.65 (multibuffer B). During the ascending pH gradient the target protein elutes with a maxima occurring around pH 6.2, followed by a slight bump during tailing fractions at higher pH. By SDS-PAGE analysis, the early eluting material is monomeric and exhibits a mobility shift when reduced and non-reduced SDS-PAGE samples are compared. The later fractions (tailing bump) reveal higher order multimers and were excluded from the pooled monomeric fractions.

The HIC pool is adjusted to 20 mM Tris and the pH is adjusted to 7.8. Thus adjusted, the material is loaded directly to a Poros HQ 50 anion exchange bed (1cm. dia 14 ml vol) at 8 ml / min. The column is equilibrated in 20 mM Tris at pH 7.8 (Buffer A). No protein target is passed under these conditions as determined by RP HPLC assay on the column effluent. Upon completing the load, the bed was washed with equilibration buffer for 10 CV prior to initiating a 20 CV gradient formed between equilibration buffer (Buffer A) and the same buffer containing 0.5 M NaCl (Buffer B), exactly 0% to 60 % Buffer B over 20 CV. Very early in this gradient a sharp symmetric peak elutes followed by a broad low level peak. By SDS-PAGE and HPLC analysis the early symmetric peak is the product, in monomeric form, whilst the later, low level absorption, broad peak is mostly aggregate species not completely excluded from the pool generated in the previous HIC step. The protein in the symmetric peak is pooled and concentrated for application to the SEC column step.

The Poros HQ 50 AIEX pool was concentrated and injected to a 120 ml bed (1.6 cm. dia) of Superdex 75 equilibrated in 50 mM Na Phosphate; 109 mM NaCl @ pH 7.0. The protein eluted as a sharp symmetric peak @ ~ 0.55-0.6 CV and there was no detectable multimer in any fraction by HPLC and overloaded SDS-PAGE coomassie stained gels. The peak fractions were pooled; 0.2 micron sterile filtered and stored as such at 4 degrees for 2 days prior to aliquoting and freezing at - 80 °C. Aliquots are submitted for AAA, N-terminal sequence determination, Endotoxin testing and SEC-MALLS analysis.

### Example 9

### Refolding and purification of murine IL-31 C108S following expression in E.coli

E. coli cells that have been transfected with the murine IL-31 C108S sequence are thawed in a beaker and 4 ml ice cold lysis buffer per gram wet weight of cells is added.

The bacterial cells cool by placing the beaker on ice in an ice bucket. The cells are homogenized using a Polytron tissue-grinder homogenizer until all clumps are disrupted.

The cells are lysed with two passes through the APV 2000 @ 8500 -9000 psi keeping the cell suspension chilled to 4°C, and an aliquot of whole cell lysate is saved for SDS PAGE. The viscosity of the suspension is reduced by sonicating 5 min at full power with 50% duty cycle (on for 5 sec, off for 5 sec) using an ultrasonic homogenizer or make a third pass through the APV. The lysed cell suspension is clarified by centrifugation for 30 min. at 22,000 x g (12,000 rpm in a JA-14 rotor in a Beckman J2-21M centrifuge), 40 C. Unbroken cells, large cellular debris, and the inclusion body protein are pelleted by centrifugation.

The supernatant is carefully poured off from the pellet, which is suspended using a tissue homogenizer in 4 to 6 ml wash buffer per gram wet weight cells. Complete homogenization of the pellet is important to wash out soluble proteins and cellular components. Removal of cell wall and outer membrane material can be improved by increasing the amount of wash solution to 10 ml per gram cells. The suspension is centrifuged for 30 min at 22,000 x g (12,000 rpm in JA-14), 4°C. The supernatant is discarded and the pellet is suspended using the tissue homogenizer in 4 to 6 ml wash buffer per gram, wet weight of cells. This step is repeated two more times. If the supernatant is still cloudy or colored, the pellet is washed until the supernatant is clear. The pellet is suspended with wash buffer minus the urea, using 4 to 6 ml buffer per gram wet cells, then centrifuged for 30 min at 22,000 x g (12,000 rpm in (JA-14 rotor), 4 °C.

If necessary the washed pellets can be stored at -80 ° C.

Using the tissue homogenizer, the pellet is suspended with guanidine-HCl-containing extraction buffer. If the extract will be subjected to gel filtration, 0.5 to 1.0 ml buffer per gram wet weight of original cells is used. If the extract will be used in protein folding procedures, 2 to 4 ml buffer is used. This step is performed at room temperature and allowed to gently agitate overnight at 4 0 C. The suspension is centrifuged for 1 hr at 35,000 x g at 4 °C. The supernatant is carefully poured off from the pellet, and the supernatant is filtered through a 0.45-um filter. The clarified inclusion body extract is used for preparing folded protein. The extract can be stored at -80 °C until required.

The inclusion bodies are diluted into the following buffer: 0.75 M Arginine, PEG 3350 0.055 % (w/v), 20 % glycerol; 10.56 mM NaCl; 0.44 mM KCl; 2.2 mM MgCl2; 2.2 mM CaCl2; 0.055 M Tris at pH 8.2 (room temperature pH). The redox pair and concentrations in this refold buffer are as follows: [DTT] = 0.5 mM:[Cystamine] = 0.2 mM. The redox pair is added to the buffer immediately prior to dilution of the solubilized inclusion bodies. 55 ml of the soluble inclusion bodies @ 47 mg/ ml (By RP HPLC assay) are added drop-wise, at room temperature, to 19 Liters of the above refold buffer with vigorous stirring. The final target protein concentration during refolding is 0.15 mg/ml. Following dilution, the vessel was caped and allowed to gently stir at room temperature for 16 hours. At this point the RP HPLC assay indicates a single sharp peak. The reaction is quenched through addition of Acetic acid to 25 mM and titration of the pH down to pH 5.2. The refold reaction is now ready for HIC capture of the product. The quenched, titrated refold media was passed through 0.45micron filtration prior to loading the butyl HIC column for product capture.

The quenched refold reaction (pH 5.5) was 0.45 micron filtered. The entire filtered preparation is fed to a bed of Toso Haas Butyl 650-M (5cm. dia. 190 ml bed) at 30 ml/min via in-line proportioning with 3 M (NH4)2S04; 25 mM Acetic acid @ pH 5.2 as the diluent (room temperature process). The ratio for proportioning is 75 % refold reaction to 25 % diluent (to deliver 0.75 M (NH4)2S04 nominal feed conc.).

The feed stream behaves ideal during the HIC column loading, zero deviation in operational pressure was observed throughout the entire load. About 8% of target was passed during the load under these operational parameters. The column was washed to baseline with 20 CV of 0.75 M(NH4)2 S04; 25mM Acetic acid buffer at pH 5.2. Upon completing the wash, a 3 CV gradient from the wash condition to 25 mM Acetic acid; 25 mM MOPS; 25 mM Boric acid @ pH 5.2 ("multibuffer A") is initiated. During this conversion to low ionic strength, little protein elutes from the HIC matrix. Upon washing for another 5CV an ascending pH Gradient (over 5 CV) is formed between the pH 5.2 "multibuffer A" and the same multibuffer at pH 8.65 (multibuffer B). During the ascending pH gradient the target protein elutes with a maxima occurring around pH 6.2, followed by a slight bump during tailing fractions at higher pH. By SDS-PAGE analysis, the early eluting material is monomeric and exhibits a mobility shift when reduced and non-reduced SDS-PAGE samples are compared. The later fractions (tailing bump) reveal higher order multimers and were excluded from the pooled monomeric fractions.

The HIC pool is adjusted to 20 mM Tris and the pH is adjusted to 7.8. Thus adjusted, the material is loaded directly to a Poros HQ 50 anion exchange bed (5cm. dia 366 ml vol) at 30 ml / min. The column is equilibrated in 20 mM Tris at pH 7.8 (Buffer A). No protein target is passed under these conditions as determined by RP HPLC assay on the column effluent. Upon completing the load, the bed was washed with equilibration buffer for 10 CV prior to initiating a 20 CV gradient formed between equilibration buffer (Buffer A) and the same buffer containing 0.5 M NaCl (Buffer B) (exactly 0% to 60 % B over 20 CV). Very early in this gradient a sharp symmetric peak elutes followed by a broad low level peak. By SDS-PAGE and HPLC analysis the early symmetric peak is the product, in monomeric form, whilst the later, low level absorption, broad peak is mostly aggregate species not completely excluded from the pool generated in the previous HIC step. The protein in the symmetric peak is pooled and concentrated for application to the SEC column step.

The Poros HQ 50 AIEX pool was concentrated and injected to a 320 ml bed (2.6 cm. dia) of Superdex 75 equilibrated in 50 mM Na Phosphate; 109 mM NaCl @ pH 7.0. The protein eluted as a sharp symmetric peak @ ~ 0.55-0.6 CV and there was no detectable multimer in any fraction by HPLC and overloaded SDS-PAGE Coomassie stained gels. The peak fractions were pooled; 0.2 micron sterile filtered and stored as such at 4 degrees for 2 days prior to aliquoting and freezing at - 80 °C. Aliquots are submitted for AAA, N-terminal sequence determination, Endotoxin testing and SEC-MALLS analysis.

### Example 10

### IL-31 Luciferase Activity assay

BAF3 cells transfected with zCYTOR17 (human or mouse), OSMRB (human or mouse), and KZ134 are grown to between 5x10⁵ - 1x10⁶ cells/mL. Cells are washed with assay media (RPMI 1640, 10% FBS, L-Glutamine, Sodium Pyruvate, and Pen/Strep) 1.5x and then resuspended at 3x10⁵ cell/mL in assay medium. In a 96 well opaque plate (Costar) standards of IL-31 are titered in duplicate from 600pg/mL to 9.38pg/mL in assay medium via 1:2 serial dilution, 100uL/well. Quality control standards are added in duplicate to the plate at 350pg/mL and 35pg/mL at 100uL/well. Samples are added in duplicate to the sample wells. 100uL of the washed cells are then added to each well for a final concentration of 3x10⁴ cells/well. This plate is then incubated 16-24 hours at 37C in a 5% CO2 incubator. The plate is then centrifuged at 1200RPM for 5 minutes. The media is flicked off and 25uL/well of lysis buffer (Promega) is added. After 10 minutes the plate is read on a luminometer (Berthold). The luminometer adds 40uL/well of luciferase substrate mix (Promega) and integrates the luminescence for period of 4 seconds.

Protein from E. coli transfected with human or mouse native polynucleotide sequence was compared to protein from E. coli transfected the human or mouse C108S mutantsu in this assay. The cysteine mutant material had equivalent activity in this assay as the native material.

### Example 11

In vivo activity of E.coli and BHK Produced IL-31

In this study 7 day osmotic mini-pumps filled with E. Coli derived zcytor17lig protein or BHK derived protein were used to examine whether E. Coli derived protein had the same in vivo activity and caused the same hair loss and scratching phenotype.

Balb/C mice were given 5, 1 or 0.2µg dose of IL-31 via osmotic mini pumps for 6 days, and monitored closely for signs of scratching and hair loss. The mice were divided into seven groups: Group 1 (n = 5), #401-405, s.c. implant of BHK produced zcytor17 ligand pump at µg/day; Group 2 (n = 5) #406-410, s.c. implant of BHK produced zcytor17 ligand pump at 1µg/day; Group 3 (n = 5) #411-415, s.c. implant of BHK produced zcytor177 ligand pump 0.2µg/day; Group 4 (n = 5) #416-420, s.c. implant of E. coli produced zcytor17 ligand ump 5µg/day; Group 5 (n = 5) #421-425, s.c. implant of E. coli produced zcytor17 ligand pump 1µg/day; Group 6 (n = 5) #426-430, s.c. implant of E. coli produced zcytor17 ligand pump 0.2µg/day; and Group 7 (n = 5) #431-435, s.c. implant of vehicle control (PBS/0.1%BSA). The Alzet 7 day pump model 1007D was used (Durect Corporation, Cupertino California). Protein was diluted with sterile PBS/0.1%BSA.

Mice were ear tagged prior to the start of the study. On day -1 Mice were anesthetized and pre-bleeds were collected via retro-orbital for serum collection. On day 0 Balb/C mice were given 5, 1 or 0.2 µg dose of zcytor17L via osmotic mini pumps. On days 1-5 mice were monitored closely every day for signs of scratching and hair loss. Each day the mice were given a visual score from 0 to 4, 0 = normal and 4 = severe hair loss / excessive. On day 6 the mice were visually scored then euthanized, serum was collected to test for expression of cytokines.

The results from the observations score showed that E. coli derived IL-31 gave the same phenotypic data as the BHK derived material.

## Claims

1. An isolated polypeptide comprising an amino acid sequence selected from the group consisting of SEQ ID NOs: 15, 18, 23, 28, and residue 2 to residue 133 of SEQ ID NO: 23.

2. An isolated polypeptide according to Claim 1, consisting of an amino acid sequence selected from the group consisting of SEQ ID NOs: 15, 18, 23, 28, and residue 2 to residue 133 of SEQ ID NO: 23.

3. The polypeptide according to Claim 1, wherein the polypeptide is pro-inflammatory.

4. The polypeptide according to Claim 1, wherein the amino acid sequence is SEQ ID NO: 15 or 18.

5. The polypeptide according to Claim 1, wherein the amino acid sequence is SEQ ID NO: 23 or 28.

6. An expression vector comprising the following operably linked elements:
a transcription promoter;
a DNA segment encoding a polypeptide of any preceding claim; and
a transcription terminator.

7. A cultured cell into which has been introduced an expression vector of Claim 6, wherein the cell expresses the polypeptide encoded by the DNA segment.

8. The cultured cell according to Claim 7, wherein the cultured cell is a prokaryotic cell.

9. The cultured cell according to Claim 8, wherein the cell is a gram negative cell, preferably wherein the cell is *E. coli,* more preferably wherein the *E. coli* cell is *E. coli* strain W3110

10. A process for producing a polypeptide comprising:
culturing a cell into which has been introduced an expression vector of Claim 6, wherein the cell expresses the polypeptide encoded by the DNA segment; and
recovering the expressed polypeptide.

11. An antibody or antibody fragment that specifically binds to a polypeptide of any of Claims 1-5.

12. The antibody of Claim 11, wherein the antibody is selected from the group consisting of a polyclonal antibody, a murine monoclonal antibody, a humanized antibody derived from a murine monoclonal antibody, an antibody fragment, neutralizing antibody, and a human monoclonal antibody.

13. The antibody fragment of Claim 11, wherein the antibody fragment is selected from the group consisting of F(ab'), F(ab), Fab', Fab, Fv, scFv, and minimal recognition unit.

14. An anti-idiotype antibody that specifically binds to the antibody of Claim 11.

15. A formulation comprising:
an isolated polypeptide according to any of Claims 1-5; and
a pharmaceutically acceptable vehicle.

16. A kit comprising the formulation of Claim 15.

## Patentansprüche

1. Isoliertes Polypeptid, das eine Aminosäuresequenz umfasst, die aus den SEQ ID Nrn. 15, 18, 23, 28 und Rest 2 bis Rest 133 der SEQ ID Nr.: 23 ausgewählt ist.

2. Isoliertes Polypeptid nach Anspruch 1, das aus einer Aminosäuresequenz besteht, die aus den SEQ ID Nrn.: 15, 18, 23, 28 und Rest 2 bis Rest 133 der SEQ ID Nr.: 23 ausgewählt ist.

3. Polypeptid nach Anspruch 1, wobei das Polypeptid entzündungsfördernd ist.

4. Polypeptid nach Anspruch 1, wobei die Aminosäuresequenz SEQ ID Nr.: 15 oder 18 ist.

5. Polypeptid nach Anspruch 1, wobei die Aminosäuresequenz SEQ ID Nr.: 23 oder 28 ist.

6. Expressionsvektor, der die folgenden funktionsfähig gebundenen Elemente umfasst:
einen Transkriptionspromotor;
ein DNA-Segment, das für ein Polypeptid nach einem der vorstehenden Ansprüche codiert; und
einen Transkriptionsterminator.

7. Kulturzelle, in die ein Expressionsvektor nach Anspruch 6 eingefügt wurde, wobei die Zelle das von dem DNA-Segment codierte Polypeptid exprimiert.

8. Kulturzelle nach Anspruch 7, wobei die Kulturzelle eine prokaryotische Zelle ist.

9. Kulturzelle nach Anspruch 8, wobei die Zelle eine Gram-negative Zelle ist, wobei die Zelle bevorzugt *E. coli* ist, wobei die *E. coli-Zelle* stärker bevorzugt *E*. *coli*-Stamm W3110 ist.

10. Verfahren zur Herstellung eines Polipeptids, das Folgendes umfasst:
Kultivieren einer Zelle, in die ein Expressionsvektor nach Anspruch 6 eingefügt wurde, wobei die Zelle das von dem DNA-Segment codierte Polypeptid exprimiert; und
Gewinnen des exprimierten Polypeptids.

11. Antikörper oder Antikörperfragment, das spezifisch an ein Polypeptid nach einem der Ansprüche 1-5 bindet.

12. Antikörper nach Anspruch 11, wobei der Antikörper aus einem polyklonalen Antikörper, einem murinen monoklonalen Antikörper, einem von einem murinen monoklonalen Antikörper abgeleiteten humanisierten Antikörper, einem Antikörperfragment, einem neutralisierenden Antikörper und einem humanen monoklonalen Antikörper ausgewählt ist.

13. Antikörperfragment nach Anspruch 11, wobei das Antikörperfragment aus der Gruppe bestehend aus F(ab'), F(ab), Fab', Fab, Fv, scFv und einer minimalen Erkennungseinheit ausgewählt ist.

14. Anti-idiotypischer Antikörper, der spezifisch an den Antikörper nach Anspruch 11 bindet.

15. Formulierung, die Folgendes umfasst:
ein isoliertes Polypeptid nach einem der Ansprüche 1-5; und
einen pharmazeutisch verträglichen Träger.

16. Kit, das die Formulierung nach Anspruch 15 umfasst.

## Revendications

1. Polypeptide isolé, comprenant une séquence d'acides aminés sélectionnée parmi le groupe constitué des SEQ ID N^{os} : 15, 18, 23, 28 et des résidus 2 à 133 de la SEQ ID N^{o}: 23.

2. Polypeptide isolé selon la revendication 1, constitué d'une séquence d'acides aminés sélectionnée parmi le groupe constitué des SEQ ID N^{os}: 15, 18, 23, 28 et des résidus 2 à 133 de la SEQ ID N^{o}: 23.

3. Polypeptide selon la revendication 1, dans lequel le polypeptide est pro-inflammatoire.

4. Polypeptide selon la revendication 1, dans lequel la séquence d'acides aminés est la SEQ ID N^{o}: 15 ou 18.

5. Polypeptide selon la revendication 1, dans lequel la séquence d'acides aminés est la SEQ ID N^{o}: 23 ou 28.

6. Vecteur d'expression comprenant les éléments fonctionnellement liés suivants:
un promoteur de transcription ;
un segment d'ADN encodant un polypeptide selon l'une quelconque des revendications précédentes ; et
un terminateur de transcription.

7. Cellule mise en culture dans laquelle a été introduit un vecteur d'expression selon la revendication 6, la cellule exprimant le polypeptide encodé par le segment d'ADN.

8. Cellule mise en culture selon la revendication 7, dans laquelle la cellule mise en culture est une cellule procaryote.

9. Cellule mise en culture selon la revendication 8, dans laquelle la cellule est une cellule Gram-négative, de préférence dans laquelle la cellule est *E. coli,* plus préférablement dans laquelle la cellule *E. coli* est une souche d'*E*. *coli* W3110.

10. Procédé pour produire un polypeptide comprenant :
la mise en culture d'une cellule dans laquelle un vecteur d'expression selon la revendication 6 a été introduit, la cellule exprimant le polypeptide encodé par le segment d'ADN ; et
la récupération du polypeptide exprimé.

11. Anticorps ou fragment d'anticorps qui se lie spécifiquement à un polypeptide selon l'une quelconque des revendications 1 à 5.

12. Anticorps selon la revendication 11, dans lequel l'anticorps est sélectionné parmi le groupe constitué d'un anticorps polyclonal, d'un anticorps mutin monoclonal, d'un anticorps humanisé dérivé d'un anticorps mutin monoclonal, d'un fragment d'anticorps, d'un anticorps neutralisant, et d'un anticorps humain monoclonal.

13. Fragment d'anticorps selon la revendication 11, dans lequel le fragment d'anticorps est sélectionné parmi le groupe constitué de F(ab'), F(ab), Fab', Fab, Fv, scFv, et d'une unité de reconnaissance minimale.

14. Anticorps anti-idiotype qui se lie spécifiquement à l'anticorps selon la revendication 11.

15. Formulation comprenant:
un polypeptide isolé selon l'une quelconque des revendications 1 à 5; et
un véhicule pharmaceutiquement acceptable.

16. Kit comprenant la formulation selon la revendication 15.


### SEQUENCE LISTING

<110> ZYMOGENETICS, INC. BRADY, LOWELL J. BUKOWSKI, THOMAS R. CHAN, CHUNG
<120> HOMOGENEOUS PREPARATIONS OF IL-31
<130> 05-01PC
<150> 60/648,189
   <151> 2005-06-28
<160> 49
<170> FastSEQ for Windows Version 4.0
<210> 1
   <211> 904
   <212> DNA
   <213> Homo sapiens
<220>
   <221> CDS
   <222> (28)...(519)
<400> 1
   ctgaagctgg ccttgctctc tctcgcc atg gcc tct cac tca ggc ccc tcg acg 54
                                 Met Ala Ser His Ser Gly Pro Ser Thr
                                  1               5
   tct gtg ctc ttt ctg ttc tgc tgc ctg gga ggc tgg ctg gcc tcc cac    102
   Ser Val Leu Phe Leu Phe Cys Cys Leu Gly Gly Trp Leu Ala Ser His
    10                  15                  20                  25
   acg ttg ccc gtc cgt tta cta cga cca agt gat gat gta cag aaa ata    150
   Thr Leu Pro Val Arg Leu Leu Arg Pro Ser Asp Asp Val Gln Lys Ile
                    30                  35                  40
   gtc gag gaa tta cag tcc ctc tcg aag atg ctt ttg aaa gat gtg gag    198
   Val Glu Glu Leu Gln Ser Leu Ser Lys Met Leu Leu Lys Asp Val Glu
                45                  50                  55
   gaa gag aag ggc gtg ctc gtg tcc cag aat tac acg ctg ccg tgt ctc    246
   Glu Glu Lys Gly Val Leu Val Ser Gln Asn Tyr Thr Leu Pro Cys Leu
            60                  65                  70
   agc cct gac gcc cag ccg cca aac aac atc cac agc cca gcc atc cgg    294
   Ser Pro Asp Ala Gln Pro Pro Asn Asn Ile His Ser Pro Ala Ile Arg
        75                  80                  85
   gca tat ctc aag aca atc aga cag cta gac aac aaa tct gtt att gat    342
   Ala Tyr Leu Lys Thr Ile Arg Gln Leu Asp Asn Lys Ser Val Ile Asp
    90                  95                 100                 105
   gag atc ata gag cac ctc gac aaa ctc ata ttt caa gat gca cca gaa    390
   Glu Ile Ile Glu His Leu Asp Lys Leu Ile Phe Gln Asp Ala Pro Glu
                   110                 115                 120
   aca aac att tct gtg cca aca gac acc cat gaa tgt aaa cgc ttc atc    438
   Thr Asn Ile Ser Val Pro Thr Asp Thr His Glu Cys Lys Arg Phe Ile
               125                 130                 135
   ctg act att tct caa cag ttt tca gag tgc atg gac ctc gca cta aaa   486
   Leu Thr Ile Ser Gln Gln Phe Ser Glu Cys Met Asp Leu Ala Leu Lys
           140                 145                 150
   tca ttg acc tct gga gcc caa cag gcc acc act taaggccatc tcttcctttc 539
   Ser Leu Thr Ser Gly Ala Gln Gln Ala Thr Thr
       155                 160
   ggattggcag gaacttaagg agccttaaaa agatgaccga cagctaagtg tgggaactct 599
   gccgtgattc cttaagtaca tttttccaat gaataatctc agggacccct catatgggct 659
   agtcccggga gggctgagat gtgaatttgt gaattacctt gaaaaacatt aggttattgt 719
   tattagtctt ggtatttatg gaatgctttt cttctgcagg cttaagtctt acttattata 779
   ccctcgtgag ggtgggaggt ggcagctatg ttaatttatt gatatttatt gtactaagag 839
   ttgtcaatgc tccctggggg agccctcgga atctatttaa taaattatat tgaatttttc 899
   tcata                                                             904
<210> 2
   <211> 164
   <212> PRT
   <213> Homo sapiens
<400> 2
   Met Ala Ser His Ser Gly Pro Ser Thr Ser Val Leu Phe Leu Phe Cys
    1               5                  10                  15
   Cys Leu Gly Gly Trp Leu Ala Ser His Thr Leu Pro Val Arg Leu Leu
               20                  25                  30
   Arg Pro Ser Asp Asp Val Gln Lys Ile Val Glu Glu Leu Gln Ser Leu
           35                  40                  45
   Ser Lys Met Leu Leu Lys Asp Val Glu Glu Glu Lys Gly Val Leu Val
       50                  55                  60
   Ser Gln Asn Tyr Thr Leu Pro Cys Leu Ser Pro Asp Ala Gln Pro Pro
   65                  70                  75                  80
   Asn Asn Ile His Ser Pro Ala Ile Arg Ala Tyr Leu Lys Thr Ile Arg
                   85                  90                  95
   Gln Leu Asp Asn Lys Ser Val Ile Asp Glu Ile Ile Glu His Leu Asp
               100                 105                 110
   Lys Leu Ile Phe Gln Asp Ala Pro Glu Thr Asn Ile Ser Val Pro Thr
           115                 120                 125
   Asp Thr His Glu Cys Lys Arg Phe Ile Leu Thr Ile Ser Gln Gln Phe
       130                 135                 140
   Ser Glu Cys Met Asp Leu Ala Leu Lys Ser Leu Thr Ser Gly Ala Gln
   145                 150                 155                 160
   Gln Ala Thr Thr
<210> 3
   <211> 492
   <212> DNA
   <213> Artificial Sequence
<220>
   <223> human Il-31 degenerate polynucleotide of SEQ ID NO:2
<221> misc_feature
   <222> 6, 9, 15, 18, 21, 24, 27, 30, 33, 36, 42, 54, 57, 60, 66,
   69, 72, 78, 81, 84, 87, 90, 93, 96, 99, 102, 105, 114
   <223> n = A,T,C or G
<221> misc_feature
   <222> 126, 135, 141, 144, 147, 156, 159, 168, 183, 186, 189, 192,
   195, 207, 210, 213, 219, 222, 225, 231, 237, 240, 255
<223> n = A,T,C or G
<221> misc_feature
   <222> 258, 261, 267, 270, 276, 282, 288, 294, 306, 309, 333, 342,
   357, 360, 366, 375, 378, 381, 384, 390, 405, 414, 417
   <223> n = A,T,C or G
<221> misc_feature
   <222> 423, 435, 450, 453, 456, 462, 465, 468, 471, 474, 477, 486,
   489, 492
   <223> n = A,T,C or G
<400> 3
   atggcnwsnc aywsnggncc nwsnacnwsn gtnytnttyy tnttytgytg yytnggnggn 60
   tggytngcnw sncayacnyt nccngtnmgn ytnytnmgnc cnwsngayga ygtncaraar 120
   athgtngarg arytncarws nytnwsnaar atgytnytna argaygtnga rgargaraar 180
   ggngtnytng tnwsncaraa ytayacnytn ccntgyytnw snccngaygc ncarccnccn 240
   aayaayathc aywsnccngc nathmgngcn tayytnaara cnathmgnca rytngayaay 300
   aarwsngtna thgaygarat hathgarcay ytngayaary tnathttyca rgaygcnccn 360
   garacnaaya thwsngtncc nacngayacn caygartgya armgnttyat hytnacnath 420
   wsncarcart tywsngartg yatggayytn gcnytnaarw snytnacnws nggngcncar 480
   cargcnacna cn                                                    492
<210> 4
   <211> 755
   <212> DNA
   <213> Mus musculus
<220>
   <221> CDS
   <222> (1)...(489)
<400> 4
   atg atc ttc cac aca gga aca acg aag cct acc ctg gtg ctg ctt tgc    48
   Met Ile Phe His Thr Gly Thr Thr Lys Pro Thr Leu Val Leu Leu Cys
    1               5                  10                  15
   tgt ata gga acc tgg ctg gcc acc tgc agc ttg tcc ttc ggt gcc cca    96
   Cys Ile Gly Thr Trp Leu Ala Thr Cys Ser Leu Ser Phe Gly Ala Pro
               20                  25                  30
   ata tcg aag gaa gac tta aga act aca att gac ctc ttg aaa caa gag    144
   Ile Ser Lys Glu Asp Leu Arg Thr Thr Ile Asp Leu Leu Lys Gln Glu
           35                  40                  45
   tct cag gat ctt tat aac aac tat agc ata aag cag gca tct ggg atg    192
   Ser Gln Asp Leu Tyr Asn Asn Tyr Ser Ile Lys Gln Ala Ser Gly Met
       50                  55                  60
   tca gca gac gaa tca ata cag ctg ccg tgt ttc agc ctg gac cgg gaa    240
   Ser Ala Asp Glu Ser Ile Gln Leu Pro Cys Phe Ser Leu Asp Arg Glu
   65                  70                  75                  80
   gca tta acc aac atc tcg gtc atc ata gca cat ctg gag aaa gtc aaa    288
   Ala Leu Thr Asn Ile Ser Val Ile Ile Ala His Leu Glu Lys Val Lys
                   85                  90                  95
   gtg ttg agc gag aac aca gta gat act tct tgg gtg ata aga tgg cta    336
   Val Leu Ser Glu Asn Thr Val Asp Thr Ser Trp Val Ile Arg Trp Leu
               100                 105                 110
   aca aac atc agc tgt ttc aac cca ctg aat tta aac att tct gtg cct    384
   Thr Asn Ile Ser Cys Phe Asn Pro Leu Asn Leu Asn Ile Ser Val Pro
           115                 120                 125
   gga aat act gat gaa tcc tat gat tgt aaa gtg ttc gtg ctt acg gtt    432
   Gly Asn Thr Asp Glu Ser Tyr Asp Cys Lys Val Phe Val Leu Thr Val
       130                 135                 140
   tta aag cag ttc tca aac tgc atg gca gaa ctg cag gct aag gac aat    480
   Leu Lys Gln Phe Ser Asn Cys Met Ala Glu Leu Gln Ala Lys Asp Asn
   145                 150                 155                 160
   act aca tgc tgagtgatgg gggggggggg ggtgcagtgt cctcagcagt              529
   Thr Thr Cys
   gcctgtcctt cgagggctga gcttgcaacc caggacttaa ctccaaaggg actgtgcggt 589
   cattactagt catgttattt atgtttttat tttgtccact gaaatcttgt tctgctaccc 649
   tgtagggact ggaagtggca gctatattta tttatttatg tactgagttt gttaacgctc 709
   catggaggag ccttcagagt ctatttaata aattatattg acatga                755
<210> 5
   <211> 163
   <212> PRT
   <213> Mus musculus
<400> 5
   Met Ile Phe His Thr Gly Thr Thr Lys Pro Thr Leu Val Leu Leu Cys
    1               5                  10                  15
   Cys Ile Gly Thr Trp Leu Ala Thr Cys Ser Leu Ser Phe Gly Ala Pro
               20                  25                  30
   Ile Ser Lys Glu Asp Leu Arg Thr Thr Ile Asp Leu Leu Lys Gln Glu
           35                  40                  45
   Ser Gln Asp Leu Tyr Asn Asn Tyr Ser Ile Lys Gln Ala Ser Gly Met
       50                  55                  60
   Ser Ala Asp Glu Ser Ile Gln Leu Pro Cys Phe Ser Leu Asp Arg Glu
   65                  70                  75                  80
   Ala Leu Thr Asn Ile Ser Val Ile Ile Ala His Leu Glu Lys Val Lys
                   85                  90                  95
   Val Leu Ser Glu Asn Thr Val Asp Thr Ser Trp Val Ile Arg Trp Leu
               100                 105                 110
   Thr Asn Ile Ser Cys Phe Asn Pro Leu Asn Leu Asn Ile Ser Val Pro
           115                 120                 125
   Gly Asn Thr Asp Glu Ser Tyr Asp Cys Lys Val Phe Val Leu Thr Val
       130                 135                 140
   Leu Lys Gln Phe Ser Asn Cys Met Ala Glu Leu Gln Ala Lys Asp Asn
   145                 150                 155                 160
   Thr Thr Cys
<210> 6
   <211> 489
   <212> DNA
   <213> Artificial Sequence
<220>
   <223> mouse Il-31 degenerate polynucleotide of SEQ ID NO:5
<221> misc_feature
   <222> 15, 18, 21, 24, 30, 33, 36, 39, 42, 45, 57, 60, 66, 69, 72,
   78, 81, 84, 90, 93, 96, 102, 114, 117, 120, 123, 132
   <223> n = A,T,C or G
<221> misc_feature
   <222> 135, 147, 156, 171, 183, 186, 189, 195, 198, 207, 216, 219,
   228, 231, 237, 243, 246, 249, 258, 261, 270, 276, 285
   <223> n = A,T,C or G
<221> misc_feature
   <222> 291, 294, 297, 306, 309, 315, 318, 324, 330, 336, 339, 348,
   360, 363, 369, 378, 381, 384, 387, 393, 402, 417, 423
   <223> n = A,T,C or G
<221> misc_feature
   <222> 426, 429, 432, 435, 447, 459, 465, 471, 483, 486
   <223> n = A,T,C or G
<400> 6
   atgathttyc ayacnggnac nacnaarccn acnytngtny tnytntgytg yathggnacn 60
   tggytngcna cntgywsnyt nwsnttyggn gcnccnathw snaargarga yytnmgnacn 120
   acnathgayy tnytnaarca rgarwsncar gayytntaya ayaaytayws nathaarcar 180
   gcnwsnggna tgwsngcnga ygarwsnath carytnccnt gyttywsnyt ngaymgngar 240
   gcnytnacna ayathwsngt nathathgcn cayytngara argtnaargt nytnwsngar 300
   aayacngtng ayacnwsntg ggtnathmgn tggytnacna ayathwsntg yttyaayccn 360
   ytnaayytna ayathwsngt nccnggnaay acngaygarw sntaygaytg yaargtntty 420
   gtnytnacng tnytnaarca rttywsnaay tgyatggcng arytncargc naargayaay 480
   acnacntgy                                                        489
<210> 7
   <211> 1557
   <212> DNA
   <213> Homo sapiens
<220>
   <221> CDS
   <222> (1)...(1557)
<400> 7
   atg atg tgg acc tgg gca ctg tgg atg ctc ccc tca ctc tgc aaa ttc    48
   Met Met Trp Thr Trp Ala Leu Trp Met Leu Pro Ser Leu Cys Lys Phe
    1               5                  10                  15
   agc ctg gca gct ctg cca gct aag cct gag aac att tcc tgt gtc tac    96
   Ser Leu Ala Ala Leu Pro Ala Lys Pro Glu Asn Ile Ser Cys Val Tyr
               20                  25                  30
   tac tat agg aaa aat tta acc tgc act tgg agt cca gga aag gaa acc   144
   Tyr Tyr Arg Lys Asn Leu Thr Cys Thr Trp Ser Pro Gly Lys Glu Thr
           35                  40                  45
   agt tat acc cag tac aca gtt aag aga act tac gct ttt gga gaa aaa   192
   Ser Tyr Thr Gln Tyr Thr Val Lys Arg Thr Tyr Ala Phe Gly Glu Lys
       50                  55                  60
   cat gat aat tgt aca acc aat agt tct aca agt gaa aat cgt gct tcg   240
   His Asp Asn Cys Thr Thr Asn Ser Ser Thr Ser Glu Asn Arg Ala Ser
    65                  70                  75                  80
   tgc tct ttt ttc ctt cca aga ata acg atc cca gat aat tat acc att   288
   Cys Ser Phe Phe Leu Pro Arg Ile Thr Ile Pro Asp Asn Tyr Thr Ile
                   85                  90                  95
   gag gtg gaa gct gaa aat gga gat ggt gta att aaa tct cat atg aca   336
   Glu Val Glu Ala Glu Asn Gly Asp Gly Val Ile Lys Ser His Met Thr
               100                 105                 110
   tac tgg aga tta gag aac ata gcg aaa act gaa cca cct aag att ttc   384
   Tyr Trp Arg Leu Glu Asn Ile Ala Lys Thr Glu Pro Pro Lys Ile Phe
           115                 120                 125
   cgt gtg aaa cca gtt ttg ggc atc aaa cga atg att caa att gaa tgg    432
   Arg Val Lys Pro Val Leu Gly Ile Lys Arg Met Ile Gln Ile Glu Trp
       130                 135                 140
   ata aag cct gag ttg gcg cct gtt tca tct gat tta aaa tac aca ctt    480
   Ile Lys Pro Glu Leu Ala Pro Val Ser Ser Asp Leu Lys Tyr Thr Leu
   145                 150                 155                 160
   cga ttc agg aca gtc aac agt acc agc tgg atg gaa gtc aac ttc gct    528
   Arg Phe Arg Thr Val Asn Ser Thr Ser Trp Met Glu Val Asn Phe Ala
                   165                 170                 175
   aag aac cgt aag gat aaa aac caa acg tac aac ctc acg ggg ctg cag    576
   Lys Asn Arg Lys Asp Lys Asn Gln Thr Tyr Asn Leu Thr Gly Leu Gln
               180                 185                 190
   cct ttt aca gaa tat gtc ata gct ctg cga tgt gcg gtc aag gag tca    624
   Pro Phe Thr Glu Tyr Val Ile Ala Leu Arg Cys Ala Val Lys Glu Ser
           195                 200                 205
   aag ttc tgg agt gac tgg agc caa gaa aaa atg gga atg act gag gaa    672
   Lys Phe Trp Ser Asp Trp Ser Gln Glu Lys Met Gly Met Thr Glu Glu
       210                 215                 220
   gaa gct cca tgt ggc ctg gaa ctg tgg aga gtc ctg aaa cca gct gag    720
   Glu Ala Pro Cys Gly Leu Glu Leu Trp Arg Val Leu Lys Pro Ala Glu
   225                 230                 235                 240
   gcg gat gga aga agg cca gtg cgg ttg tta tgg aag aag gca aga gga    768
   Ala Asp Gly Arg Arg Pro Val Arg Leu Leu Trp Lys Lys Ala Arg Gly
                   245                 250                 255
   gcc cca gtc cta gag aaa aca ctt ggc tac aac ata tgg tac tat cca    816
   Ala Pro Val Leu Glu Lys Thr Leu Gly Tyr Asn Ile Trp Tyr Tyr Pro
               260                 265                 270
   gaa agc aac act aac ctc aca gaa aca atg aac act act aac cag cag    864
   Glu Ser Asn Thr Asn Leu Thr Glu Thr Met Asn Thr Thr Asn Gln Gln
           275                 280                 285
   ctt gaa ctg cat ctg gga ggc gag agc ttt tgg gtg tct atg att tct    912
   Leu Glu Leu His Leu Gly Gly Glu Ser Phe Trp Val Ser Met Ile Ser
       290                 295                 300
   tat aat tct ctt ggg aag tct cca gtg gcc acc ctg agg att cca gct    960
   Tyr Asn Ser Leu Gly Lys Ser Pro Val Ala Thr Leu Arg Ile Pro Ala
   305                 310                 315                 320
   att caa gaa aaa tca ttt cag tgc att gag gtc atg cag gcc tgc gtt    1008
   Ile Gln Glu Lys Ser Phe Gln Cys Ile Glu Val Met Gln Ala Cys Val
                   325                 330                 335
   gct gag gac cag cta gtg gtg aag tgg caa agc tct gct cta gac gtg    1056
   Ala Glu Asp Gln Leu Val Val Lys Trp Gln Ser Ser Ala Leu Asp Val
               340                 345                 350
   aac act tgg atg att gaa tgg ttt ccg gat gtg gac tca gag ccc acc    1104
   Asn Thr Trp Met Ile Glu Trp Phe Pro Asp Val Asp Ser Glu Pro Thr
           355                 360                 365
   acc ctt tcc tgg gaa tct gtg tct cag gcc acg aac tgg acg atc cag    1152
   Thr Leu Ser Trp Glu Ser Val Ser Gln Ala Thr Asn Trp Thr Ile Gln
       370                 375                 380
   caa gat aaa tta aaa cct ttc tgg tgc tat aac atc tct gtg tat cca    1200
   Gln Asp Lys Leu Lys Pro Phe Trp Cys Tyr Asn Ile Ser Val Tyr Pro
   385                 390                 395                 400
   atg ttg cat gac aaa gtt ggc gag cca tat tcc atc cag gct tat gcc    1248
   Met Leu His Asp Lys Val Gly Glu Pro Tyr Ser Ile Gln Ala Tyr Ala
                   405                 410                 415
   aaa gaa ggc gtt cca tca gaa ggt cct gag acc aag gtg gag aac att    1296
   Lys Glu Gly Val Pro Ser Glu Gly Pro Glu Thr Lys Val Glu Asn Ile
               420                 425                 430
   ggc gtg aag acg gtc acg atc aca tgg aaa gag att ccc aag agt gag    1344
   Gly Val Lys Thr Val Thr Ile Thr Trp Lys Glu Ile Pro Lys Ser Glu
           435                 440                 445
   aga aag ggt atc atc tgc aac tac acc atc ttt tac caa gct gaa ggt    1392
   Arg Lys Gly Ile Ile Cys Asn Tyr Thr Ile Phe Tyr Gln Ala Glu Gly
       450                 455                 460
   gga aaa gga ttc tcc aag aca gtc aat tcc agc atc ttg cag tac ggc    1440
   Gly Lys Gly Phe Ser Lys Thr Val Asn Ser Ser Ile Leu Gln Tyr Gly
   465                 470                 475                 480
   ctg gag tcc ctg aaa cga aag acc tct tac att gtt cag gtc atg gcc    1488
   Leu Glu Ser Leu Lys Arg Lys Thr Ser Tyr Ile Val Gln Val Met Ala
                   485                 490                 495
   agc acc agt gct ggg gga acc aac ggg acc agc ata aat ttc aag aca    1536
   Ser Thr Ser Ala Gly Gly Thr Asn Gly Thr Ser Ile Asn Phe Lys Thr
               500                 505                 510
   ttg tca ttc agt gtc ttt gag                                        1557
   Leu Ser Phe Ser Val Phe Glu
           515
<210> 8
   <211> 519
   <212> PRT
   <213> Homo sapiens
<400> 8
   Met Met Trp Thr Trp Ala Leu Trp Met Leu Pro Ser Leu Cys Lys Phe
    1               5                  10                  15
   Ser Leu Ala Ala Leu Pro Ala Lys Pro Glu Asn Ile Ser Cys Val Tyr
               20                  25                  30
   Tyr Tyr Arg Lys Asn Leu Thr Cys Thr Trp Ser Pro Gly Lys Glu Thr
           35                  40                  45
   Ser Tyr Thr Gln Tyr Thr Val Lys Arg Thr Tyr Ala Phe Gly Glu Lys
       50                  55                  60
   His Asp Asn Cys Thr Thr Asn Ser Ser Thr Ser Glu Asn Arg Ala Ser
   65                  70                  75                  80
   Cys Ser Phe Phe Leu Pro Arg Ile Thr Ile Pro Asp Asn Tyr Thr Ile
                   85                  90                  95
   Glu Val Glu Ala Glu Asn Gly Asp Gly Val Ile Lys Ser His Met Thr
               100                 105                 110
   Tyr Trp Arg Leu Glu Asn Ile Ala Lys Thr Glu Pro Pro Lys Ile Phe
           115                 120                 125
   Arg Val Lys Pro Val Leu Gly Ile Lys Arg Met Ile Gln Ile Glu Trp
       130                 135                 140
   Ile Lys Pro Glu Leu Ala Pro Val Ser Ser Asp Leu Lys Tyr Thr Leu
   145                 150                 155                 160
   Arg Phe Arg Thr Val Asn Ser Thr Ser Trp Met Glu Val Asn Phe Ala
                   165                 170                 175
   Lys Asn Arg Lys Asp Lys Asn Gln Thr Tyr Asn Leu Thr Gly Leu Gln
               180                 185                 190
   Pro Phe Thr Glu Tyr Val Ile Ala Leu Arg Cys Ala Val Lys Glu Ser
           195                 200                 205
   Lys Phe Trp Ser Asp Trp Ser Gln Glu Lys Met Gly Met Thr Glu Glu
       210                 215                 220
   Glu Ala Pro Cys Gly Leu Glu Leu Trp Arg Val Leu Lys Pro Ala Glu
   225                 230                 235                 240
   Ala Asp Gly Arg Arg Pro Val Arg Leu Leu Trp Lys Lys Ala Arg Gly
                   245                 250                 255
   Ala Pro Val Leu Glu Lys Thr Leu Gly Tyr Asn Ile Trp Tyr Tyr Pro
               260                 265                 270
   Glu Ser Asn Thr Asn Leu Thr Glu Thr Met Asn Thr Thr Asn Gln Gln
           275                 280                 285
   Leu Glu Leu His Leu Gly Gly Glu Ser Phe Trp Val Ser Met Ile Ser
       290                 295                 300
   Tyr Asn Ser Leu Gly Lys Ser Pro Val Ala Thr Leu Arg Ile Pro Ala
   305                 310                 315                 320
   Ile Gln Glu Lys Ser Phe Gln Cys Ile Glu Val Met Gln Ala Cys Val
                   325                 330                 335
   Ala Glu Asp Gln Leu Val Val Lys Trp Gln Ser Ser Ala Leu Asp Val
               340                 345                 350
   Asn Thr Trp Met Ile Glu Trp Phe Pro Asp Val Asp Ser Glu Pro Thr
           355                 360                 365
   Thr Leu Ser Trp Glu Ser Val Ser Gln Ala Thr Asn Trp Thr Ile Gln
       370                 375                 380
   Gln Asp Lys Leu Lys Pro Phe Trp Cys Tyr Asn Ile Ser Val Tyr Pro
   385                 390                 395                 400
   Met Leu His Asp Lys Val Gly Glu Pro Tyr Ser Ile Gln Ala Tyr Ala
                   405                 410                 415
   Lys Glu Gly Val Pro Ser Glu Gly Pro Glu Thr Lys Val Glu Asn Ile
               420                 425                 430
   Gly Val Lys Thr Val Thr Ile Thr Trp Lys Glu Ile Pro Lys Ser Glu
           435                 440                 445
   Arg Lys Gly Ile Ile Cys Asn Tyr Thr Ile Phe Tyr Gln Ala Glu Gly
       450                 455                 460
   Gly Lys Gly Phe Ser Lys Thr Val Asn Ser Ser Ile Leu Gln Tyr Gly
   465                 470                 475                 480
   Leu Glu Ser Leu Lys Arg Lys Thr Ser Tyr Ile Val Gln Val Met Ala
                   485                 490                 495
   Ser Thr Ser Ala Gly Gly Thr Asn Gly Thr Ser Ile Asn Phe Lys Thr
               500                 505                 510
   Leu Ser Phe Ser Val Phe Glu
           515
<210> 9
   <211> 2748
   <212> DNA
   <213> Mus musculus
<220>
   <221> CDS
   <222> (237)...(2222)
<400> 9
   gatggggccc tgaatgttga tctgacagaa ttccagacca acctggtggt tattgtcctt 60
   ttcatctggt catgctgaat atactctcaa gatgtgctgg agaaggtgct gctgtccggg 120
   ctctcagaga aggcagtgct ggaggcgttc ctggcccggg tctcctccta ctgttcctgg 180
   tagcccagcc ttctcggggt ggaaggagaa gctggccagg tgagctctga ggaagc atg 239
                                                                 Met
                                                                  1
   ctg agc agc cag aag gga tcc tgc agc cag gaa cca ggg gca gcc cac    287
   Leu Ser Ser Gln Lys Gly Ser Cys Ser Gln Glu Pro Gly Ala Ala His
                   5                  10                  15
   gtc cag cct ctg ggt gtg aac gct gga ata atg tgg acc ttg gca ctg    335
   Val Gln Pro Leu Gly Val Asn Ala Gly Ile Met Trp Thr Leu Ala Leu
               20                  25                  30
   tgg gca ttc tct ttc ctc tgc aaa ttc agc ctg gca gtc ctg ccg act    383
   Trp Ala Phe Ser Phe Leu Cys Lys Phe Ser Leu Ala Val Leu Pro Thr
           35                  40                  45
   aag cca gag aac att tcc tgc gtc ttt tac ttc gac aga aat ctg act    431
   Lys Pro Glu Asn Ile Ser Cys Val Phe Tyr Phe Asp Arg Asn Leu Thr
       50                  55                  60                  65
   tgc act tgg aga cca gag aag gaa acc aat gat acc agc tac att gtg    479
   Cys Thr Trp Arg Pro Glu Lys Glu Thr Asn Asp Thr Ser Tyr Ile Val
                       70                  75                  80
   act ttg act tac tcc tat gga aaa agc aat tat agt gac aat gct aca    527
   Thr Leu Thr Tyr Ser Tyr Gly Lys Ser Asn Tyr Ser Asp Asn Ala Thr
                   85                  90                  95
   gag gct tca tat tct ttt ccc cgt tcc tgt gca atg ccc cca gac atc    575
   Glu Ala Ser Tyr Ser Phe Pro Arg Ser Cys Ala Met Pro Pro Asp Ile
               100                 105                 110
   tgc agt gtt gaa gta caa gct caa aat gga gat ggt aaa gtt aaa tct    623
   Cys Ser Val Glu Val Gln Ala Gln Asn Gly Asp Gly Lys Val Lys Ser
           115                 120                 125
   gac atc aca tat tgg cat tta atc tcc ata gca aaa acc gaa cca cct    671
   Asp Ile Thr Tyr Trp His Leu Ile Ser Ile Ala Lys Thr Glu Pro Pro
       130                 135                 140                 145
   ata att tta agt gtg aat cca att tgt aat aga atg ttc cag ata caa    719
   Ile Ile Leu Ser Val Asn Pro Ile Cys Asn Arg Met Phe Gln Ile Gln
                       150                 155                 160
   tgg aaa ccg cgt gaa aag act cgt ggg ttt cct tta gta tgc atg ctt    767
   Trp Lys Pro Arg Glu Lys Thr Arg Gly Phe Pro Leu Val Cys Met Leu
                   165                 170                 175
   cgg ttc aga act gtc aac agt agc cgc tgg acg gaa gtc aat ttt gaa    815
   Arg Phe Arg Thr Val Asn Ser Ser Arg Trp Thr Glu Val Asn Phe Glu
               180                 185                 190
   aac tgt aaa cag gtc tgc aac ctc aca gga ctt cag gct ttc aca gaa    863
   Asn Cys Lys Gln Val Cys Asn Leu Thr Gly Leu Gln Ala Phe Thr Glu
           195                 200                 205
   tat gtc ctg gct cta cga ttc agg ttc aat gac tca aga tat tgg agc    911
   Tyr Val Leu Ala Leu Arg Phe Arg Phe Asn Asp Ser Arg Tyr Trp Ser
       210                 215                 220                 225
   aag tgg agc aaa gaa gaa acc aga gtg act atg gag gaa gtt cca cat    959
   Lys Trp Ser Lys Glu Glu Thr Arg Val Thr Met Glu Glu Val Pro His
                       230                 235                 240
   gtc ctg gac ctg tgg aga att ctg gaa cca gca gac atg aac gga gac    1007
   Val Leu Asp Leu Trp Arg Ile Leu Glu Pro Ala Asp Met Asn Gly Asp
                   245                 250                 255
   agg aag gtg cga ttg ctg tgg aag aag gca aga gga gcc ccc gtc ttg    1055
   Arg Lys Val Arg Leu Leu Trp Lys Lys Ala Arg Gly Ala Pro Val Leu
           260                 265                 270
   gag aaa aca ttt ggc tac cac ata cag tac ttt gca gag aac agc act    1103
   Glu Lys Thr Phe Gly Tyr His Ile Gln Tyr Phe Ala Glu Asn Ser Thr
       275                 280                 285
   aac ctc aca gag ata aac aac atc acc acc cag cag tat gaa ctg ctt    1151
   Asn Leu Thr Glu Ile Asn Asn Ile Thr Thr Gln Gln Tyr Glu Leu Leu
   290                 295                 300                 305
   ctg atg agc cag gca cac tct gtg tcc gtg act tct ttt aat tct ctt    1199
   Leu Met Ser Gln Ala His Ser Val Ser Val Thr Ser Phe Asn Ser Leu
                   310                 315                 320
   ggc aag tcc caa gag acc atc ctg agg atc cca gat gtc cat gag aag    1247
   Gly Lys Ser Gln Glu Thr Ile Leu Arg Ile Pro Asp Val His Glu Lys
               325                 330                 335
   acc ttc cag tac att aag agc atg cag gcc tac ata gcc gag ccc ctg    1295
   Thr Phe Gln Tyr Ile Lys Ser Met Gln Ala Tyr Ile Ala Glu Pro Leu
           340                 345                 350
   ttg gtg gtg aac tgg caa agc tcc att cct gcg gtg gac act tgg ata    1343
   Leu Val Val Asn Trp Gln Ser Ser Ile Pro Ala Val Asp Thr Trp Ile
       355                 360                 365
   gtg gag tgg ctc cca gaa gct gcc atg tcg aag ttc cct gcc ctt tcc    1391
   Val Glu Trp Leu Pro Glu Ala Ala Met Ser Lys Phe Pro Ala Leu Ser
   370                 375                 380                 385
   tgg gaa tct gtg tct cag gtc acg aac tgg acc atc gag caa gat aaa    1439
   Trp Glu Ser Val Ser Gln Val Thr Asn Trp Thr Ile Glu Gln Asp Lys
                   390                 395                 400
   cta aaa cct ttc aca tgc tat aat ata tca gtg tat cca gtg ttg gga    1487
   Leu Lys Pro Phe Thr Cys Tyr Asn Ile Ser Val Tyr Pro Val Leu Gly
               405                 410                 415
   cac cga gtt gga gag ccg tat tca atc caa gct tat gcc aaa gaa gga    1535
   His Arg Val Gly Glu Pro Tyr Ser Ile Gln Ala Tyr Ala Lys Glu Gly
           420                 425                 430
   act cca tta aaa ggt cct gag acc agg gtg gag aac atc ggt ctg agg    1583
   Thr Pro Leu Lys Gly Pro Glu Thr Arg Val Glu Asn Ile Gly Leu Arg
       435                 440                 445
   aca gcc acg atc aca tgg aag gag att cct aag agt gct agg aat gga    1631
   Thr Ala Thr Ile Thr Trp Lys Glu Ile Pro Lys Ser Ala Arg Asn Gly
   450                 455                 460                 465
   ttt atc aac aat tac act gta ttt tac caa gct gaa ggt gga aaa gaa    1679
   Phe Ile Asn Asn Tyr Thr Val Phe Tyr Gln Ala Glu Gly Gly Lys Glu
                   470                 475                 480
   ctc tcc aag act gtt aac tct cat gcc ctg cag tgt gac ctg gag tct    1727
   Leu Ser Lys Thr Val Asn Ser His Ala Leu Gln Cys Asp Leu Glu Ser
               485                 490                 495
   ctg aca cga agg acc tct tat act gtt tgg gtc atg gcc agc acc aga    1775
   Leu Thr Arg Arg Thr Ser Tyr Thr Val Trp Val Met Ala Ser Thr Arg
           500                 505                 510
   gct gga ggt acc aac ggg gtg aga ata aac ttc aag aca ttg tca atc    1823
   Ala Gly Gly Thr Asn Gly Val Arg Ile Asn Phe Lys Thr Leu Ser Ile
       515                 520                 525
   agt gtg ttt gaa att gtc ctt cta aca tct cta gtt gga gga ggc ctt    1871
   Ser Val Phe Glu Ile Val Leu Leu Thr Ser Leu Val Gly Gly Gly Leu
   530                 535                 540                 545
   ctt cta ctt agc atc aaa aca gtg act ttt ggc ctc aga aag cca aac    1919
   Leu Leu Leu Ser Ile Lys Thr Val Thr Phe Gly Leu Arg Lys Pro Asn
                   550                 555                 560
   cgg ttg act ccc ctg tgt tgt cct gat gtt ccc aac cct gct gaa agt    1967
   Arg Leu Thr Pro Leu Cys Cys Pro Asp Val Pro Asn Pro Ala Glu Ser
               565                 570                 575
   agt tta gcc aca tgg ctc gga gat ggt ttc aag aag tca aat atg aag    2015
   Ser Leu Ala Thr Trp Leu Gly Asp Gly Phe Lys Lys Ser Asn Met Lys
           580                 585                 590
   gag act gga aac tct ggg aac aca gaa gac gtg gtc cta aaa cca tgt    2063
   Glu Thr Gly Asn Ser Gly Asn Thr Glu Asp Val Val Leu Lys Pro Cys
       595                 600                 605
   ccc gtc ccc gcg gat ctc att gac aag ctg gta gtg aac ttt gag aat    2111
   Pro Val Pro Ala Asp Leu Ile Asp Lys Leu Val Val Asn Phe Glu Asn
   610                 615                 620                 625
   ttt ctg gaa gta gtt ttg aca gag gaa gct gga aag ggt cag gcg agc    2159
   Phe Leu Glu Val Val Leu Thr Glu Glu Ala Gly Lys Gly Gln Ala Ser
                   630                 635                 640
   att ttg gga gga gaa gcg aat gag tat atc tta tcc cag gaa cca agc    2207
   Ile Leu Gly Gly Glu Ala Asn Glu Tyr Ile Leu Ser Gln Glu Pro Ser
               645                 650                 655
   tgt cct ggc cat tgc tgaagctacc ctcagggtcc aggacagctg tcttgttggc    2262
   Cys Pro Gly His Cys
           660
   acttgactct ggcaggaacc tgatctctac ttttcttctc cctgtctccg gacactttct 2322
   ctccttcatg cagagaccag gactagagcg gattcctcat ggtttgccag gctcctcagt 2382
   ccttgctcgg gctcaggatc ttcaacaatg ccctttctgg gacactccat catccactta 2442
   tatttatttt ttgcaacatt gtggattgaa cccagggact tgtttatgcg cgcaacttca 2502
   gtaactgtgg cagagactta ggaatggaga tctgaccctt tgcagaaggt ttctggacat 2562
   ccgtccctgt gtgagcctca gacagcattg tctttacttt gaatcagctt ccaagttaat 2622
   aaaagaaaaa cagagaggtg gcataacagc tcctgcttcc tgacctgctt gagttccagt 2682
   tctgacttcc tttggtgatg aacagcaatg tgggaagtgt aagctgaata aaccctttcc 2742
   tcccca                                                            2748
<210> 10
   <211> 662
   <212> PRT
   <213> Mus musculus
<400> 10
   Met Leu Ser Ser Gln Lys Gly Ser Cys Ser Gln Glu Pro Gly Ala Ala
   1               5                   10                  15
   His Val Gln Pro Leu Gly Val Asn Ala Gly Ile Met Trp Thr Leu Ala
               20                  25                  30
   Leu Trp Ala Phe Ser Phe Leu Cys Lys Phe Ser Leu Ala Val Leu Pro
           35                  40                  45
   Thr Lys Pro Glu Asn Ile Ser Cys Val Phe Tyr Phe Asp Arg Asn Leu
       50                  55                  60
   Thr Cys Thr Trp Arg Pro Glu Lys Glu Thr Asn Asp Thr Ser Tyr Ile
   65 70 75 80
   Val Thr Leu Thr Tyr Ser Tyr Gly Lys Ser Asn Tyr Ser Asp Asn Ala
   85 90 95
   Thr Glu Ala Ser Tyr Ser Phe Pro Arg Ser Cys Ala Met Pro Pro Asp
   100 105 110
   Ile Cys Ser Val Glu Val Gln Ala Gln Asn Gly Asp Gly Lys Val Lys
   115 120 125
   Ser Asp Ile Thr Tyr Trp His Leu Ile Ser Ile Ala Lys Thr Glu Pro
   130 135 140
   Pro Ile Ile Leu Ser Val Asn Pro Ile Cys Asn Arg Met Phe Gln Ile
   145 150 155 160
   Gln Trp Lys Pro Arg Glu Lys Thr Arg Gly Phe Pro Leu Val Cys Met
   165 170 175
   Leu Arg Phe Arg Thr Val Asn Ser Ser Arg Trp Thr Glu Val Asn Phe
   180 185 190
   Glu Asn Cys Lys Gln Val Cys Asn Leu Thr Gly Leu Gln Ala Phe Thr
   195 200 205
   Glu Tyr Val Leu Ala Leu Arg Phe Arg Phe Asn Asp Ser Arg Tyr Trp
   210 215 220
   Ser Lys Trp Ser Lys Glu Glu Thr Arg Val Thr Met Glu Glu Val Pro
   225 230 235 240
   His Val Leu Asp Leu Trp Arg Ile Leu Glu Pro Ala Asp Met Asn Gly
   245 250 255
   Asp Arg Lys Val Arg Leu Leu Trp Lys Lys Ala Arg Gly Ala Pro Val
   260 265 270
   Leu Glu Lys Thr Phe Gly Tyr His Ile Gln Tyr Phe Ala Glu Asn Ser
   275 280 285
   Thr Asn Leu Thr Glu Ile Asn Asn Ile Thr Thr Gln Gln Tyr Glu Leu
   290 295 300
   Leu Leu Met Ser Gln Ala His Ser Val Ser Val Thr Ser Phe Asn Ser
   305 310 315 320
   Leu Gly Lys Ser Gln Glu Thr Ile Leu Arg Ile Pro Asp Val His Glu
   325 330 335
   Lys Thr Phe Gln Tyr Ile Lys Ser Met Gln Ala Tyr Ile Ala Glu Pro
   340 345 350
   Leu Leu Val Val Asn Trp Gln Ser Ser Ile Pro Ala Val Asp Thr Trp
   355 360 365
   Ile Val Glu Trp Leu Pro Glu Ala Ala Met Ser Lys Phe Pro Ala Leu
   370 375 380
   Ser Trp Glu Ser Val Ser Gln Val Thr Asn Trp Thr Ile Glu Gln Asp
   385 390 395 400
   Lys Leu Lys Pro Phe Thr Cys Tyr Asn Ile Ser Val Tyr Pro Val Leu
   405 410 415
   Gly His Arg Val Gly Glu Pro Tyr Ser Ile Gln Ala Tyr Ala Lys Glu
   420 425 430
   Gly Thr Pro Leu Lys Gly Pro Glu Thr Arg Val Glu Asn Ile Gly Leu
   435 440 445
   Arg Thr Ala Thr Ile Thr Trp Lys Glu Ile Pro Lys Ser Ala Arg Asn
   450 455 460
   Gly Phe Ile Asn Asn Tyr Thr Val Phe Tyr Gln Ala Glu Gly Gly Lys
   465 470 475 480
   Glu Leu Ser Lys Thr Val Asn Ser His Ala Leu Gln Cys Asp Leu Glu
   485 490 495
   Ser Leu Thr Arg Arg Thr Ser Tyr Thr Val Trp Val Met Ala Ser Thr
   500 505 510
   Arg Ala Gly Gly Thr Asn Gly Val Arg Ile Asn Phe Lys Thr Leu Ser
   515 520 525
   Ile Ser Val Phe Glu Ile Val Leu Leu Thr Ser Leu Val Gly Gly Gly
   530 535 540
   Leu Leu Leu Leu Ser Ile Lys Thr Val Thr Phe Gly Leu Arg Lys Pro
   545 550 555 560
   Asn Arg Leu Thr Pro Leu Cys Cys Pro Asp Val Pro Asn Pro Ala Glu
   565 570 575
   Ser Ser Leu Ala Thr Trp Leu Gly Asp Gly Phe Lys Lys Ser Asn Met
               580                 585                 590
   Lys Glu Thr Gly Asn Ser Gly Asn Thr Glu Asp Val Val Leu Lys Pro
           595                 600                 605
   Cys Pro Val Pro Ala Asp Leu Ile Asp Lys Leu Val Val Asn Phe Glu
       610                 615                 620
   Asn Phe Leu Glu Val Val Leu Thr Glu Glu Ala Gly Lys Gly Gln Ala
   625                 630                 635                 640
   Ser Ile Leu Gly Gly Glu Ala Asn Glu Tyr Ile Leu Ser Gln Glu Pro
                   645                 650                 655
   Ser Cys Pro Gly His Cys
               660
<210> 11
   <211> 2964
   <212> DNA
   <213> Homo sapiens
<220>
   <221> CDS
   <222> (13)...(2949)
<400> 11
gaattcgcca cc atg gct cta ttt gca gtc ttt cag aca aca ttc ttc tta 51
   aca ttg ctg tcc ttg agg act tac cag agt gaa gtc ttg gct gaa cgt   99
   tta cca ttg act cct gta tca ctt aaa gtt tcc acc aat tct acg cgt   147
   cag agt ttg cac tta caa tgg act gtc cac aac ctt cct tat cat cag   195
   gaa ttg aaa atg gta ttt cag atc cag atc agt agg att gaa aca tcc   243
   aat gtc atc tgg gtg ggg aat tac agc acc act gtg aag tgg aac cag   291
   gtt ctg cat tgg agc tgg gaa tct gag ctc cct ttg gaa tgt gcc aca   339
   cac ttt gta aga ata aag agt ttg gtg gac gat gcc aag ttc cct gag   387
   cca aat ttc tgg agc aac tgg agt tcc tgg gag gaa gtc agt gta caa   435
   gat tct act gga cag gat ata ttg ttc gtt ttc cct aaa gat aag ctg   483
   gtg gaa gaa ggc acc aat gtt acc att tgt tac gtt tct agg aac att     531
   caa aat aat gta tcc tgt tat ttg gaa ggg aaa cag att cat gga gaa     579
   caa ctt gat cca cat gta act gca ttc aac ttg aat agt gtg cct ttc     627
   att agg aat aaa ggg aca aat atc tat tgt gag gca agt caa gga aat     675
   gtc agt gaa ggc atg aaa ggc atc gtt ctt ttt gtc tca aaa gta ctt     723
   gag gag ccc aag gac ttt tct tgt gaa acc gag gac ttc aag act ttg     771
   cac tgt act tgg gat cct ggg acg gac act gcc ttg ggg tgg tct aaa     819
   caa cct tcc caa agc tac act tta ttt gaa tca ttt tct ggg gaa aag     867
   aaa ctt tgt aca cac aaa aac tgg tgt aat tgg caa ata act caa gac     915
   tca caa gaa acc tat aac ttc aca ctc ata gct gaa aat tac tta agg     963
   aag aga agt gtc aat atc ctt ttt aac ctg act cat cga gtt tat tta     1011
   atg aat cct ttt agt gtc aac ttt gaa aat gta aat gcc aca aat gcc     1059
   atc atg acc tgg aag gtg cac tcc ata agg aat aat ttc aca tat ttg     1107
   tgt cag att gaa ctc cat ggt gaa gga aaa atg atg caa tac aat gtt     1155
   tcc atc aag gtg aac ggt gag tac ttc tta agt gaa ctg gaa cct gcc     1203
   aca gag tac atg gcg cga gta cgg tgt gct gat gcc agc cac ttc tgg     1251
   aaa tgg agt gaa tgg agt ggt cag aac ttc acc aca ctt gaa gct gct    1299
   ccc tca gag gcc cct gat gtc tgg aga att gtg agc ttg gag cca gga    1347
   aat cat act gtg acc tta ttc tgg aag cca tta tca aaa ctg cat gcc    1395
   aat gga aag atc ctg ttc tat aat gta gtt gta gaa aac cta gac aaa    1443
   cca tcc agt tca gag ctc cat tcc att cca gca cca gcc aac agc aca    1491
   aaa cta atc ctt gac agg tgt tcc tac caa atc tgc gtc ata gcc aac    1539
   aac agt gtg ggt gct tct cct gct tct gta ata gtc atc tct gca gac    1587
   ccc gaa aac aaa gag gtt gag gaa gaa aga att gca ggc aca gag ggt    1635
   gga ttc tct ctg tct tgg aaa ccc caa cct gga gat gtt ata ggc tat    1683
   gtt gtg gac tgg tgt gac cat acc cag gat gtg ctc ggt gat ttc cag    1731
   tgg aag aat gta ggt ccc aat acc aca agc aca gtc att agc aca gat    1779
   gct ttt agg cca gga gtt cga tat gac ttc aga att tat ggg tta tct    1827
   aca aaa agg att gct tgt tta tta gag aaa aaa aca gga tac tct cag    1875
   gaa ctt gct cct tca gac aac cct cac gtg ctg gtg gat aca ttg aca    1923
   tcc cac tcc ttc act ctg agt tgg aaa gat tac tct act gaa tct caa    1971
   cct ggt ttt ata caa ggg tac cat gtc tat ctg aaa tcc aag gcg agg    2019
   cag tgc cac cca cga ttt gaa aag gca gtt ctt tca gat ggt tca gaa   2067
   tgt tgc aaa tac aaa att gac aac ccg gaa gaa aag gca ttg att gtg   2115
   gac aac cta aag cca gaa tcc ttc tat gag ttt ttc atc act cca ttc   2163
   act agt gct ggt gaa ggc ccc agt gct acg ttc acg aag gtc acg act   2211
   ccg gat gaa cac tcc tcg atg ctg att cat atc cta ctg ccc atg gtt   2259
   ttc tgc gtc ttg ctc atc atg gtc atg tgc tac ttg aaa agt cag tgg   2307
   atc aag gag acc tgt tat cct gac atc cct gac cct tac aag agc agc   2355
   atc ctg tca tta ata aaa ttc aag gag aac cct cac cta ata ata atg   2403
   aat gtc agt gac tgt atc cca gat gct att gaa gtt gta agc aag cca   2451
   gaa ggg aca aag ata cag ttc cta ggc act agg aag tca ctc aca gaa   2499
   acc gag ttg act aag cct aac tac ctt tat ctc ctt cca aca gaa aag   2547
   aat cac tct ggc cct ggc ccc tgc atc tgt ttt gag aac ttg acc tat   2595
   aac cag gca gct tct gac tct ggc tct tgt ggc cat gtt cca gta tcc   2643
   cca aaa gcc cca agt atg ctg gga cta atg acc tca cct gaa aat gta   2691
   cta aag gca cta gaa aaa aac tac atg aac tcc ctg gga gaa atc cca   2739
   gct gga gaa aca agt ttg aat tat gtg tcc cag ttg gct tca ccc atg   2787
   ttt gga gac aag gac agt ctc cca aca aac cca gta gag gca cca cac     2835
   tgt tca gag tat aaa atg caa atg gca gtc tcc ctg cgt ctt gcc ttg     2883
   cct ccc ccg acc gag aat agc agc ctc tcc tca att acc ctt tta gat     2931
   cca ggt gaa cac tac tgc taaccagcac tcgag                            2964
<210> 12
   <211> 979
   <212> PRT
   <213> Homo sapiens
<400> 12
<210> 13
   <211> 138
   <212> PRT
   <213> Homo sapiens
<400> 13
   Leu Pro Val Arg Leu Leu Arg Pro Ser Asp Asp Val Gln Lys Ile Val
    1               5                  10                  15
   Glu Glu Leu Gln Ser Leu Ser Lys Met Leu Leu Lys Asp Val Glu Glu
               20                  25                  30
   Glu Lys Gly Val Leu Val Ser Gln Asn Tyr Thr Leu Pro Cys Leu Ser
           35                  40                  45
   Pro Asp Ala Gln Pro Pro Asn Asn Ile His Ser Pro Ala Ile Arg Ala
       50                  55                  60
   Tyr Leu Lys Thr Ile Arg Gln Leu Asp Asn Lys Ser Val Ile Asp Glu
   65                  70                  75                  80
   Ile Ile Glu His Leu Asp Lys Leu Ile Phe Gln Asp Ala Pro Glu Thr
                   85                  90                  95
   Asn Ile Ser Val Pro Thr Asp Thr His Glu Cys Lys Arg Phe Ile Leu
               100                 105                 110
   Thr Ile Ser Gln Gln Phe Ser Glu Cys Met Asp Leu Ala Leu Lys Ser
           115                 120                 125
   Leu Thr Ser Gly Ala Gln Gln Ala Thr Thr
       130                 135
<210> 14
   <211> 138
   <212> PRT
   <213> Homo sapiens
<400> 14
   Leu Pro Val Arg Leu Leu Arg Pro Ser Asp Asp Val Gln Lys Ile Val
    1               5                  10                  15
   Glu Glu Leu Gln Ser Leu Ser Lys Met Leu Leu Lys Asp Val Glu Glu
               20                  25                  30
   Glu Lys Gly Val Leu Val Ser Gln Asn Tyr Thr Leu Pro Ser Leu Ser
           35                  40                  45
   Pro Asp Ala Gln Pro Pro Asn Asn Ile His Ser Pro Ala Ile Arg Ala
       50                  55                  60
   Tyr Leu Lys Thr Ile Arg Gln Leu Asp Asn Lys Ser Val Ile Asp Glu
   65                  70                  75                  80
   Ile Ile Glu His Leu Asp Lys Leu Ile Phe Gln Asp Ala Pro Glu Thr
                   85                  90                  95
   Asn Ile Ser Val Pro Thr Asp Thr His Glu Cys Lys Arg Phe Ile Leu
               100                 105                 110
   Thr Ile Ser Gln Gln Phe Ser Glu Cys Met Asp Leu Ala Leu Lys Ser
           115                 120                 125
   Leu Thr Ser Gly Ala Gln Gln Ala Thr Thr
       130                 135
<210> 15
   <211> 138
   <212> PRT
   <213> Homo sapiens
<400> 15
   Leu Pro Val Arg Leu Leu Arg Pro Ser Asp Asp Val Gln Lys Ile Val
    1               5                  10                  15
   Glu Glu Leu Gln Ser Leu Ser Lys Met Leu Leu Lys Asp Val Glu Glu
               20                  25                  30
   Glu Lys Gly Val Leu Val Ser Gln Asn Tyr Thr Leu Pro Cys Leu Ser
           35                  40                  45
   Pro Asp Ala Gln Pro Pro Asn Asn Ile His Ser Pro Ala Ile Arg Ala
       50                  55                  60
   Tyr Leu Lys Thr Ile Arg Gln Leu Asp Asn Lys Ser Val Ile Asp Glu
   65                  70                  75                  80
   Ile Ile Glu His Leu Asp Lys Leu Ile Phe Gln Asp Ala Pro Glu Thr
                   85                  90                  95
   Asn Ile Ser Val Pro Thr Asp Thr His Glu Ser Lys Arg Phe Ile Leu
               100                 105                 110
   Thr Ile Ser Gln Gln Phe Ser Glu Cys Met Asp Leu Ala Leu Lys Ser
           115                 120                 125
   Leu Thr Ser Gly Ala Gln Gln Ala Thr Thr
       130                 135
<210> 16
   <211> 138
   <212> PRT
   <213> Homo sapiens
<400> 16
   Leu Pro Val Arg Leu Leu Arg Pro Ser Asp Asp Val Gln Lys Ile Val
    1               5                  10                  15
   Glu Glu Leu Gln Ser Leu Ser Lys Met Leu Leu Lys Asp Val Glu Glu
               20                  25                  30
   Glu Lys Gly Val Leu Val Ser Gln Asn Tyr Thr Leu Pro Cys Leu Ser
           35                  40                  45
   Pro Asp Ala Gln Pro Pro Asn Asn Ile His Ser Pro Ala Ile Arg Ala
       50                  55                  60
   Tyr Leu Lys Thr Ile Arg Gln Leu Asp Asn Lys Ser Val Ile Asp Glu
   65                  70                  75                  80
   Ile Ile Glu His Leu Asp Lys Leu Ile Phe Gln Asp Ala Pro Glu Thr
                   85                  90                  95
   Asn Ile Ser Val Pro Thr Asp Thr His Glu Cys Lys Arg Phe Ile Leu
               100                 105                 110
   Thr Ile Ser Gln Gln Phe Ser Glu Ser Met Asp Leu Ala Leu Lys Ser
           115                 120                 125
   Leu Thr Ser Gly Ala Gln Gln Ala Thr Thr
       130                 135
<210> 17
   <211> 139
   <212> PRT
   <213> Homo sapiens
<400> 17
   Met Leu Pro Val Arg Leu Leu Arg Pro Ser Asp Asp Val Gln Lys Ile
    1               5                  10                  15
   Val Glu Glu Leu Gln Ser Leu Ser Lys Met Leu Leu Lys Asp Val Glu
               20                  25                  30
   Glu Glu Lys Gly Val Leu Val Ser Gln Asn Tyr Thr Leu Pro Ser Leu
           35                  40                  45
   Ser Pro Asp Ala Gln Pro Pro Asn Asn Ile His Ser Pro Ala Ile Arg
       50                  55                  60
   Ala Tyr Leu Lys Thr Ile Arg Gln Leu Asp Asn Lys Ser Val Ile Asp
   65                  70                  75                  80
   Glu Ile Ile Glu His Leu Asp Lys Leu Ile Phe Gln Asp Ala Pro Glu
                   85                  90                  95
   Thr Asn Ile Ser Val Pro Thr Asp Thr His Glu Cys Lys Arg Phe Ile
               100                 105                 110
   Leu Thr Ile Ser Gln Gln Phe Ser Glu Cys Met Asp Leu Ala Leu Lys
           115                 120                 125
   Ser Leu Thr Ser Gly Ala Gln Gln Ala Thr Thr
       130                 135
<210> 18
   <211> 139
   <212> PRT
   <213> Homo sapiens
<400> 18
   Met Leu Pro Val Arg Leu Leu Arg Pro Ser Asp Asp Val Gln Lys Ile
    1               5                  10                  15
   Val Glu Glu Leu Gln Ser Leu Ser Lys Met Leu Leu Lys Asp Val Glu
               20                  25                  30
   Glu Glu Lys Gly Val Leu Val Ser Gln Asn Tyr Thr Leu Pro Cys Leu
           35                  40                  45
   Ser Pro Asp Ala Gln Pro Pro Asn Asn Ile His Ser Pro Ala Ile Arg
       50                  55                  60
   Ala Tyr Leu Lys Thr Ile Arg Gln Leu Asp Asn Lys Ser Val Ile Asp
   65                  70                  75                  80
   Glu Ile Ile Glu His Leu Asp Lys Leu Ile Phe Gln Asp Ala Pro Glu
                   85                  90                  95
   Thr Asn Ile Ser Val Pro Thr Asp Thr His Glu Ser Lys Arg Phe Ile
               100                 105                 110
   Leu Thr Ile Ser Gln Gln Phe Ser Glu Cys Met Asp Leu Ala Leu Lys
           115                 120                 125
   Ser Leu Thr Ser Gly Ala Gln Gln Ala Thr Thr
       130                 135
<210> 19
   <211> 139
   <212> PRT
   <213> Homo sapiens
<400> 19
   Met Leu Pro Val Arg Leu Leu Arg Pro Ser Asp Asp Val Gln Lys Ile
    1               5                  10                  15
   Val Glu Glu Leu Gln Ser Leu Ser Lys Met Leu Leu Lys Asp Val Glu
               20                  25                  30
   Glu Glu Lys Gly Val Leu Val Ser Gln Asn Tyr Thr Leu Pro Cys Leu
           35                  40                  45
   Ser Pro Asp Ala Gln Pro Pro Asn Asn Ile His Ser Pro Ala Ile Arg
       50                  55                  60
   Ala Tyr Leu Lys Thr Ile Arg Gln Leu Asp Asn Lys Ser Val Ile Asp
   65                  70                  75                  80
   Glu Ile Ile Glu His Leu Asp Lys Leu Ile Phe Gln Asp Ala Pro Glu
                   85                  90                  95
   Thr Asn Ile Ser Val Pro Thr Asp Thr His Glu Cys Lys Arg Phe Ile
               100                 105                 110
   Leu Thr Ile Ser Gln Gln Phe Ser Glu Ser Met Asp Leu Ala Leu Lys
           115                 120                 125
   Ser Leu Thr Ser Gly Ala Gln Gln Ala Thr Thr
       130                 135
<210> 20
   <211> 133
   <212> PRT
   <213> Mus musculus
<400> 20
   Ala Pro Ile Ser Lys Glu Asp Leu Arg Thr Thr Ile Asp Leu Leu Lys
    1              5                   10                  15
   Gln Glu Ser Gln Asp Leu Tyr Asn Asn Tyr Ser Ile Lys Gln Ala Ser
               20                  25                  30
   Gly Met Ser Ala Asp Glu Ser Ile Gln Leu Pro Cys Phe Ser Leu Asp
           35                  40                  45
   Arg Glu Ala Leu Thr Asn Ile Ser Val Ile Ile Ala His Leu Glu Lys
       50                  55                  60
   Val Lys Val Leu Ser Glu Asn Thr Val Asp Thr Ser Trp Val Ile Arg
   65                  70                  75                  80
   Trp Leu Thr Asn Ile Ser Cys Phe Asn Pro Leu Asn Leu Asn Ile Ser
                   85                  90                  95
   Val Pro Gly Asn Thr Asp Glu Ser Tyr Asp Cys Lys Val Phe Val Leu
               100                 105                 110
   Thr Val Leu Lys Gln Phe Ser Asn Cys Met Ala Glu Leu Gln Ala Lys
           115                 120                 125
   Asp Asn Thr Thr Cys
       130
<210> 21
   <211> 133
   <212> PRT
   <213> Homo sapiens
<400> 21
   Ala Pro Ile Ser Lys Glu Asp Leu Arg Thr Thr Ile Asp Leu Leu Lys
    1              5                   10                  15
   Gln Glu Ser Gln Asp Leu Tyr Asn Asn Tyr Ser Ile Lys Gln Ala Ser
               20                  25                  30
   Gly Met Ser Ala Asp Glu Ser Ile Gln Leu Pro Ser Phe Ser Leu Asp
           35                  40                  45
   Arg Glu Ala Leu Thr Asn Ile Ser Val Ile Ile Ala His Leu Glu Lys
       50                  55                  60
   Val Lys Val Leu Ser Glu Asn Thr Val Asp Thr Ser Trp Val Ile Arg
   65                  70                  75                  80
   Trp Leu Thr Asn Ile Ser Cys Phe Asn Pro Leu Asn Leu Asn Ile Ser
                   85                  90                  95
   Val Pro Gly Asn Thr Asp Glu Ser Tyr Asp Cys Lys Val Phe Val Leu
               100                 105                 110
   Thr Val Leu Lys Gln Phe Ser Asn Cys Met Ala Glu Leu Gln Ala Lys
           115                 120                 125
   Asp Asn Thr Thr Cys
       130
<210> 22
   <211> 133
   <212> PRT
   <213> Mus musculus
<400> 22
   Ala Pro Ile Ser Lys Glu Asp Leu Arg Thr Thr Ile Asp Leu Leu Lys
    1               5                  10                  15
   Gln Glu Ser Gln Asp Leu Tyr Asn Asn Tyr Ser Ile Lys Gln Ala Ser
               20                  25                  30
   Gly Met Ser Ala Asp Glu Ser Ile Gln Leu Pro Cys Phe Ser Leu Asp
           35                  40                  45
   Arg Glu Ala Leu Thr Asn Ile Ser Val Ile Ile Ala His Leu Glu Lys
       50                  55                  60
   Val Lys Val Leu Ser Glu Asn Thr Val Asp Thr Ser Trp Val Ile Arg
   65                  70                  75                  80
   Trp Leu Thr Asn Ile Ser Ser Phe Asn Pro Leu Asn Leu Asn Ile Ser
                   85                  90                  95
   Val Pro Gly Asn Thr Asp Glu Ser Tyr Asp Cys Lys Val Phe Val Leu
               100                 105                 110
   Thr Val Leu Lys Gln Phe Ser Asn Cys Met Ala Glu Leu Gln Ala Lys
           115                 120                 125
   Asp Asn Thr Thr Cys
       130
<210> 23
   <211> 133
   <212> PRT
   <213> Mus musculus
<400> 23
   Ala Pro Ile Ser Lys Glu Asp Leu Arg Thr Thr Ile Asp Leu Leu Lys
    1               5                  10                  15
   Gln Glu Ser Gln Asp Leu Tyr Asn Asn Tyr Ser Ile Lys Gln Ala Ser
               20                  25                  30
   Gly Met Ser Ala Asp Glu Ser Ile Gln Leu Pro Cys Phe Ser Leu Asp
           35                  40                  45
   Arg Glu Ala Leu Thr Asn Ile Ser Val Ile Ile Ala His Leu Glu Lys
       50                  55                  60
   Val Lys Val Leu Ser Glu Asn Thr Val Asp Thr Ser Trp Val Ile Arg
   65                  70                  75                  80
   Trp Leu Thr Asn Ile Ser Cys Phe Asn Pro Leu Asn Leu Asn Ile Ser
                   85                  90                  95
   Val Pro Gly Asn Thr Asp Glu Ser Tyr Asp Ser Lys Val Phe Val Leu
               100                 105                 110
   Thr Val Leu Lys Gln Phe Ser Asn Cys Met Ala Glu Leu Gln Ala Lys
           115                 120                 125
   Asp Asn Thr Thr Cys
       130
<210> 24
   <211> 133
   <212> PRT
   <213> Mus musculus
<400> 24
   Ala Pro Ile Ser Lys Glu Asp Leu Arg Thr Thr Ile Asp Leu Leu Lys
    1               5                  10                  15
   Gln Glu Ser Gln Asp Leu Tyr Asn Asn Tyr Ser Ile Lys Gln Ala Ser
               20                  25                  30
   Gly Met Ser Ala Asp Glu Ser Ile Gln Leu Pro Cys Phe Ser Leu Asp
           35                  40                  45
   Arg Glu Ala Leu Thr Asn Ile Ser Val Ile Ile Ala His Leu Glu Lys
       50                  55                  60
   Val Lys Val Leu Ser Glu Asn Thr Val Asp Thr Ser Trp Val Ile Arg
   65                  70                  75                  80
   Trp Leu Thr Asn Ile Ser Cys Phe Asn Pro Leu Asn Leu Asn Ile Ser
                   85                  90                  95
   Val Pro Gly Asn Thr Asp Glu Ser Tyr Asp Cys Lys Val Phe Val Leu
               100                 105                 110
   Thr Val Leu Lys Gln Phe Ser Asn Ser Met Ala Glu Leu Gln Ala Lys
           115                 120                 125
   Asp Asn Thr Thr Cys
       130
<210> 25
   <211> 133
   <212> PRT
   <213> Mus musculus
<400> 25
   Ala Pro Ile Ser Lys Glu Asp Leu Arg Thr Thr Ile Asp Leu Leu Lys
    1               5                  10                  15
   Gln Glu Ser Gln Asp Leu Tyr Asn Asn Tyr Ser Ile Lys Gln Ala Ser
               20                  25                  30
   Gly Met Ser Ala Asp Glu Ser Ile Gln Leu Pro Cys Phe Ser Leu Asp
           35                  40                  45
   Arg Glu Ala Leu Thr Asn Ile Ser Val Ile Ile Ala His Leu Glu Lys
       50                  55                  60
   Val Lys Val Leu Ser Glu Asn Thr Val Asp Thr Ser Trp Val Ile Arg
   65                  70                  75                  80
   Trp Leu Thr Asn Ile Ser Cys Phe Asn Pro Leu Asn Leu Asn Ile Ser
                   85                  90                  95
   Val Pro Gly Asn Thr Asp Glu Ser Tyr Asp Cys Lys Val Phe Val Leu
               100                 105                 110
   Thr Val Leu Lys Gln Phe Ser Asn Cys Met Ala Glu Leu Gln Ala Lys
           115                 120                 125
   Asp Asn Thr Thr Ser
       130
<210> 26
   <211> 134
   <212> PRT
   <213> Mus musculus
<400> 26
   Met Ala Pro Ile Ser Lys Glu Asp Leu Arg Thr Thr Ile Asp Leu Leu
   1 5 10 15
   Lys Gln Glu Ser Gln Asp Leu Tyr Asn Asn Tyr Ser Ile Lys Gln Ala
   20 25 30
   Ser Gly Met Ser Ala Asp Glu Ser Ile Gln Leu Pro Ser Phe Ser Leu
   35 40 45
   Asp Arg Glu Ala Leu Thr Asn Ile Ser Val Ile Ile Ala His Leu Glu
   50 55 60
   Lys Val Lys Val Leu Ser Glu Asn Thr Val Asp Thr Ser Trp Val Ile
   65 70 75 80
   Arg Trp Leu Thr Asn Ile Ser Cys Phe Asn Pro Leu Asn Leu Asn Ile
   85 90 95
   Ser Val Pro Gly Asn Thr Asp Glu Ser Tyr Asp Cys Lys Val Phe Val
   100 105 110
   Leu Thr Val Leu Lys Gln Phe Ser Asn Cys Met Ala Glu Leu Gln Ala
   115 120 125
   Lys Asp Asn Thr Thr Cys
   130
<210> 27
   <211> 134
   <212> PRT
   <213> Mus musculus
<400> 27
   Met Ala Pro Ile Ser Lys Glu Asp Leu Arg Thr Thr Ile Asp Leu Leu
   1 5 10 15
   Lys Gln Glu Ser Gln Asp Leu Tyr Asn Asn Tyr Ser Ile Lys Gln Ala
   20 25 30
   Ser Gly Met Ser Ala Asp Glu Ser Ile Gln Leu Pro Cys Phe Ser Leu
   35 40 45
   Asp Arg Glu Ala Leu Thr Asn Ile Ser Val Ile Ile Ala His Leu Glu
   50 55 60
   Lys Val Lys Val Leu Ser Glu Asn Thr Val Asp Thr Ser Trp Val Ile
   65 70 75 80
   Arg Trp Leu Thr Asn Ile Ser Ser Phe Asn Pro Leu Asn Leu Asn Ile
   85 90 95
   Ser Val Pro Gly Asn Thr Asp Glu Ser Tyr Asp Cys Lys Val Phe Val
   100 105 110
   Leu Thr Val Leu Lys Gln Phe Ser Asn Cys Met Ala Glu Leu Gln Ala
   115 120 125
   Lys Asp Asn Thr Thr Cys
   130
<210> 28
   <211> 134
   <212> PRT
   <213> Mus musculus
<400> 28
   Met Ala Pro Ile Ser Lys Glu Asp Leu Arg Thr Thr Ile Asp Leu Leu
    1               5                  10                  15
   Lys Gln Glu Ser Gln Asp Leu Tyr Asn Asn Tyr Ser Ile Lys Gln Ala
               20                  25                  30
   Ser Gly Met Ser Ala Asp Glu Ser Ile Gln Leu Pro Cys Phe Ser Leu
           35                  40                  45
   Asp Arg Glu Ala Leu Thr Asn Ile Ser Val Ile Ile Ala His Leu Glu
       50                  55                  60
   Lys Val Lys Val Leu Ser Glu Asn Thr Val Asp Thr Ser Trp Val Ile
   65                  70                  75                  80
   Arg Trp Leu Thr Asn Ile Ser Cys Phe Asn Pro Leu Asn Leu Asn Ile
                   85                  90                  95
   Ser Val Pro Gly Asn Thr Asp Glu Ser Tyr Asp Ser Lys Val Phe Val
               100                 105                 110
   Leu Thr Val Leu Lys Gln Phe Ser Asn Cys Met Ala Glu Leu Gln Ala
           115                 120                 125
   Lys Asp Asn Thr Thr Cys
       130
<210> 29
   <211> 134
   <212> PRT
   <213> Mus musculus
<400> 29
   Met Ala Pro Ile Ser Lys Glu Asp Leu Arg Thr Thr Ile Asp Leu Leu
    1               5                  10                  15
   Lys Gln Glu Ser Gln Asp Leu Tyr Asn Asn Tyr Ser Ile Lys Gln Ala
               20                  25                  30
   Ser Gly Met Ser Ala Asp Glu Ser Ile Gln Leu Pro Cys Phe Ser Leu
           35                  40                  45
   Asp Arg Glu Ala Leu Thr Asn Ile Ser Val Ile Ile Ala His Leu Glu
       50                  55                  60
   Lys Val Lys Val Leu Ser Glu Asn Thr Val Asp Thr Ser Trp Val Ile
   65                  70                  75                  80
   Arg Trp Leu Thr Asn Ile Ser Cys Phe Asn Pro Leu Asn Leu Asn Ile
                   85                  90                  95
   Ser Val Pro Gly Asn Thr Asp Glu Ser Tyr Asp Cys Lys Val Phe Val
               100                 105                 110
   Leu Thr Val Leu Lys Gln Phe Ser Asn Ser Met Ala Glu Leu Gln Ala
           115                 120                 125
   Lys Asp Asn Thr Thr Cys
       130
<210> 30
   <211> 134
   <212> PRT
   <213> Mus musculus
<400> 30
   Met Ala Pro Ile Ser Lys Glu Asp Leu Arg Thr Thr Ile Asp Leu Leu
    1               5                  10                  15
   Lys Gln Glu Ser Gln Asp Leu Tyr Asn Asn Tyr Ser Ile Lys Gln Ala
               20                  25                  30
   Ser Gly Met Ser Ala Asp Glu Ser Ile Gln Leu Pro Cys Phe Ser Leu
           35                  40                  45
   Asp Arg Glu Ala Leu Thr Asn Ile Ser Val Ile Ile Ala His Leu Glu
       50                  55                  60
   Lys Val Lys Val Leu Ser Glu Asn Thr Val Asp Thr Ser Trp Val Ile
   65                  70                  75                  80
   Arg Trp Leu Thr Asn Ile Ser Cys Phe Asn Pro Leu Asn Leu Asn Ile
                   85                  90                  95
   Ser Val Pro Gly Asn Thr Asp Glu Ser Tyr Asp Cys Lys Val Phe Val
               100                 105                 110
   Leu Thr Val Leu Lys Gln Phe Ser Asn Cys Met Ala Glu Leu Gln Ala
           115                 120                 125
   Lys Asp Asn Thr Thr Ser
       130
<210> 31
   <211> 516
   <212> DNA
   <213> Homo sapiens
<400> 31
   atggcctctc actcaggccc ctcgacgtct gtgctctttc tgttctgctg cctgggaggc 60
   tggctggcct cccacacgtt gcccgtccgt ttactacgac caagtgatga tgtacagaaa 120
   atagtcgagg aattacagtc cctctcgaag atgcttttga aagatgtgga ggaagagaag 180
   ggcgtgctcg tgtcccagaa ttacacgctg ccgtgtctca gccctgacgc ccagccgcca 240
   aacaacatcc acagcccagc catccgggca tatctcaaga caatcagaca gctagacaac 300
   aaatctgtta ttgatgagat catagagcac ctcgacaaac tcatatttca agatgcacca 360
   gaaacaaaca tttctgtgcc aacagacacc catgaatgta aacgcttcat cctgactatt 420
   tctcaacagt tttcagagtg catggacctc gcactaaaat cattgacctc tggagcccaa 480
   caggccacca ctgaagaata catgccgatg gaataa                           516
<210> 32
   <211> 49
   <212> DNA
   <213> Artificial Sequence
<220>
   <223> Oligonucleotide primer ZC41607
<400> 32
   tccagggaat tcatataggc cggccaccat ggcctctcac tcaggcccc 49
<210> 33
   <211> 82
   <212> DNA
   <213> Artificial Sequence
<220>
   <223> Oligonucleotide primer ZC41605
<400> 33
   caaccccaga gctgttttaa ggcgcgcctc tagattatta ttccatcggc atgtattctt 60
   cagtggtggc ctgttgggct cc 82
<210> 34
   <211> 513
   <212> DNA
   <213> Mus musculus
<400> 34
   atgatcttcc acacaggaac aacgaagcct accctggtgc tgctttgctg tataggaacc 60
   tggctggcca cctgcagctt gtccttcggt gccccaatat cgaaggaaga cttaagaact 120
   acaattgacc tcttgaaaca agagtctcag gatctttata acaactatag cataaagcag 180
   gcatctggga tgtcagcaga cgaatcaata cagctgccgt gtttcagcct ggaccgggaa 240
   gcattaacca acatctcggt catcatagca catctggaga aagtcaaagt gttgagcgag 300
   aacacagtag atacttcttg ggtgataaga tggctaacaa acatcagctg tttcaaccca 360
   ctgaatttaa acatttctgt gcctggaaat actgatgaat cctatgattg taaagtgttc 420
   gtgcttacgg ttttaaagca gttctcaaac tgcatggcag aactgcaggc taaggacaat 480
   actacatgcg aagaatacat gccgatggaa tga 513
<210> 35
   <211> 49
   <212> DNA
   <213> Artificial Sequence
<220>
   <223> Oligonucleotide primer ZC41643
<400> 35
   tccagggaat tcatataggc cggccaccat gatcttccac acaggaaca 49
<210> 36
   <211> 85
   <212> DNA
   <213> Artificial Sequence
<220>
   <223> Oligonucleotide primer ZC41641
<400> 36
   caaccccaga gctgttttaa ggcgcgcctc tagattatca ttccatcggc atgtattctt 60
   cgcatgtagt attgtcctta gcctg 85
<210> 37
   <211> 66
   <212> DNA
   <213> Artificial Sequence
<220>
   <223> Oligonucleotide primer ZC43156
<400> 37
   ctagaaataa ttttgtttaa ctttaagaag gagatatata tatgttgccc gtccgtttac 60
   tacgac                                                            66
<210> 38
   <211> 50
   <212> DNA
   <213> Artificial Sequence
<220>
   <223> Oligonucleotide primer ZC45307
<400> 38
   tgagaaatag tcaggatgaa gcgtttagat tcatgggtgt ctgttggcac 50
<210> 39
   <211> 64
   <212> DNA
   <213> Artificial Sequence
<220>
   <223> Oligonucleotide primer ZC43137
<400> 39
   tctgtatcag gctgaaaatc ttatctcatc cgccaaaaca ttaagtggtg gcctgttggg 60
   ctcc                                                              64
<210> 40
   <211> 50
   <212> DNA
   <213> Artificial Sequence
<220>
   <223> Oligonucleotide primer ZC45306
<400> 40
   gtgccaacag acacccatga atctaaacgc ttcatcctga ctatttctca 50
<210> 41
   <211> 420
   <212> DNA
   <213> Homo sapiens
<400> 41
   atgttgcccg tccgtttact acgaccaagt gatgatgtac agaaaatagt cgaggaatta 60
   cagtccctct cgaagatgct tttgaaagat gtggaggaag agaagggcgt gctcgtgtcc 120
   cagaattaca cgctgccgtg tctcagccct gacgcccagc cgccaaacaa catccacagc 180
   ccagccatcc gggcatatct caagacaatc agacagctag acaacaaatc tgttattgat 240
   gagatcatag agcacctcga caaactcata tttcaagatg caccagaaac aaacatttct 300
   gtgccaacag acacccatga atctaaacgc ttcatcctga ctatttctca acagttttca 360
   gagtgcatgg acctcgcact aaaatcattg acctctggag cccaacaggc caccacttaa 420
<210> 42
   <211> 139
   <212> PRT
   <213> Homo sapiens
<400> 42
   Met Leu Pro Val Arg Leu Leu Arg Pro Ser Asp Asp Val Gln Lys Ile
    1              5                  10                  15
   Val Glu Glu Leu Gln Ser Leu Ser Lys Met Leu Leu Lys Asp Val Glu
               20                  25                  30
   Glu Glu Lys Gly Val Leu Val Ser Gln Asn Tyr Thr Leu Pro Cys Leu
           35                  40                  45
   Ser Pro Asp Ala Gln Pro Pro Asn Asn Ile His Ser Pro Ala Ile Arg
       50                  55                  60
   Ala Tyr Leu Lys Thr Ile Arg Gln Leu Asp Asn Lys Ser Val Ile Asp
   65                  70                  75                  80
   Glu Ile Ile Glu His Leu Asp Lys Leu Ile Phe Gln Asp Ala Pro Glu
                   85                  90                  95
   Thr Asn Ile Ser Val Pro Thr Asp Thr His Glu Ser Lys Arg Phe Ile
               100                 105                 110
   Leu Thr Ile Ser Gln Gln Phe Ser Glu Cys Met Asp Leu Ala Leu Lys
           115                 120                 125
   Ser Leu Thr Ser Gly Ala Gln Gln Ala Thr Thr
       130                 135
<210> 43
   <211> 68
   <212> DNA
   <213> Artificial Sequence
<220>
   <223> Oligonucleotide primer ZC43883
<400> 43
   tagaaataat tttgtttaac tttaagaagg agatatatat atggccccaa tatcgaagga 60
   agacttaa                                                           68
<210> 44
   <211> 50
   <212> DNA
   <213> Artificial Sequence
<220>
   <223> Oligonucleotide primer ZC45302
<400> 44
   tttaaaaccg taagcacgaa cactttagaa tcataggatt catcagtatt 50
<210> 45
   <211> 64
   <212> DNA
   <213> Artificial Sequence
<220>
   <223> Oligonucleotide primer ZC43875
<400> 45
   tctgtatcag gctgaaaatc ttatctcatc cgccaaaact cagcatgtag tattgtcctt 60
   agcc                                                              64
<210> 46
   <211> 50
   <212> DNA
   <213> Artificial Sequence
<220>
   <223> Oligonucleotide primer ZC45303
<400> 46
   aatactgatg aatcctatga ttctaaagtg ttcgtgctta cggttttaaa 50
<210> 47
   <211> 405
   <212> DNA
   <213> Mus musculus
<400> 47
   atggccccaa tatcgaagga agacttaaga actacaattg acctcttgaa acaagagtct 60
   caggatcttt ataacaacta tagcataaag caggcatctg ggatgtcagc agacgaatca 120
   atacagctgc cgtgtttcag cctggaccgg gaagcattaa ccaacatctc ggtcatcata 180
   gcacatctgg agaaagtcaa agtgttgagc gagaacacag tagatacttc ttgggtgata 240
   agatggctaa caaacatcag ctgtttcaac ccactgaatt taaacatttc tgtgcctgga 300
   aatactgatg aatcctatga ttctaaagtg ttcgtgctta cggttttaaa gcagttctca 360
   aactgcatgg cagaactgca ggctaaggac aatactacat gctga                 405
<210> 48
   <211> 134
   <212> PRT
   <213> Mus musculus
<400> 48
   Met Ala Pro Ile Ser Lys Glu Asp Leu Arg Thr Thr Ile Asp Leu Leu
    1               5                  10                  15
   Lys Gln Glu Ser Gln Asp Leu Tyr Asn Asn Tyr Ser Ile Lys Gln Ala
               20                  25                  30
   Ser Gly Met Ser Ala Asp Glu Ser Ile Gln Leu Pro Cys Phe Ser Leu
           35                  40                  45
   Asp Arg Glu Ala Leu Thr Asn Ile Ser Val Ile Ile Ala His Leu Glu
       50                  55                  60
   Lys Val Lys Val Leu Ser Glu Asn Thr Val Asp Thr Ser Trp Val Ile
   65                  70                  75                  80
   Arg Trp Leu Thr Asn Ile Ser Cys Phe Asn Pro Leu Asn Leu Asn Ile
                   85                  90                  95
   Ser Val Pro Gly Asn Thr Asp Glu Ser Tyr Asp Ser Lys Val Phe Val
               100                 105                 110
   Leu Thr Val Leu Lys Gln Phe Ser Asn Cys Met Ala Glu Leu Gln Ala
           115                 120                 125
   Lys Asp Asn Thr Thr Cys
       130
<210> 49
   <211> 139
   <212> PRT
   <213> Homo sapiens
<400> 49
   Met Leu Pro Val Arg Leu Leu Arg Pro Ser Asp Asp Val Gln Lys Ile
    1               5                   10                  15
   Val Glu Glu Leu Gln Ser Leu Ser Lys Met Leu Leu Lys Asp Val Glu
               20                  25                  30
   Glu Glu Lys Gly Val Leu Val Ser Gln Asn Tyr Thr Leu Pro Cys Leu
           35                  40                  45
   Ser Pro Asp Ala Gln Pro Pro Asn Asn Ile His Ser Pro Ala Ile Arg
       50                  55                  60
   Ala Tyr Leu Lys Thr Ile Arg Gln Leu Asp Asn Lys Ser Val Ile Asp
   65                  70                  75                  80
   Glu Ile Ile Glu His Leu Asp Lys Leu Ile Phe Gln Asp Ala Pro Glu
                   85                  90                  95
   Thr Asn Ile Ser Val Pro Thr Asp Thr His Glu Cys Lys Arg Phe Ile
               100                 105                 110
   Leu Thr Ile Ser Gln Gln Phe Ser Glu Cys Met Asp Leu Ala Leu Lys
           115                 120                 125
   Ser Leu Thr Ser Gly Ala Gln Gln Ala Thr Thr
       130                 135